(19) Europäisches Patentamt
European Patent Office
Office européen des brevets

(11) EP 3 862 352 A1

(12) EUROPEAN PATENT APPLICATION
published in accordance with Art. 153(4) EPC

(43) Date of publication:
**11.08.2021 Bulletin 2021/32**

(21) Application number: **19868563.8**

(22) Date of filing: **30.09.2019**

(51) Int Cl.:
*C07D 403/14* (2006.01) *A01N 43/54* (2006.01)
*A01N 43/653* (2006.01) *A01P 5/00* (2006.01)
*A01P 7/02* (2006.01) *A01P 7/04* (2006.01)
*A61K 31/4439* (2006.01) *A61K 31/444* (2006.01)
*A61K 31/506* (2006.01) *A61P 33/14* (2006.01)
*C07D 401/04* (2006.01) *C07D 401/14* (2006.01)
*C07D 403/04* (2006.01)

(86) International application number:
**PCT/JP2019/038480**

(87) International publication number:
**WO 2020/071304 (09.04.2020 Gazette 2020/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2018 JP 2018187675**
**29.10.2018 JP 2018202997**

(71) Applicant: **Nippon Soda Co., Ltd.**
**Tokyo 100-8165 (JP)**

(72) Inventors:
- **SAKANISHI, Keita**
  **Odawara-shi, Kanagawa 250-0280 (JP)**
- **SAKIYAMA, Norifumi**
  **Odawara-shi, Kanagawa 250-0280 (JP)**
- **AOYAMA, Hikaru**
  **Odawara-shi, Kanagawa 250-0280 (JP)**
- **IWASA, Takao**
  **Odawara-shi, Kanagawa 250-0280 (JP)**
- **MATSUI, Maki**
  **Odawara-shi, Kanagawa 250-0280 (JP)**
- **KOBAYASHI, Tomomi**
  **Odawara-shi, Kanagawa 250-0280 (JP)**
- **USHIJIMA, Daisuke**
  **Odawara-shi, Kanagawa 250-0280 (JP)**

(74) Representative: **Wibbelmann, Jobst**
**Wuesthoff & Wuesthoff**
**Patentanwälte PartG mbB**
**Schweigerstrasse 2**
**81541 München (DE)**

# (54) HETEROARYLAZOLE COMPOUND AND PEST CONTROL AGENT

(57) An object of the present invention is to provide a heteroaryl azole compound that is excellent in pest control activity, particularly, insecticidal activity and/or miticidal activity, is excellent in safety, and may be industrially advantageously synthesized. The compound of the present invention is a compound represented by the formula (I), an N-oxide compound, stereoisomer, tautomer or hydrate thereof or a salt of any of these compounds. In the formula (I), A represents CH or a nitrogen atom; $B^1$ represents $CX^1$ or a nitrogen atom; $X^1$, $X^2$ and $X^3$ each independently represent a hydrogen atom, a substituted or unsubstituted Cl-6 alkyl group, a substituted or unsubstituted C2-6 alkenyl group, or the like; $R^1$ represents a substituted or unsubstituted Cl-6 alkylthio group, a substituted or unsubstituted Cl-6 alkylsulfinyl group, or the like; $R^2$ represents a substituted or unsubstituted Cl-6 alkyl group; and R represents a substituted or unsubstituted Cl-6 alkyl group.

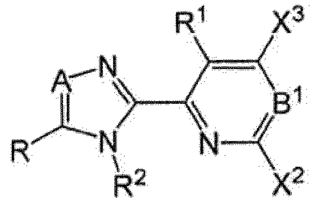

(I)

EP 3 862 352 A1

## Description

### Technical Field

The present invention relates to a heteroaryl azole compound and a pest control agent. More specifically, the present invention relates to a heteroaryl azole compound that has excellent insecticidal activity and/or miticidal activity, is excellent in safety, and may be industrially advantageously synthesized, and a pest control agent containing the same as an active ingredient. The present application claims the priority of Japanese Patent Application No. 2018-187675 filed on October 2, 2018 and Japanese Patent Application No. 2018-202997 filed on October 29, 2018, the contents of which are incorporated herein.

### Background Art

Various compounds having insecticidal or miticidal activity have been proposed. For practical use of such compounds as agrochemicals, it is required not only to have sufficiently high efficacy but to be less likely to cause chemical resistance, to cause neither phytotoxicity to plants nor soil pollution, and to be low toxic to livestock, fishes, etc.

Patent document 1 discloses a compound represented by the formula (A) or (B), etc.

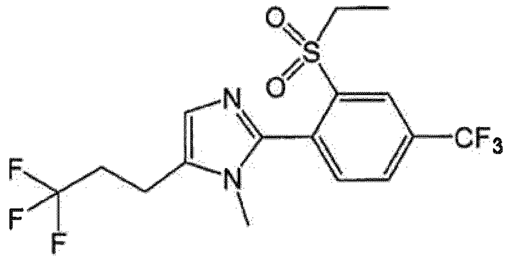

(A)

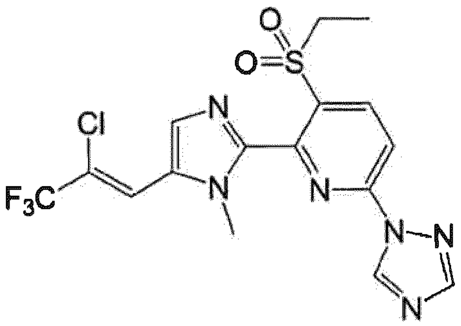

(B)

Patent document 2 discloses a compound represented by the formula (C), etc.

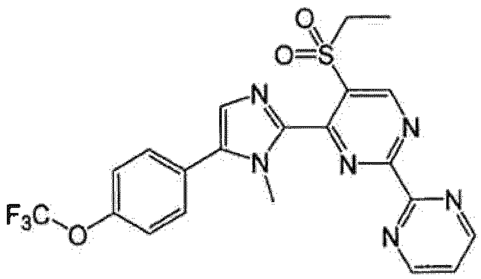

(C)

### Prior Art Documents

### Patent Documents

Patent document 1: WO2017/104741A
Patent document 2: WO2018/052035A

## Summary of the Invention

### Object to be Solved by the Invention

An object of the present invention is to provide a heteroaryl azole compound that is excellent in pest control activity, particularly, insecticidal activity and/or miticidal activity, is excellent in safety, and may be industrially advantageously synthesized. Another object of the present invention is to provide a pest control agent, an insecticide or miticide, an ectoparasite control agent, or an endoparasite control agent or expellant containing the heteroaryl azole compound as an active ingredient.

### Means to Solve the Object

As a result of diligent studies to attain the objects, the present invention including the following aspects has been completed.

[1] A compound represented by the formula (I), an N-oxide compound, stereoisomer, tautomer or hydrate thereof or a salt of any of these compounds:

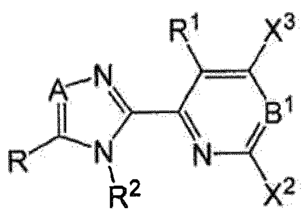

(I)

wherein

A represents CH or a nitrogen atom;
$B^1$ represents $CX^1$ or a nitrogen atom;
$X^1$, $X^2$ and $X^3$ each independently represent a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$;
$R^3$ and $R^4$ each independently represent a hydrogen atom, a C1-6 alkyl group, or a C1-6 haloalkyl group;
$R^5$, $R^6$, $R^7$ and $R^8$ each represent a C1-6 alkyl group;
q represents 0 or 1;
$R^1$ represents a substituted or unsubstituted C1-6 alkylthio group, a substituted or unsubstituted Cl-6 alkylsulfinyl group, or a substituted or unsubstituted C1-6 alkylsulfonyl group;
$R^2$ represents a substituted or unsubstituted C1-6 alkyl group; and
R represents a substituted or unsubstituted C1-6 alkyl group.

[2] The compound according to the above [1], an N-oxide compound, stereoisomer, tautomer or hydrate thereof or a salt of any of these compounds, wherein the formula (I) is the formula (II):

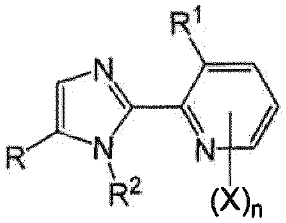

(II)

wherein

$R^1$, $R^2$, and R represent the same meanings as described in the formula (I);
X represents a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$;
$R^3$ and $R^4$ each independently represent a hydrogen atom, a C1-6 alkyl group, or a C1-6 haloalkyl group;
$R^5$, $R^6$, $R^7$ and $R^8$ each represent a C1-6 alkyl group;
q represents 0 or 1; and
n represents a chemically acceptable number of X and is any integer of 0 to 3, and when n is 2 or larger, each of the X is the same or different.

[3] The compound according to the above [1], an N-oxide compound, stereoisomer, tautomer or hydrate thereof or a salt of any of these compounds, wherein the formula (I) is the formula (III):

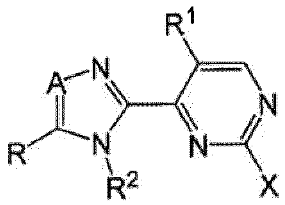

(III)

wherein

A, $R^1$, $R^2$, and R have the same meanings as described in the formula (I); and
X has the same meaning as described in the formula (II).

[4] The compound according to the above [1], an N-oxide compound, stereoisomer, tautomer or hydrate thereof or a salt of any of these compounds, wherein in the formula (I), R represents a C1-6 haloalkyl group.
[5] A pest control agent comprising at least one active ingredient selected from the group consisting of a compound according to any one of the above [1] to [4], an N-oxide compound, stereoisomer, tautomer and hydrate thereof and a salt of any of these compounds.
[6] An insecticide or miticide comprising at least one active ingredient selected from the group consisting of a compound according to any one of the above [1] to [4], an N-oxide compound, stereoisomer, tautomer and hydrate thereof and a salt of any of these compounds.
[7] An ectoparasite control agent comprising at least one active ingredient selected from the group consisting of a compound according to any one of the above [1] to [4], an N-oxide compound, stereoisomer, tautomer and hydrate thereof and a salt of any of these compounds.
[8] An endoparasite control agent or expellant comprising at least one active ingredient selected from the group consisting of a compound according to any one of the above [1] to [4], an N-oxide compound, stereoisomer, tautomer and hydrate thereof and a salt of any of these compounds.

[9] A seed treatment agent or vegetative propagation organ treatment agent comprising at least one active ingredient selected from the group consisting of a compound according to any one of the above [1] to [4], an N-oxide compound, stereoisomer, tautomer and hydrate thereof and a salt of any of these compounds.

[10] A granular agrochemical composition for paddy rice seedling nursery box treatment comprising at least one active ingredient selected from the group consisting of a compound according to any one of the above [1] to [4], an N-oxide compound, stereoisomer, tautomer and hydrate thereof and a salt of any of these compounds.

[11] A soil treatment agent comprising at least one active ingredient selected from the group consisting of a compound according to any one of the above [1] to [4], an N-oxide compound, stereoisomer, tautomer and hydrate thereof and a salt of any of these compounds.

[12] A bait agent comprising at least one active ingredient selected from the group consisting of a compound according to any one of the above [1] to [4], an N-oxide compound, stereoisomer, tautomer and hydrate thereof and a salt of any of these compounds.

[13] A plant growth promoter comprising at least one active ingredient selected from the group consisting of a compound according to any one of the above [1] to [4], an N-oxide compound, stereoisomer, tautomer and hydrate thereof and a salt of any of these compounds.

## Effect of the Invention

The heteroaryl azole compound of the present invention may control pests that are problems associated with crops or hygiene. Particularly, the heteroaryl azole compound of the present invention may effectively control agricultural insect pests and mites at a lower concentration. Furthermore, the heteroaryl azole compound of the present invention may effectively control ectoparasites and endoparasites harmful to humans and animals.

## Mode of Carrying Out the Invention

[Heteroaryl azole compound]

The heteroaryl azole compound of the present invention is a compound represented by the formula (I) (hereinafter, also referred to as compound (I)), an N-oxide compound, stereoisomer, tautomer or hydrate thereof or a salt of any of these compounds. The compound represented by the formula (I) includes every stereoisomer which is an enantiomer or a diastereomer.

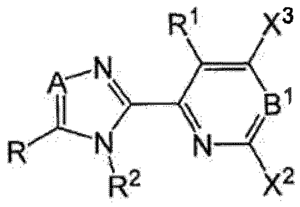

(I)

In the present invention, the term "unsubstituted" means a group consisting of only a mother nucleus. Only the name of a group consisting of a mother nucleus without the term "substituted" means an "unsubstituted" group unless otherwise specified.

On the other hand, the term "substituted" means that any hydrogen atom of a group consisting of a mother nucleus is substituted with a group (substituent) having a structure that is the same as or different from that of the mother nucleus. Thus, the "substituent" means another group bonded to the group consisting of a mother nucleus. The number of the substituent may be one or more. Two or more substituents are the same or different.

Terms such as "C1-6" mean that the number of carbon atoms in the group consisting of a mother nucleus is 1 to 6, etc. This number of carbon atoms does not include the number of carbon atoms present in the substituent. For example, a butyl group having an ethoxy group as a substituent is classified into a C2 alkoxy C4 alkyl group.

The "substituent" is not particularly limited as long as the substituent is chemically acceptable and produces the effect of the present invention. Hereinafter, a group capable of serving as the "substituent" is exemplified:

a C1-6 alkyl group such as a methyl group, an ethyl group, a n-propyl group, an i-propyl group, a n-butyl group, a s-butyl group, an i-butyl group, a t-butyl group, a n-pentyl group, and a n-hexyl group;

a C2-6 alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;

a C2-6 alkynyl group such as an ethynyl group, a 1-propynyl group, a 2-propynyl group, a 1-butynyl group, a 2-butynyl group, a 3-butynyl group, and a 1-methyl-2-propynyl group;
a C3-8 cycloalkyl group such as a cyclopropyl group, a cyclobutyl group, a cyclopentyl group, a cyclohexyl group, and a cubanyl group;
a C6-10 aryl group such as a phenyl group and a naphthyl group;
a C6-10 aryl C1-6 alkyl group such as a benzyl group and a phenethyl group;
a 3- to 6-membered heterocyclyl group;
a 3- to 6-membered heterocyclyl C1-6 alkyl group;
a hydroxy group;
a C1-6 alkoxy group such as a methoxy group, an ethoxy group, a n-propoxy group, an i-propoxy group, a n-butoxy group, a s-butoxy group, an i-butoxy group, and a t-butoxy group;
a C2-6 alkenyloxy group such as a vinyloxy group, an allyloxy group, a propenyloxy group, and a butenyloxy group;
a C2-6 alkynyloxy group such as an ethynyloxy group and a propargyloxy group;
a C6-10 aryloxy group such as a phenoxy group and a naphthoxy group;
a C6-10 aryl C1-6 alkoxy group such as a benzyloxy group and a phenethyloxy group;
a 5- or 6-membered heteroaryloxy group such as a thiazolyloxy group and a pyridyloxy group;
a 5- or 6-membered heteroaryl C1-6 alkyloxy group such as a thiazolylmethyloxy group and a pyridylmethyloxy group;
a formyl group;
a C1-6 alkylcarbonyl group such as an acetyl group and a propionyl group;
a formyloxy group;
a C1-6 alkylcarbonyloxy group such as an acetyloxy group and a propionyloxy group;
a C6-10 arylcarbonyl group such as a benzoyl group;
a C1-6 alkoxycarbonyl group such as a methoxycarbonyl group, an ethoxycarbonyl group, a n-propoxycarbonyl group, an i-propoxycarbonyl group, a n-butoxycarbonyl group, and a t-butoxycarbonyl group;
a C1-6 alkoxycarbonyloxy group such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxy group;
a halogeno group such as a fluoro group, a chloro group, a bromo group, and an iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group;
a C2-6 haloalkenyl group such as a 2-chloro-1-propenyl group and a 2-fluoro-1-butenyl group;
a C2-6 haloalkynyl group such as a 4,4-dichloro-1-butynyl group, a 4-fluoro-1-pentynyl group, and a 5-bromo-2-pentynyl group;
a C1-6 haloalkoxy group such as a trifluoromethoxy group, a 2-chloro-n-propoxy group, and a 2,3-dichlorobutoxy group;
a C2-6 haloalkenyloxy group such as a 2-chloropropenyloxy group and a 3-bromobutenyloxy group;
a C1-6 haloalkylcarbonyl group such as a chloroacetyl group, a trifluoroacetyl group, and a trichloroacetyl group;
an amino group;
a C1-6 alkyl-substituted amino group such as a methylamino group, a dimethylamino group, and a diethylamino group;
a C6-10 arylamino group such as an anilino group and a naphthylamino group;
a C6-10 aryl C1-6 alkylamino group such as a benzylamino group and a phenethylamino group;
a formylamino group;
a C1-6 alkylcarbonylamino group such as an acetylamino group, a propanoylamino group, a butyrylamino group, and an i-propylcarbonylamino group;
a C1-6 alkoxycarbonylamino group such as a methoxycarbonylamino group, an ethoxycarbonylamino group, a n-propoxycarbonylamino group, and an i-propoxycarbonylamino group;
an unsubstituted or substituted aminocarbonyl group such as an aminocarbonyl group, a dimethylaminocarbonyl group, a phenylaminocarbonyl group, and a N-phenyl-N-methylaminocarbonyl group;
an imino C1-6 alkyl group such as an iminomethyl group, a (1-imino)ethyl group, and a (1-imino)-n-propyl group;
a substituted or unsubstituted N-hydroxyimino C1-6 alkyl group such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
a C1-6 alkoxyimino group such as a methoxyimino group, an ethoxyimino group, a n-propoxyimino group, an i-propoxyimino group, and a n-butoxyimino group;

an aminocarbonyloxy group;
a C1-6 alkyl-substituted aminocarbonyloxy group such as an ethylaminocarbonyloxy group and a dimethylaminocarbonyloxy group;
a mercapto group;
a C1-6 alkylthio group such as a methylthio group, an ethylthio group, a n-propylthio group, an i-propylthio group, a n-butylthio group, an i-butylthio group, a s-butylthio group, and a t-butylthio group;
a C1-6 haloalkylthio group such as a trifluoromethylthio group and a 2,2,2-trifluoroethylthio group;
a C6-10 arylthio group such as a phenylthio group and a naphthylthio group;
a 5- or 6-membered heteroarylthio group such as a thiazolylthio group and a pyridylthio group;
a C1-6 alkylsulfinyl group such as a methylsulfinyl group, an ethylsulfinyl group, and a t-butylsulfinyl group;
a C1-6 haloalkylsulfinyl group such as a trifluoromethylsulfinyl group and a 2,2,2-trifluoroethylsulfinyl group;
a C6-10 arylsulfinyl group such as a phenylsulfinyl group;
a 5- or 6-membered heteroarylsulfinyl group such as a thiazolylsulfinyl group and a pyridylsulfinyl group;
a C1-6 alkylsulfonyl group such as a methylsulfonyl group, an ethylsulfonyl group, and a t-butylsulfonyl group;
a C1-6 haloalkylsulfonyl group such as a trifluoromethylsulfonyl group and a 2,2,2-trifluoroethylsulfonyl group;
a C6-10 arylsulfonyl group such as a phenylsulfonyl group;
a 5- or 6-membered heteroarylsulfonyl group such as a thiazolylsulfonyl group and a pyridylsulfonyl group;
a C1-6 alkylsulfonyloxy group such as a methylsulfonyloxy group, an ethylsulfonyloxy group, and a t-butylsulfonyloxy group;
a C1-6 haloalkylsulfonyloxy group such as a trifluoromethylsulfonyloxy group and a 2,2,2-trifluoroethylsulfonyloxy group;
a tri-C1-6 alkyl-substituted silyl group such as a trimethylsilyl group, a triethylsilyl group, and a t-butyldimethylsilyl group;
a tri-C6-10 aryl-substituted silyl group such as a triphenylsilyl group;
a cyano group; and a nitro group.

For these "substituents", any hydrogen atom in each substituent may be substituted with a group having a distinct structure. In this case, as the "substituent", a C1-6 alkyl group, a C1-6 haloalkyl group, a C1-6 alkoxy group, a C1-6 haloalkoxy group, a halogeno group, a cyano group, a nitro group or the like may be exemplified.

The "3- to 6-membered heterocyclyl group" described above contains 1 to 4 heteroatoms selected from the group consisting of a nitrogen atom, an oxygen atom and a sulfur atom as ring-constituting atoms. The heterocyclyl group may be either monocyclic or polycyclic. The polycyclic heterocyclyl group has at least one hetero ring, and the remaining ring(s) may be any of a saturated alicyclic ring, an unsaturated alicyclic ring and an aromatic ring. As the "3- to 6-membered heterocyclyl group", a 3- to 6-membered saturated heterocyclyl group, a 5- or 6-membered heteroaryl group, a 5- or 6-membered partially unsaturated heterocyclyl group or the like may be exemplified.

As the 3- to 6-membered saturated heterocyclyl group, an aziridinyl group, an epoxy group, a pyrrolidinyl group, a tetrahydrofuranyl group, a thiazolidinyl group, a piperidyl group, a piperazinyl group, a morpholinyl group, a dioxolanyl group, a dioxanyl group or the like may be exemplified.

As the 5-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group or the like may be exemplified.

As the 6-membered heteroaryl group, a pyridyl group, a pyrazinyl group, a pyrimidinyl group, a pyridazinyl group, a triazinyl group or the like may be exemplified.

[A] In the formula (I), A represents CH or a nitrogen atom.
Specifically, the compound represented by the formula (I) is a compound represented by the formula (I-1) or the formula (1-2).

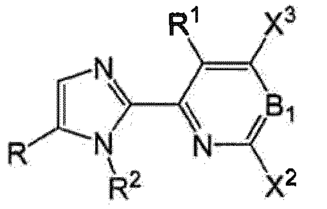
(I-1)

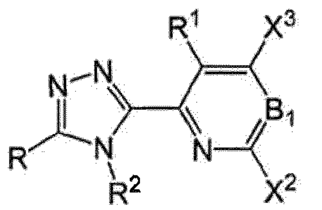

(I-2)

In the formula (I-1) and the formula (1-2), $B^1$, $R^1$, $R^2$, R, $X^2$ and $X^3$ have the same meanings as described in the formula (I).

[$B^1$] In the formula (I), $B^1$ represents $CX^1$ or a nitrogen atom.

Specifically, the compound represented by the formula (I) is a compound represented by the formula (1-3) or the formula (1-4).

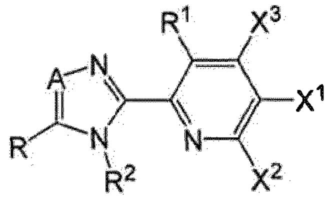

(I-3)

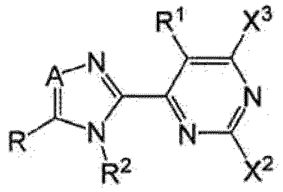

(I-4)

In the formula (1-3) and the formula (1-4), A, $R^1$, $R^2$, R, $X^2$ and $X^3$ have the same meanings as described in the formula (I).

In the formula (1-3), $X^1$ represents a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$. $R^3$ and $R^4$ each independently represent a hydrogen atom, a C1-6 alkyl group, or a C1-6 haloalkyl group. $R^5$, $R^6$, $R^7$ and $R^8$ each represent a C1-6 alkyl group. q represents 0 or 1.

The "C1-6 alkyl group" in $X^1$ may be linear or branched. As the "C1-6 alkyl group", a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, an i-hexyl group or the like may be exemplified.

As the substituent on the "C1-6 alkyl group" in $X^1$, a halogeno group such as a fluoro group, a chloro group, a bromo group, and an iodo group; a cyano group; an alkoxycarbonyl group such as an ethoxycarbonyl group, or the like may be preferably exemplified.

As the "C2-6 alkenyl group" in $X^1$, a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group or the like may be exemplified.

As the "C2-6 alkynyl group" in $X^1$, an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a

2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group or the like may be exemplified.

As the "C1-6 alkoxy group" in $X^1$, a methoxy group, an ethoxy group, a n-propoxy group, an i-propoxy group, a n-butoxy group, a s-butoxy group, an i-butoxy group, a t-butoxy group or the like may be exemplified.

As the "C1-6 alkoxycarbonyl group" in $X^1$, a methoxycarbonyl group, an ethoxycarbonyl group, a n-propoxycarbonyl group, an i-propoxycarbonyl group, a n-butoxycarbonyl group, a t-butoxycarbonyl group or the like may be exemplified.

As the "C1-6 alkylthio group" in $X^1$, a methylthio group, an ethylthio group, a n-propylthio group, a n-butylthio group, a n-pentylthio group, a n-hexylthio group, an i-propylthio group, an i-butylthio group or the like may be exemplified.

As the "C1-6 alkylsulfinyl group" in $X^1$, a methylsulfinyl group, an ethylsulfinyl group, a t-butylsulfinyl group or the like may be exemplified.

As the "C1-6 alkylsulfonyl group" in $X^1$, a methylsulfonyl group, an ethylsulfonyl group, a t-butylsulfonyl group or the like may be exemplified.

As the substituents on the "C2-6 alkenyl group", the "C2-6 alkynyl group", the "C1-6 alkoxy group", the "C1-6 alkylthio group", the "C1-6 alkylsulfinyl group" and the "C1-6 alkylsulfonyl group" in $X^1$, a halogeno group such as a fluoro group, a chloro group, a bromo group, and an iodo group; and a C1-6 alkoxy group such as a methoxy group, an ethoxy group, a n-propoxy group, an i-propoxy group, a n-butoxy group, a s-butoxy group, an i-butoxy group, and a t-butoxy group may be preferably exemplified.

As the "C3-8 cycloalkyl group" in $X^1$, a cyclopropyl group, a cyclobutyl group, a cyclopentyl group, a cyclohexyl group, a cubanyl group or the like may be exemplified.

As the "C6-10 aryl group" in $X^1$, a phenyl group, a naphthyl group, an indenyl group, an indanyl group, a tetralinyl group or the like may be exemplified.

The "3- to 6-membered heterocyclyl group" in $X^1$ contains 1 to 4 heteroatoms selected from the group consisting of a nitrogen atom, an oxygen atom and a sulfur atom as ring-constituting atoms. The heterocyclyl group may be either monocyclic or polycyclic. The polycyclic heterocyclyl group has at least one hetero ring, and the remaining ring(s) may be any of a saturated alicyclic ring, an unsaturated alicyclic ring and an aromatic ring. As the "3- to 6-membered heterocyclyl group", a 3- to 6-membered saturated heterocyclyl group, a 5- or 6-membered heteroaryl group, a 5- or 6-membered partially unsaturated heterocyclyl group or the like may be exemplified.

As the 3- to 6-membered saturated heterocyclyl group, an aziridinyl group, an epoxy group, a pyrrolidinyl group, a tetrahydrofuranyl group, a thiazolidinyl group, a piperidyl group, a piperazinyl group, a morpholinyl group, a dioxolanyl group, a dioxanyl group or the like may be exemplified.

As the 5- or 6-membered partially unsaturated heterocyclyl group, a 2-oxopyridin-1(2H)-yl group or the like may be exemplified.

As the 5-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group or the like may be exemplified.

As the 6-membered heteroaryl group, a pyridyl group, a pyrazinyl group, a pyrimidinyl group, a pyridazinyl group, a triazinyl group or the like may be exemplified.

As the "C6-10 aryloxy group" in $X^1$, a phenoxy group, a naphthyloxy group or the like may be exemplified.

As the "5- or 6-membered heteroaryloxy group" in $X^1$, a pyridyloxy group, a pyrimidyloxy group or the like may be exemplified.

As the substituents on the "C3-8 cycloalkyl group", the "C6-10 aryl group", the "3- to 6-membered heterocyclyl group", the "C6-10 aryloxy group", and the "5- or 6-membered heteroaryloxy group" in $X^1$, a halogeno group such as a fluoro group, a chloro group, a bromo group, and an iodo group; a Cl-6 alkyl group such as a methyl group, an ethyl group, a n-propyl group, an i-propyl group, a n-butyl group, a s-butyl group, an i-butyl group, a t-butyl group, a n-pentyl group, and a n-hexyl group; a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group; a C1-6 alkoxy group such as a methoxy group, an ethoxy group, a n-propoxy group, an i-propoxy group, a n-butoxy group, a s-butoxy group, an i-butoxy group, and a t-butoxy group; a C1-6 haloalkoxy group such as a 2-chloro-n-propoxy group, a 2,3-dichlorobutoxy group, and a trifluoromethoxy group; and an amino group may be preferably exemplified.

The "substituted or unsubstituted amino group" in $X^1$ is a group represented by "$-NR^aR^b$" or the like. In the formula, $R^a$ and $R^b$ each independently represent a hydrogen atom, a C1-6 alkyl group, a C3-8 cycloalkyl group, a formyl group, a C1-6 alkylcarbonyl group, a C3-8 cycloalkylcarbonyl group, or a substituted or unsubstituted aminocarbonyl

group.

As the "C1-6 alkyl group" in $R^a$ and $R^b$, the same group as listed in the above $X^1$ is exemplified.

As the "C3-8 cycloalkyl group" in $R^a$ and $R^b$, the same group as listed in the above $X^1$ is exemplified.

As the "C1-6 alkylcarbonyl group" in $R^a$ and $R^b$, an acetyl group, a propionyl group or the like may be exemplified.

As the "C3-8 cycloalkylcarbonyl group" in $R^a$ and $R^b$, a cyclopropylcarbonyl group, a cyclobutylcarbonyl group, a cyclopentylcarbonyl group or the like may be exemplified.

As the "substituted or unsubstituted aminocarbonyl group" in $R^a$ and $R^b$, an aminocarbonyl group, a methylaminocarbonyl group, an ethylaminocarbonyl group, a dimethylaminocarbonyl group or the like may be exemplified.

As the "substituted or unsubstituted aminocarbonyl group" in $X^1$, the same group as listed in the above $R^a$ and $R^b$ is exemplified.

The "substituted or unsubstituted hydrazinyl group" in $X^1$ is a group represented by the formula (a) or the like.

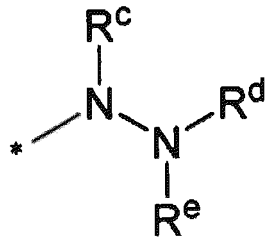

(a)

In the formula (a), * is a bonding position, and $R^c$, $R^d$ and $R^e$ each independently represent a hydrogen atom, a C1-6 alkyl group, or a substituted or unsubstituted phenylsulfonyl group or the like.

As the "C1-6 alkyl group" in $R^c$, $R^d$ and $R^e$, the same group as listed in the above $X^1$ is exemplified.

As the "substituted phenylsulfonyl group" in $R^c$, $R^d$ and $R^e$, a p-toluenesulfonyl group or the like may be exemplified.

As the "halogeno group" in $X^1$, a fluoro group, a chloro group, a bromo group, an iodo group or the like may be exemplified.

As the "C1-6 alkyl group" in $R^3$, $R^4$, $R^5$, $R^6$, $R^7$ and $R^8$ of the "group represented by the formula: $-CR^3=N-OR^4$", the "group represented by the formula: $-N=CHNR^5R^6$" and the "group represented by the formula: $-N=S(O)_qR^7R^8$" in $X^1$, the same group as listed in the above $X^1$ is exemplified.

As the "C1-6 haloalkyl group" in $R^3$ and $R^4$, a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, a 2,4,6-trichlorohexyl group, or the like may be exemplified.

Specifically, the compound represented by the formula (I) is a compound represented by the formula (1-5), the formula (1-6), the formula (1-7) or the formula (1-8).

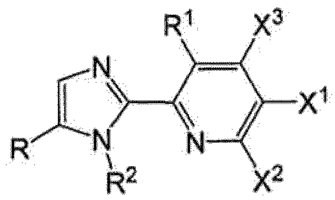

(I-5)

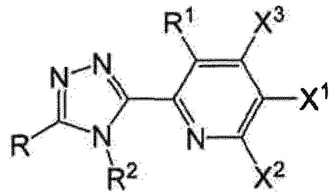

(I-6)

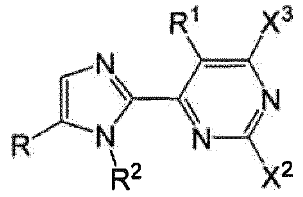

(I-7)

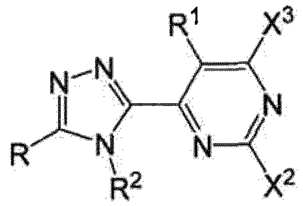

(I-8)

In the formula (1-5), the formula (1-6), the formula (I-7) and the formula (1-8), $R^1$, $R^2$, R, $X^2$ and $X^3$ have the same meanings as described in the formula (I).

In the formula (1-5) and the formula (1-6), $X^1$ has the same meaning as described in the formula (1-3).

[$X^2$ and $X^3$]

$X^2$ and $X^3$ each independently represent a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$. $R^3$ and $R^4$ each independently represent a hydrogen atom, a C1-6 alkyl group, or a C1-6 haloalkyl group. $R^5$, $R^6$, $R^7$ and $R^8$ each represent a C1-6 alkyl group. q represents 0 or 1.

As the "substituted or unsubstituted C1-6 alkyl group", the "substituted or unsubstituted C2-6 alkenyl group", the "substituted or unsubstituted C2-6 alkynyl group", the "substituted or unsubstituted Cl-6 alkoxy group", the "substituted or unsubstituted C1-6 alkoxycarbonyl group", the "substituted or unsubstituted C1-6 alkylthio group", the "substituted or unsubstituted C1-6 alkylsulfinyl group", the "substituted or unsubstituted C1-6 alkylsulfonyl group", the "substituted or unsubstituted C3-8 cycloalkyl group", the "substituted or unsubstituted C6-10 aryl group", the "substituted or unsubstituted 3- to 6-membered heterocyclyl group", the "substituted or unsubstituted C6-10 aryloxy group", the "substituted or unsubstituted 5- or 6-membered heteroaryloxy group", the "substituted or unsubstituted amino group", the "substituted or unsubstituted aminocarbonyl group", the "substituted or unsubstituted hydrazinyl group" and the "halogeno group" in $X^2$ and $X^3$, the same group as listed in the above $X^1$ is exemplified.

The "C1-6 alkyl group" and the "C1-6 haloalkyl group" in $R^3$ and $R^4$ in the formula: $-CR^3=N-OR^4$ have the same meanings as already described.

The "C1-6 alkyl group" in $R^5$ and $R^6$ of the formula: $-N=CHNR^5R^6$ has the same meaning as already described.

The "C1-6 alkyl group" in $R^7$ and $R^8$ of the formula: $-N=S(O)_qR^7R^8$ has the same meaning as already described.

[$R^1$]

In the formula (I), $R^1$ represents a substituted or unsubstituted C1-6 alkylthio group, a substituted or unsubstituted C1-6 alkylsulfinyl group, or a substituted or unsubstituted C1-6 alkylsulfonyl group.

As the "C1-6 alkylthio group", the "C1-6 alkylsulfinyl group" and the "C1-6 alkylsulfonyl group" in $R^1$, the same group as listed in the above $X^1$ is exemplified.

As the substituents on the "C1-6 alkylthio group", the "C1-6 alkylsulfinyl group" and the "C1-6 alkylsulfonyl group" in $R^1$, a halogeno group such as a fluoro group, a chloro group, a bromo group, and an iodo group may be preferably exemplified.

[$R^2$]

In the formula (I), $R^2$ represents a substituted or unsubstituted C1-6 alkyl group.

As the "C1-6 alkyl group" in $R^2$, the same group as listed in the above $X^1$ is exemplified.

As the substituent on the "C1-6 alkyl group" represented by $R^2$, a halogeno group such as a fluoro group, a chloro group, a bromo group, and an iodo group may be preferably exemplified.

[R]

In the formula (I), R represents a substituted or unsubstituted C1-6 alkyl group.

As the "C1-6 alkyl group" in R, the same group as listed in the above $X^1$ is exemplified.

As the "substituted C1-6 alkyl group" in R, a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a 2,2,3,3,4,4,5,5,5-nonafluoropentyl group, a 1-chloro-2,2,3,3,4,4,5,5,5-nonafluoropentyl group, a 1,2,2,3,3,4,4,5,5,5-decafluoropentyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group; a hydroxy group-substituted C1-6 haloalkyl group such as a 1-hydroxy-2,2,3,3,4,4,5,5,5-nonafluoropentyl group; a C1-6 alkylcarbonyloxy group-substituted C1-6 haloalkyl group such as a 1-acetyloxy-2,2,3,3,4,4,5,5,5-nonafluoropentyl group; a C1-6 haloalkoxy C1-6 alkyl group such as a (2,2,3,3,3-pentafluoropropoxy)methyl group and a (2,2,3,3,4,4,4-heptafluorobutoxy)methyl group; and a C1-6 haloalkoxy C1-6 haloalkoxy C1-6 alkyl group such as a ((1,1,2-trifluoro-2-(trifluoromethoxy)ethoxy)methyl group may be exemplified.

As the substituent on the "C1-6 alkyl group" in R, a halogeno group such as a fluoro group, a chloro group, a bromo group, and an iodo group; a hydroxy group; a C1-6 haloalkoxy group such as a trifluoromethoxy group, a 2-chloro-n-propoxy group, a 2,3-dichlorobutoxy group, a 2,2,3,3,3-pentafluoropropoxy group, and a 2,2,3,3,4,4,4-heptafluorobutoxy group; and a C1-6 alkylcarbonyloxy group such as an acetyloxy group may be preferably exemplified.

[Salt]

The salt of the compound (I) is not particularly limited as long as the salt is agriculturally or horticulturally acceptable. As the salt of the compound (I), for example, a salt of an inorganic acid such as hydrochloric acid and sulfuric acid; a salt of an organic acid such as acetic acid and lactic acid; a salt of an alkali metal such as lithium, sodium and potassium; a salt of an alkaline earth metal such as calcium and magnesium; a salt of a transition metal such as iron and copper; a salt of an organic base such as triethylamine, tributylamine, pyridine, and hydrazine; an ammonium salt, or the like may be exemplified.

[Production method]

The production method of the compound (I), the N-oxide compound, the stereoisomer, the tautomer, the hydrate or the salt of any of these compounds is not particularly limited. For example, these compounds may be obtained by known production methods described in Examples, etc. Alternatively, the N-oxide compound, salt, or the like of the compound (I) may be obtained by a known method from the compound (I).

[Compound represented by formula (II)]

The compound represented by the formula (I) is preferably a compound represented by the formula (II).

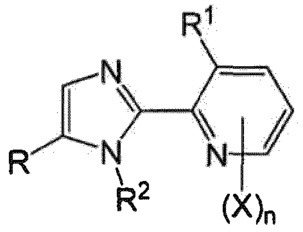

(II)

In the formula (II),

$R^1$, $R^2$, and R have the same meanings as described in the formula (I).

X represents a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$.

n represents a chemically acceptable number of X and is any integer of 0 to 3, and when n is 2 or larger, each of the X is the same or different.

As the "substituted or unsubstituted C1-6 alkyl group", the "substituted or unsubstituted C2-6 alkenyl group", the "substituted or unsubstituted C2-6 alkynyl group", the "substituted or unsubstituted C1-6 alkoxy group", the "substituted or unsubstituted C1-6 alkoxycarbonyl group", the "substituted or unsubstituted Cl-6 alkylthio group", the "substituted or unsubstituted C1-6 alkylsulfinyl group", the "substituted or unsubstituted C1-6 alkylsulfonyl group", the "substituted or unsubstituted C3-8 cycloalkyl group", the "substituted or unsubstituted C6-10 aryl group", the "substituted or unsubstituted 3- to 6-membered heterocyclyl group", the "substituted or unsubstituted C6-10 aryloxy group", the "substituted or unsubstituted 5- or 6-membered heteroaryloxy group", the "substituted or unsubstituted amino group", the "substituted or unsubstituted aminocarbonyl group", the "substituted or unsubstituted hydrazinyl group" and the "halogeno group" in X, the same group as listed in the above $X^1$ is exemplified.

The "C1-6 alkyl group" and the "C1-6 haloalkyl group" in $R^3$ and $R^4$ of the formula: $-CR^3=N-OR^4$ have the same meanings as already described.

The "C1-6 alkyl group" in $R^5$ and $R^6$ of the formula: $-N=CHNR^5R^6$ has the same meaning as already described.

The "C1-6 alkyl group" in $R^7$ and $R^8$ of the formula: $-N=S(O)_qR^7R^8$ has the same meaning as already described.

In the formula (II), $R^1$ is preferably a C1-6 alkylsulfonyl group, and $R^2$ is preferably a C1-6 alkyl group.

In the formula (II), R is preferably a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a 2,2,3,3,4,4,5,5,5-nonafluoropentyl group, a 1-chloro-2,2,3,3,4,4,5,5,5-nonafluoropentyl group, a 1,2,2,3,3,4,4,5,5,5-decafluoropentyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, or a 2,4,6-trichlorohexyl group, and more preferably a C1-6 fluoroalkyl group such as a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1,2,3,3,3-pentafluoro-2-trifluoromethylpropyl group, a 2,2,3,3,4,4,5,5,5-nonafluoropentyl group, a 1,2,2,3,3,4,4,5,5,5-decafluoropentyl group, a perfluoropentyl group, or a perfluorohexyl group.

In the formula (II), X is preferably a halogeno group, a substituted or unsubstituted C1-6 alkyl group, a substituted or unsubstituted C1-6 alkoxy group, a formyl group, a C3-8 cycloalkyl group, a substituted or unsubstituted C6-10 aryl group, a substituted or unsubstituted 3- to 6-membered heterocyclyl group, a substituted or unsubstituted 5- or 6-membered heteroaryloxy group, a substituted or unsubstituted amino group, or a group represented by the formula: "-

$CR^3$=N-$OR^{4}$", more preferably a substituted or unsubstituted C1-6 alkyl group, a C3-8 cycloalkyl group, a substituted or unsubstituted phenyl group, or a substituted or unsubstituted 6-membered heteroaryl group, and particularly preferably a C3-8 cycloalkyl group, a substituted or unsubstituted phenyl group, or a substituted or unsubstituted 6-membered heteroaryl group.

In the formula (II), n is preferably 1.

[Compound represented by formula (III)]

The compound represented by the formula (I) is preferably a compound represented by the formula (III).

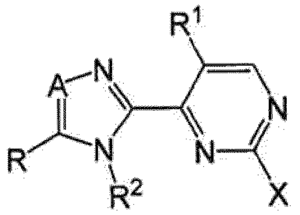

(III)

In the formula (III), A, $R^1$, $R^2$, and R have the same meanings as described in the formula (I).

X has the same meaning as described in the formula (II).

In the formula (III), $R^1$ is preferably a C1-6 alkylsulfonyl group. $R^2$ is preferably a C1-6 alkyl group. R is preferably a C1-6 fluoroalkyl group. X is preferably a substituted or unsubstituted 5- or 6-membered heteroaryl group, and more preferably a substituted or unsubstituted 5-membered heteroaryl group.

The compound represented by the formula (I) is preferably any of the following compounds.

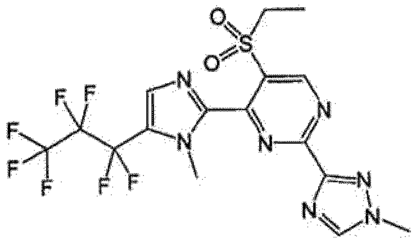

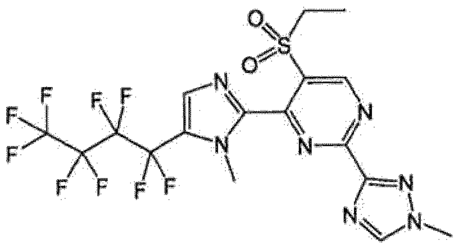

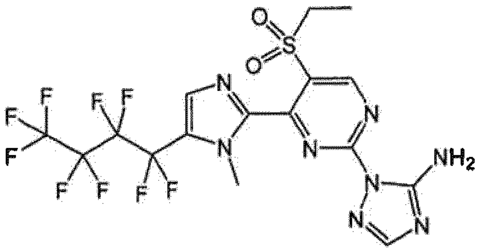

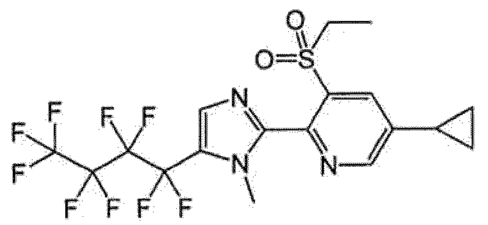

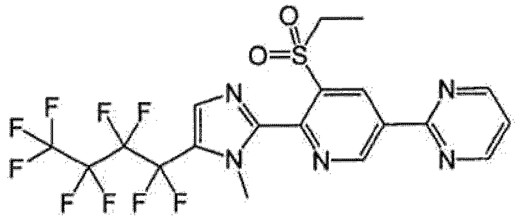

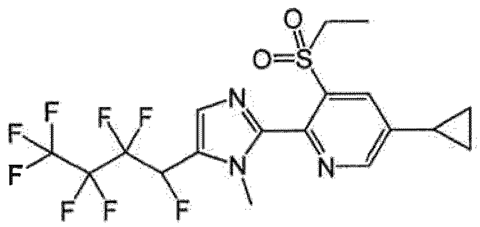

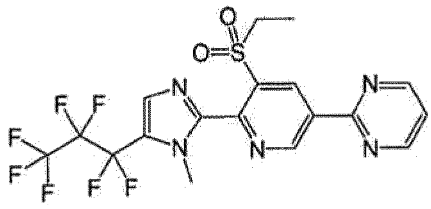

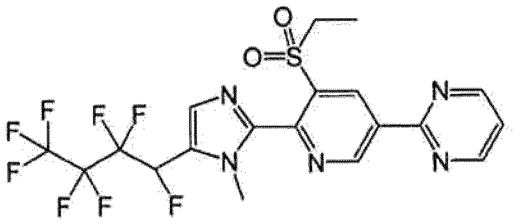

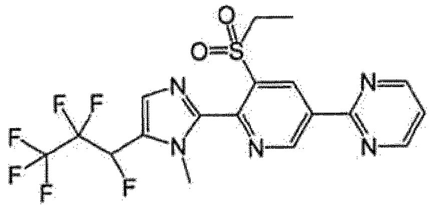

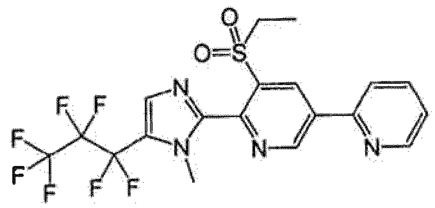

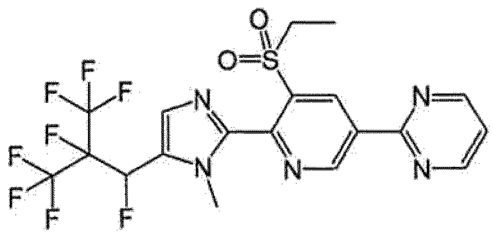

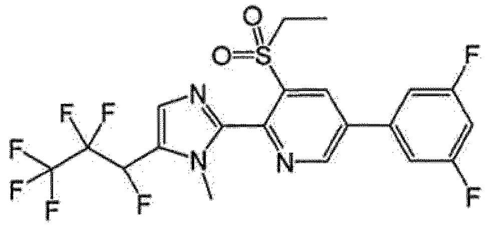

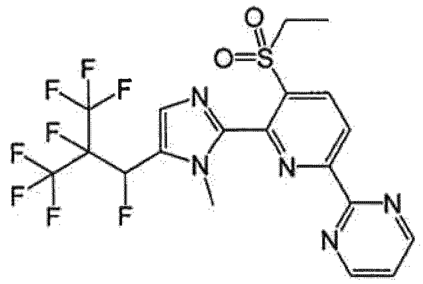

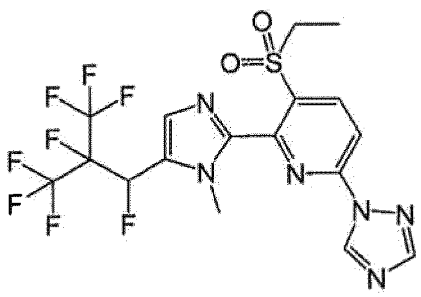

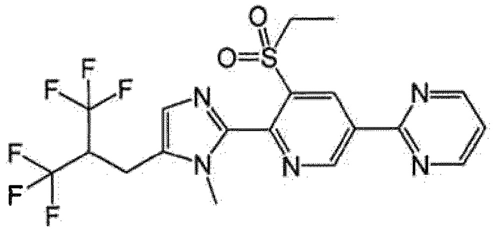

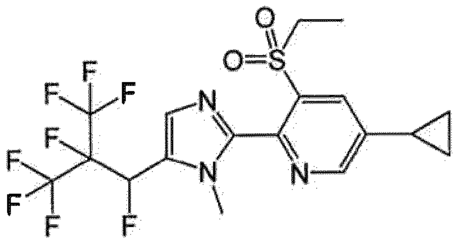

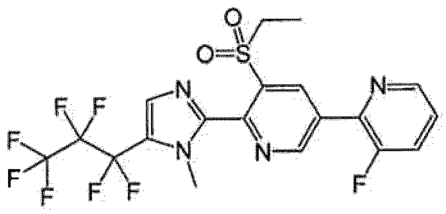

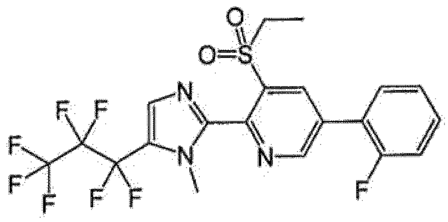

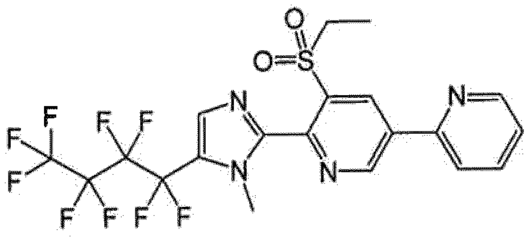

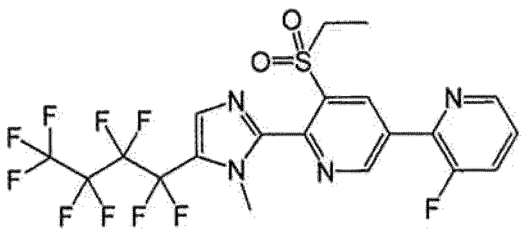

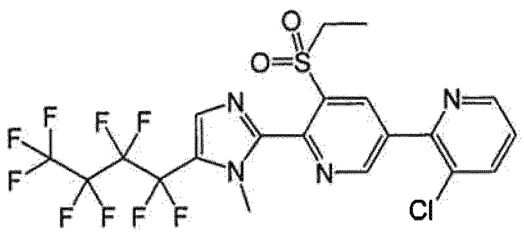

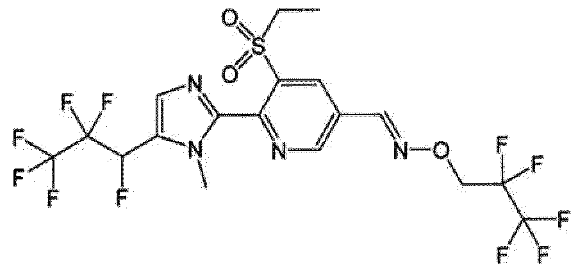

The heteroaryl azole compound of the present invention is excellent in control effect on pests such as various agricultural insect pests and mites affecting the growth of plants.

Also, the heteroaryl azole compound of the present invention is a highly safe substance because of less phytotoxicity to crops and low toxicity to fishes and warm-blooded animals. Hence, the heteroaryl azole compound of the present invention is useful as an active ingredient for insecticides or miticides.

Furthermore, in recent years, many insect pests such as diamondback moth, white-backed plant hopper, leafhopper, and aphid have developed resistance to various existing chemicals, causing problems of insufficient efficacy of these chemicals. Thus, chemicals effective for insect pests of resistant strains have been desired. The heteroaryl azole compound of the present invention exhibits an excellent control effect not only on sensitive strains but also on insect pests of various resistant strains and even mites of miticide-resistant strains.

The heteroaryl azole compound of the present invention is excellent in control effect on ectoparasites and endoparasites harmful to humans and animals. Also, the heteroaryl azole compound of the present invention is a highly safe substance because of low toxicity to fishes and warm-blooded animals. Hence, the heteroaryl azole compound of the present invention is useful as an active ingredient for ectoparasite and endoparasite control agents.

The heteroaryl azole compound of the present invention exhibits efficacy at every developmental stage of organisms to be controlled, and exhibits an excellent control effect on, for example, eggs, nymphs, larvae, pupae, and adults of mites, insects, and the like.

[Pest control agent]

The pest control agent of the present invention contains at least one active ingredient selected from the heteroaryl azole compounds of the present invention. The amount of the heteroaryl azole compound contained in the pest control agent of the present invention is not particularly limited as long as its pest control effect is exhibited. The pest control agent is an agent controlling pests and includes an insecticide or miticide, an ectoparasite control agent, or an endoparasite control agent or expellant, or the like.

[Insecticide or miticide]

The insecticide or miticide of the present invention contains at least one active ingredient selected from the heteroaryl azole compounds of the present invention. The amount of the heteroaryl azole compound contained in the insecticide or miticide of the present invention is not particularly limited as long as its insecticidal or miticidal effect is exhibited.

The pest control agent or the insecticide or miticide of the present invention is preferably used for plants such as cereals; vegetables; root vegetables; tubers and roots; flowers and ornamental plants; fruit trees; ornamental foliage plants and trees of tea, coffee, cacao, and the like; feed crops; lawn grasses; and cotton.

In the application to plants, the pest control agent or the insecticide or miticide of the present invention may be used for any site such as a leaf, a stem, a stalk, a flower, a bud, a fruit, a seed, a sprout, a root, a tuber, a tuberous root, a shoot, or a slip.

The pest control agent or the insecticide or miticide of the present invention is not particularly limited by the species of the plant to which the pest control agent or the insecticide or miticide is applied. As the plant species, for example, an original species, a variant species, an improved variety, a cultivar, a mutant, a hybrid, a genetically modified organism (GMO) or the like may be exemplified.

The pest control agent of the present invention may be used in seed treatment, foliage application, soil application, submerged application, or the like in order to control various agricultural insect pests and mites.

Specific examples of various agricultural insect pests and mites controllable with the pest control agent of the present invention will be shown below.

(1) Butterflies or moths of the order *Lepidoptera*

(a) moths of the family *Arctiidae,* for example, *Hyphantria* cunea and *Lemyra imparilis;*
(b) moths of the family *Bucculatricidae,* for example, *Bucculatrix pyrivorella;*
(c) moths of the family *Carposinidae,* for example, *Carposina sasakii;*
(d) moths of the family *Crambidae,* for example, *Diaphania indica* and *Diaphania nitidalis* of *Diaphania* spp.; for example, *Ostrinia furnacalis, Ostrinia nubilalis,* and *Ostrinia scapulalis* of *Ostrinia* spp.; and *Chilo suppressalis, Cnaphalocrocis medinalis, Conogethes punctiferalis, Diatraea grandiosella, Glyphodes pyloalis, Hellula undalis,* and *Parapediasia teterrella;*
(e) moths of the family *Gelechiidae,* for example, *Helcystogramma triannulella, Pectinophora gossypiella, Phthorimaea operculella,* and *Sitotroga cerealella;*
(f) moths of the family *Geometridae,* for example, *Ascotis selenaria;*
(g) moths of the family *Gracillariidae,* for example, *Caloptilia theivora, Phyllocnistis citrella,* and *Phyllonorycter ringoniella;*
(h) butterflies of the family *Hesperiidae,* for example, *Parnara guttata;*
(i) moths of the family *Lasiocampidae,* for example, *Malacosoma neustria;*
(j) moths of the family *Lymantriidae,* for example, *Lymantria dispar* and *Lymantria monacha* of *Lymantria* spp.; and *Euproctis pseudoconspersa* and *Orgyia thyellina;*
(k) moths of the family Lyonetiidae, for example, *Lyonetia clerkella* and *Lyonetia prunifoliella malinella* of *Lyonetia* spp.;
(l) moths of the family *Noctuidae,* for example, *Spodoptera depravata, Spodoptera eridania, Spodoptera exigua, Spodoptera frugiperda, Spodoptera littoralis,* and *Spodoptera litura* of *Spodoptera* spp.; for example, *Autographa gamma* and *Autographa nigrisigna* of *Autographa* spp.; for example, *Agrotis ipsilon* and *Agrotis segetum* of *Agrotis* spp.; for example, *Helicoverpa armigera, Helicoverpa assulta,* and *Helicoverpa zea* of *Helicoverpa* spp.; for example, *Heliothis armigera* and *Heliothis virescens* of *Heliothis* spp.; and *Aedia leucomelas, Ctenoplusia agnata, Eudocima tyrannus, Mamestra brassicae, Mythimna separata, Naranga aenescens, Panolis japonica, Peridroma saucia, Pseudoplusia includens,* and *Trichoplusia ni;*
(m) moths of the family *Nolidae,* for example, *Earias insulana;*
(n) butterflies of the family *Pieridae,* for example, *Pieris brassicae* and *Pieris rapae crucivora* of *Pieris* spp. ;
(o) moths of the family *Plutellidae,* for example, *Acrolepiopsis sapporensis* and *Acrolepiopsis suzukiella* of *Acrolepiopsis* spp.; and *Plutella xylostella;*
(p) moths of the family *Pyralidae,* for example, *Cadra cautella, Elasmopalpus lignosellus, Etiella zinckenella,* and *Galleria mellonella;*
(q) moths of the family *Sphingidae,* for example, *Manduca quinquemaculata* and *Manduca sexta* of *Manduca* spp.;
(r) moths of the family *Stathmopodidae,* for example, *Stathmopoda masinissa;*
(s) moths of the family *Tineidae,* for example, *Tinea translucens;*
(t) moths of the family *Tortricidae,* for example, *Adoxophyes honmai* and *Adoxophyes orana* of *Adoxophyes* spp.; for example, *Archips breviplicanus* and *Archips fuscocupreanus* of *Archips* spp.; and *Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana,* and *Sparganothis pilleriana;*
(u) moths of the family *Yponomeutidae,* for example, *Argyresthia conjugella.*

(2) Insect pests of the order *Thysanoptera*

(a) insect pests of the family *Phlaeothripidae,* for example, *Ponticulothrips diospyrosi;*
(b) insect pests of the family *Thripidae,* for example, *Frankliniella intonsa* and *Frankliniella occidentalis* of *Frankliniella* spp.; for example, *Thrips palmi* and *Thrips tabaci* of *Thrips* spp.; and *Heliothrips haemorrhoidalis* and *Scirtothrips dorsalis.*

(3) Insect pests of the order *Hemiptera*

(A) the suborder *Archaeorrhyncha*

(a) insect pests of the family *Delphacidae,* for example, *Laodelphax striatella, Nilaparvata lugens, Perkinsiella saccharicida,* and *Sogatella furcifera.*

(B) the suborder *Clypeorrhyncha*

(a) insect pests of the family *Cicadellidae,* for example, *Empoasca fabae, Empoasca nipponica, Empoasca*

*onukii,* and *Empoasca sakaii* of *Empoasca* spp.; and *Arboridia apicalis, Balclutha saltuella, Epiacanthus stramineus, Macrosteles striifrons,* and *Nephotettix cinctinceps.*

(C) the suborder *Heteroptera*

(a) insect pests of the family *Alydidae,* for example, *Riptortus clavatus;*
(b) insect pests of the family *Coreidae,* for example, *Cletus punctiger* and *Leptocorisa chinensis;*
(c) insect pests of the family *Lygaeidae,* for example, *Blissus leucopterus, Cavelerius saccharivorus,* and *Togo hemipterus;*
(d) insect pests of the family *Miridae,* for example, *Halticus insularis, Lygus lineolaris, Psuedatomoscelis seriatus, Stenodema sibiricum, Stenotus rubrovittatus,* and *Trigonotylus caelestialium;*
(e) insect pests of the family *Pentatomidae,* for example, *Nezara antennata* and *Nezara viridula* of *Nezara* spp.; for example, *Eysarcoris aeneus, Eysarcoris lewisi,* and *Eysarcoris ventralis* of *Eysarcoris* spp.; and *Dolycoris baccarum, Eurydema rugosum, Glaucias subpunctatus, Halyomorpha halys, Piezodorus hybneri, Plautia crossota,* and *Scotinophora lurida;*
(f) insect pests of the family *Pyrrhocoridae,* for example, *Dysdercus cingulatus;*
(g) insect pests of the family *Rhopalidae,* for example, *Rhopalus msculatus;*
(h) insect pests of the family *Scutelleridae,* for example, *Eurygaster integriceps;*
(i) insect pests of the family *Tingidae,* for example, *Stephanitis nashi.*

(D) the suborder *Sternorrhyncha*

(a) insect pests of the family *Adelgidae,* for example, *Adelges laricis;*
(b) insect pests of the family *Aleyrodidae,* for example, *Bemisia argentifolii* and *Bemisia tabaci* of *Bemisia* spp.; and *Aleurocanthus spiniferus, Dialeurodes citri,* and *Trialeurodes vaporariorum;*
(c) insect pests of the family *Aphididae,* for example, *Aphis craccivora, Aphis fabae, Aphis forbesi, Aphis gossypii, Aphis pomi, Aphis sambuci,* and *Aphis spiraecola* of *Aphis* spp.; for example, *Rhopalosiphum maidis* and *Rhopalosiphum padi* of *Rhopalosiphum* spp.; for example, *Dysaphis plantaginea* and *Dysaphis radicola* of *Dysaphis* spp.; for example, *Macrosiphum avenae* and *Macrosiphum euphorbiae* of *Macrosiphum* spp.; for example, *Myzus cerasi, Myzus persicae,* and *Myzus varians* of *Myzus* spp.; and *Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae,* and *Toxoptera aurantii;*
(d) insect pests of the family *Coccidae,* for example, *Ceroplastes ceriferus* and *Ceroplastes rubens* of *Ceroplastes* spp.;
(e) insect pests of the family *Diaspididae, Pseudaulacaspis pentagona* and *Pseudaulacaspis prunicola* of *Pseudaulacaspis* spp.; for example, *Unaspis euonymi* and *Unaspis yanonensis* of *Unaspis* spp.; and *Aonidiella aurantii, Comstockaspis perniciosa, Fiorinia theae*, and *Pseudaonidia paeoniae;*
(f) insect pests of the family *Margarodidae,* for example, *Drosicha corpulenta* and *Icerya purchasi;*
(g) insect pests of the family *Phylloxeridae,* for example, *Viteus vitifolii;*
(h) insect pests of the family *Pseudococcidae,* for example, *Planococcus citri* and *Planococcus kuraunhiae* of *Planococcus* spp.; and *Phenacoccus solani* and *Pseudococcus comstocki;*
(i) insect pests of the family *Psyllidae,* for example, *Psylla mali* and *Psylla pyrisuga* of *Psylla* spp.; and *Diaphorina citri.*

(4) Insect pests of the suborder *Polyphaga*

(a) insect pests of the family *Anobiidae,* for example, *Lasioderma serricorne;*
(b) insect pests of the family *Attelabidae,* for example, *Byctiscus betulae* and *Rhynchites heros;*
(c) insect pests of the family *Bostrichidae,* for example, *Lyctus brunneus;*
(d) insect pests of the family *Brentidae,* for example, *Cylas formicarius;*
(e) insect pests of the family *Buprestidae,* for example, *Agrilus sinuatus;*
(f) insect pests of the family *Cerambycidae,* for example, *Anoplophora malasiaca, Monochamus alternatus, Psacothea hilaris,* and *Xylotrechus pyrrhoderus;*
(g) insect pests of the family *Chrysomelidae,* for example, *Bruchus pisorum* and *Bruchus rufimanus* of *Bruchus* spp.; for example, *Diabrotica barberi, Diabrotica undecimpunctata,* and *Diabrotica virgifera* of *Diabrotica* spp.; for example, *Phyllotreta nemorum* and *Phyllotreta striolata* of *Phyllotreta* spp.; and *Aulacophora femoralis, Callosobruchus chinensis, Cassida nebulosa, Chaetocnema concinna, Leptinotarsa decemlineata, Oulema*

*oryzae,* and *Psylliodes angusticollis;*
(h) insect pests of the family *Coccinellidae,* for example, *Epilachna varivestis* and *Epilachna vigintioctopunctata* of *Epilachna* spp.;
(i) insect pests of the family *Curculionidae,* for example, *Anthonomus grandis* and *Anthonomus pomorum* of *Anthonomus* spp.; for example, *Sitophilus granarius* and *Sitophilus zeamais* of *Sitophilus* spp.; and *Echinocnemus squameus, Euscepes postfasciatus, Hylobius abietis, Hypera postica, Lissohoptrus oryzophilus, Otiorhynchus sulcatus, Sitona lineatus,* and *Sphenophorus venatus;*
(j) insect pests of the family *Elateridae,* for example, *Melanotus fortnumi* and *Melanotus tamsuyensis* of *Melanotus* spp. ;
(k) insect pests of the family *Nitidulidae,* for example, *Epuraea domina;*
(l) insect pests of the family *Scarabaeidae,* for example, *Anomala cuprea* and *Anomala rufocuprea* of *Anomala* spp.; and *Cetonia aurata, Gametis jucunda, Heptophylla picea, Melolontha melolontha,* and *Popillia japonica;*
(m) insect pests of the family *Scolytidae,* for example, *Ips typographus;*
(n) insect pests of the family *Staphylinidae,* for example, *Paederus fuscipes;*
(o) insect pests of the family *Tenebrionidae,* for example, *Tenebrio molitor* and *Tribolium castaneum;*
(p) insect pests of the family *Trogossitidae,* for example, *Tenebroides mauritanicus.*

(5) Insect pests of the order *Diptera*

(A) the suborder *Brachycera*

(a) insect pests of the family *Agromyzidae,* for example, *Liriomyza bryoniae, Liriomyza chinensis, Liriomyza sativae,* and *Liriomyza trifolii* of *Liriomyza* spp.; and *Chromatomyia horticola* and *Agromyza oryzae;*
(b) insect pests of the family *Anthomyiidae,* for example, *Delia platura* and *Delia radicum* of *Delia* spp.; and *Pegomya cunicularia;*
(c) insect pests of the family *Drosophilidae,* for example, *Drosophila melanogaster* and *Drosophila suzukii* of *Drosophila* spp.;
(d) insect pests of the family *Ephydridae,* for example, *Hydrellia griseola;*
(e) insect pests of the family *Psilidae,* for example, *Psila rosae;*
(f) insect pests of the family *Tephritidae,* for example, *Bactrocera cucurbitae* and *Bactrocera dorsalis* of *Bactrocera* spp.; for example, *Rhagoletis cerasi* and *Rhagoletis pomonella* of *Rhagoletis* spp.; and *Ceratitis capitata* and *Dacus oleae.*

(B) the suborder Nematocera

(a) insect pests of the family *Cecidomyiidae,* for example, *Asphondylia yushimai, Contarinia sorghicola, Mayetiola destructor,* and *Sitodiplosis mosellana.*

(6) Insect pests of the order *Orthoptera*

(a) insect pests of the family *Acrididae,* for example, *Schistocerca americana* and *Schistocerca gregaria* of *Schistocerca* spp.; and *Chortoicetes terminifera, Dociostaurus maroccanus, Locusta migratoria, Locustana pardalina, Nomadacris septemfasciata,* and *Oxya yezoensis;*
(b) insect pests of the family *Gryllidae,* for example, *Acheta domestica* and *Teleogryllus emma;*
(c) insect pests of the family *Gryllotalpidae,* for example, *Gryllotalpa orientalis;*
(d) insect pests of the family *Tettigoniidae,* for example, *Tachycines asynamorus.*

(7) *Acari*

(A) *Acaridida* of the order *Astigmata*

(a) mites of the family *Acaridae,* for example, *Rhizoglyphus echinopus* and *Rhizoglyphus robini* of *Rhizoglyphus* spp.; for example, *Tyrophagus neiswanderi, Tyrophagus perniciosus, Tyrophagus putrescentiae,* and *Tyrophagus similis* of *Tyrophagus* spp.; and *Acarus siro, Aleuroglyphus ovatus,* and *Mycetoglyphus fungivorus;*

(B) *Actinedida* of the order *Prostigmata*

(a) mites of the family *Tetranychidae,* for example, *Bryobia praetiosa* and *Bryobia rubrioculus* of *Bryobia* spp.; for example, *Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis,* and *Eotetranychus uncatus* of *Eotetranychus* spp.; for example, *Oligonychus hondoensis, Oligonychus ilicis, Oligonychus karamatus, Oligonychus mangiferus, Oligonychus orthius, Oligonychus perseae, Oligonychus pustulosus, Oligonychus shinkajii,* and *Oligonychus ununguis* of *Oligonychus* spp.; for example, *Panonychus citri, Panonychus mori,* and *Panonychus ulmi* of *Panonychus* spp.; for example, *Tetranychus cinnabarinus, Tetranychus kanzawai, Tetranychus ludeni, Tetranychus quercivorus, Tetranychus phaselus, Tetranychus urticae, Tetranychus viennensis,* and *Tetranychus evansi* of *Tetranychus* spp.; for example, *Aponychus corpuzae* and *Aponychus firmianae* of *Aponychus* spp.; for example, *Sasanychus akitanus* and *Sasanychus pusillus* of *Sasanychus* spp.; for example, *Schizotetranychus celarius, Schizotetranychus longus, Schizotetranychus miscanthi, Schizotetranychus recki,* and *Schizotetranychus schizopus* of *Schizotetranychus* spp.; and *Tetranychina harti, Tuckerella pavoniformis,* and *Yezonychus sapporensis;*

(b) mites of the family *Tenuipalpidae,* for example, *Brevipalpus lewisi, Brevipalpus obovatus, Brevipalpus phoenicis, Brevipalpus russulus,* and *Brevipalpus californicus* of *Brevipalpus* spp.; for example, *Tenuipalpus pacificus* and *Tenuipalpus zhizhilashviliae* of *Tenuipalpus* spp.; and *Dolichotetranychus floridanus;*

(c) mites of the family *Eriophyidae,* for example, *Aceria diospyri, Aceria ficus, Aceria japonica, Aceria kuko, Aceria paradianthi, Aceria tiyingi, Aceria tulipae,* and *Aceria zoysiea* of *Aceria* spp.; for example, *Eriophyes chibaensis* and *Eriophyes emarginatae* of *Eriophyes* spp.; for example, *Aculops lycopersici* and *Aculops pelekassi* of *Aculops* spp.; for example, *Aculus fockeui* and *Aculus schlechtendali* of *Aculus* spp.; and *Acaphylla theavagrans, Calacarus carinatus, Colomerus vitis, Calepitrimerus vitis, Epitrimerus pyri, Paraphytoptus kikus, Paracalacarus podocarpi,* and *Phyllocotruta citri;*

(d) mites of the family *Tarsonemidae,* for example, *Tarsonemus bilobatus* and *Tarsonemus waitei* of *Tarsonemus* spp.; and *Phytonemus pallidus* and *Polyphagotarsonemus latus;*

(e) mites of the family Penthaleidae, for example, *Penthaleus erythrocephalus* and *Penthaleus major* of *Penthaleus* spp.

The pest control agent of the present invention may be used as a mixture or in combination with another active ingredient such as a fungicide, an insecticide or miticide, a nematicide, or a pesticide for soil insect pests; a plant regulating agent, a synergist, a fertilizer, a soil improvement agent, animal feed, or the like.

The combination of the heteroaryl azole compound of the present invention with another active ingredient may be expected to have synergistic effects on insecticidal, miticidal, or nematicidal activity. The synergistic effects may be confirmed by the known method with the equation of Colby (Colby. S.R.; Calculating Synergistic and Antagonistic Responses of Herbicide Combinations; Weeds 15, p. 20-22, 1967) according to a standard method.

Specific examples of the insecticide or miticide, the nematicide, the pesticide for soil insect pests, the anthelmintic agent, and the like that may be used as a mixture or in combination with the pest control agent of the present invention will be shown below.

(1) Acetylcholinesterase inhibitors:

(a) carbamate-based: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, chloethocarb, metam sodium, and promecarb;

(b) organophosphorus-based: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, and sulprofos.

(2) GABAergic chloride ion channel antagonists: acetoprole, chlordene, endosulfan, ethiprole, fipronil, pyrafluprole, pyriprole, camphechlor, heptachlor, and dienochlor.
(3) Sodium channel modulators: acrinathrin, d-cis-trans allethrin, d-trans allethrin, bifenthrin, bioallethrin, bioallethrin S-cyclopentyl isomers, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomers], deltamethrin, empenthrin [(EZ)-(1R)-isomers], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomers], prallethrin, pyrethrum, resmethrin, silafluofen, tefluthrin, tetramethrin [(1R)-isomers], tralomethrin, transfluthrin, allethrin, pyrethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, and terallethrin.
(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, flupyradifurone, and flupyrimin.
(5) Nicotinic acetylcholine receptor allosteric modulators: spinetoram and spinosad.
(6) Chloride channel activators: abamectin, emamectin benzoate, lepimectin, milbemectin, ivermectin, selamectin, doramectin, eprinomectin, moxidectin, milbemycin, milbemycin oxime, and nemadectin.
(7) Juvenile hormone-like substances: hydroprene, kinoprene, methoprene, fenoxycarb, pyriproxyfen, diofenolan, epofenonane, and triprene.
(8) Other nonspecific inhibitors: methyl bromide, chloropicrin, sulfuryl fluoride, borax, and tartar emetic.
(9) Homoptera selective feeding inhibitors: flonicamid, pymetrozine, and pyrifluquinazon.
(10) Mite growth inhibitors: clofentezine, diflovidazin, hexythiazox, and etoxazole.
(11) Insect midgut inner membrane disrupting agents derived from microorganisms: *Bacillus thuringiensis* subsp. *Isuraerenshi, Bacillus sphaericus, Bacillus thuringiensis* subsp. *Aizawai, Bacillus thuringiensis* subsp. *Kurstaki, Bacillus thuringiensis* subsp. *Tenebrionis,* and Bt crop proteins: Cry1Ab, Cry1Ac, Cry1Fa, Cry1A.105, Cry2Ab, Vip3A, mCry3A, Cry3Ab, Cry3Bb, and Cry34Ab1/Cry35Ab1.
(12) Mitochondrial ATP biosynthetic enzyme inhibitors: diafenthiuron, azocyclotin, cyhexatin, fenbutatin oxide, propargite, and tetradifon.
(13) Oxidative phosphorylation uncouplers: chlorfenapyr, sulfluramid, DNOC, binapacryl, dinobuton, and dinocap.
(14) Nicotinic acetylcholine receptor channel blockers: bensultap, cartap hydrochloride, nereistoxin, thiosultap sodium, and thiocyclam.
(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron.
(16) Diptera molting disrupting agents: cyromazine.
(17) Molting hormone receptor agonists: chromafenozide, halofenozide, methoxyfenozide, and tebufenozide.
(18) Octopamine receptor agonists: amitraz, demiditraz, and chlordimeform.
(19) Mitochondrial electron transport system complex III inhibitors: acequinocyl, fluacrypyrim, and hydramethylnon.
(20) Mitochondrial electron transport system complex I inhibitors: fenazaquin, fenpyroximate, pyrimidifen, pyridaben, tebufenpyrad, tolfenpyrad, and rotenone.
(21) Voltage-gated sodium channel blockers: indoxacarb and metaflumizone.
(22) Acetyl CoA carboxylase inhibitors: spirodiclofen, spiromesifen, and spirotetramat.
(23) Mitochondrial electron transport system complex IV inhibitors: aluminum phosphide, calcium phosphide, phosphine, zinc phosphide, and cyanide.
(24) Mitochondrial electron transport system complex II inhibitors: cyenopyrafen, cyflumetofen, and pyflubumide.
(25) Ryanodine receptor modulators: chlorantraniliprole, cyantraniliprole, flubendiamide, cyclaniliprole, and tetraniliprole.
(26) Mixed function oxidase inhibitor compounds: piperonyl butoxide.
(27) Latrophilin receptor agonists: depsipeptide, cyclic depsipeptide, 24-membered cyclic depsipeptide, and emodepside.
(28) Other agents (based on an unknown mechanism of action): azadirachtin, benzoximate, bifenazate, bromopropylate, chinomethionate, cryolite, dicofol, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimin, fluphenazine, gossyplure, japonilure, metoxadiazone, petroleum, potassium oleate, tetrasul, triarathene, afidopyropen, flometoquin, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazol-3-yl]-2-(1H-1,2,4-triazol-1-yl)benzonitrile (CAS: 943137-49-3), broflanilide, and other m-diamides.
(29) Anthelmintic agents:

(a) benzimidazole-based: fenbendazole, albendazole, triclabendazole, oxibendazole, mebendazole, oxfendazole, parbendazole, flubendazole, febantel, netobimin, thiophanate, thiabendazole, and cambendazole;
(b) salicylanilide-based: closantel, oxyclozanide, rafoxanide, and niclosamide;
(c) substituted phenol-based: nitroxinil and nitroscanate;
(d) pyrimidine-based: pyrantel and morantel;
(e) imidazothiazole-based: levamisole and tetramisole;
(f) tetrahydropyrimidine-based: praziquantel and epsiprantel;
(g) other anthelmintic agents: cyclodiene, ryania, clorsulon, metronidazole, demiditraz, piperazine, diethylcarbamazine, dichlorophene, monepantel, tribendimidine, amidantel, thiacetarsamide, melarsomine, and arsenamide.

Specific examples of the fungicide that may be used as a mixture or in combination with the pest control agent of the present invention will be shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, and ofurace;
(b) adenosine deaminase inhibitors: bupirimate, dimethirimol, and ethirimol;
(c) DNA/RNA synthesis inhibitors: hymexazol and octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, and ethaboxam;
(b) cell division inhibitors: pencycuron;
(c) spectrin-like protein delocalization inhibitors: fluopicolide.

(3) Respiration inhibitors:

(a) complex I NADH oxidation-reduction enzyme inhibitors: diflumetorim and tolfenpyrad;
(b) complex II succinate dehydrogenase inhibitors: benodanil, flutolanil, mepronil, isofetamid, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, and boscalid;
(c) complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, and pyribencarb;
(d) complex III ubiquinol reductase Qi inhibitors: cyazofamid and amisulbrom;
(e) oxidative phosphorylation uncoupling agents: binapacryl, meptyldinocap, dinocap, fluazinam, and ferimzone;
(f) oxidative phosphorylation inhibitors (ATP synthase inhibitors): fentin acetate, fentin chloride, and fentin hydroxide;
(g) ATP production inhibitors: silthiofam;
(h) complex III: Qx (unknown) inhibitor of cytochrome bc1 (ubiquinone reductase): ametoctradin.

(4) Amino acid and protein synthesis inhibitors

(a) methionine biosynthesis inhibitors: andoprim, cyprodinil, mepanipyrim, and pyrimethanil;
(b) protein synthesis inhibitors: blasticidin-S, kasugamycin, kasugamycin hydrochloride, streptomycin, and oxytetracycline.

(5) Signal transduction inhibitors:

(a) signal transduction inhibitors: quinoxyfen and proquinazid;
(b) MAP/histidine kinase inhibitors in osmotic signal transduction: fenpiclonil, fludioxonil, chlozolinate, iprodione, procymidone, and vinclozolin.

(6) Lipid and cell membrane synthesis inhibitors:

(a) phospholipid biosynthesis, methyltransferase inhibitors: edifenphos, iprobenfos, pyrazophos, and isoprothiolane;
(b) lipid peroxidation agents: biphenyl, chloroneb, dicloran, quintozene, tecnazene, tolclofos-methyl, and etridiazole;
(c) agents that act on cell membranes: iodocarb, propamocarb, propamocarb hydrochloride, propamocarb fosetylate, and prothiocarb;
(d) microorganisms that disrupt cell membranes of pathogens: *Bacillus subtilis* bacteria, *Bacillus subtilis* QST713 strain, *Bacillus subtilis* FZB24 strain, *Bacillus subtilis* MBI600 strain, and *Bacillus subtilis* D747 strain; (e) agents that disrupt cell membranes: extracts of *Melaleuca alternifolia* (tea tree).

(7) Cell membrane sterol biosynthesis inhibitors:

(a) C14-demethylation inhibitors in sterol biosynthesis: triforine, pyrifenox, pyrisoxazole, fenarimol, flurprimidol, nuarimol, imazalil, imazalil sulfate, oxpoconazole, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, fluconazole-cis, hexaconazole, imibenconazole, ipconazole, metconazole, myclobutanil, penconazole, propiconazole, quinconazole, simeconazole, tebuconazole, tetraconazole, triadimefon, triadimenol, triticonazole, prothioconazole, and voriconazole;
(b) $\Delta 14$ reductase and sterol $\Delta 8 \rightarrow \Delta 7$-isomerase inhibitors in sterol biosynthesis: aldimorph, dodemorph, dodemorph acetate, fenpropimorph, tridemorph, fenpropidin, piperalin, and spiroxamine;
(c) 3-keto reductase inhibitors in C4-demethylation in the sterol biosynthesis system: fenhexamid and fenpyrazamine;
(d) squalene epoxidase inhibitors in the sterol biosynthesis system: pyributicarb, naftifine, and terbinafine.

(8) Cell wall synthesis inhibitors

(a) trehalase inhibitors: validamycin;
(b) chitin synthase inhibitors: polyoxin and polyoxorim;
(c) cellulose synthase inhibitors: dimethomorph, flumorph, pyrimorph, benthiavalicarb, iprovalicarb, tolprocarb, valifenalate, and mandipropamid.

(9) Melanin biosynthesis inhibitors

(a) melanin biosynthesis reductase inhibitors: fthalide, pyroquilon, and tricyclazole;
(b) melanin biosynthesis anhydrase inhibitors: carpropamid, diclocymet, and fenoxanil;

(10) Host plant resistance inducers:

(a) agents that act on salicylic acid synthesis pathway: acibenzolar-S-methyl;
(b) others: probenazole, tiadinil, isotianil, laminarin, and giant knotweed extracts.

(11) Agents with unknown mode of action: cymoxanil, fosetyl aluminum, phosphoric acid (phosphate), tecloftalam, triazoxide, flusulfamide, diclomezine, methasulfocarb, cyflufenamid, metrafenone, pyriofenone, dodine, dodine free base, and flutianil.
(12) Agents having multiple active sites: copper (copper salt), Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, iminoctadine triacetate, iminoctadine trialbesilate, anilazine, dithianon, chinomethionate, and fluoroimide.
(13) Other agents: DBEDC, fluoro folpet, guazatine acetate, bis(8-quinolinolato)copper(II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophene, difenzoquat, difenzoquat methyl sulfonate, flumetover, fosetyl calcium, fosetyl sodium, irumamycin, natamycin, nitrothal-isopropyl, oxamocarb, propanosine sodium, pyrrolnitrin, tebufloquin, tolnifanide, zarilamid, algophase, amicarthiazol, oxathiapiprolin, metiram zinc, benthiazole, trichlamide, uniconazole, oxyfenthiin, and picarbutrazox.

Specific examples of the plant regulating agent that may be used as a mixture or in combination with the pest

control agent of the present invention will be shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetylaminoethoxyvinylglycine (also called aviglycine), aminooxyacetic acid, silver nitrate, cobalt chloride, IAA, 4-CPA, cloprop, 2,4-D, MCPB, indole-3-butyric acid, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyl adenine, and 5-aminolevulinic acid.

[Ectoparasite control agent]

The ectoparasite control agent of the present invention contains at least one active ingredient selected from the heteroaryl azole compounds of the present invention. The amount of the heteroaryl azole compound contained in the ectoparasite control agent of the present invention is not particularly limited as long as its ectoparasite control effect is exhibited.

As the host animal to be treated with the ectoparasite control agent of the present invention, a warm-blooded animal such as a human and a livestock mammal (e.g., a cow, a horse, a pig, sheep, and a goat), a laboratory animal (e.g., a mouse, a rat, and a sand rat), a pet animal (e.g., a hamster, a guinea pig, a dog, a cat, a horse, a squirrel, a rabbit, and a ferret), wild and zoo mammals (e.g., a monkey, a fox, a deer, and a buffalo), a fowl (e.g., a turkey, a duck, a chicken, a quail, and a goose), and a pet bird (e.g., a pigeon, a parrot, a myna bird, a Java sparrow, a parakeet, a Bengalese finch, and a canary bird); and fishes such as salmon, trout, and carp may be exemplified. In addition, a bee, a stag beetle and a beetle may be exemplified.

The ectoparasite control agent of the present invention may be applied by a known veterinary method (local, oral, parenteral or subcutaneous administration). As the method therefor, a method of orally administering tablets, capsules, feed or the like containing the ectoparasite control agent to animals; a method of administering the ectoparasite control agent with dipping liquids, suppositories, injection (intramuscular, subcutaneous, intravenous, or intraperitoneal injection, etc.) or the like to animals; a method of locally administering oily or aqueous liquid formulations by spraying, pour-on, spot-on or the like; and a method of locally administering the ectoparasite control agent to animals by attaching to animals a material such as a collar or an ear tag produced by suitably shaping the resin into which the ectoparasite control agent is kneaded may be exemplified.

Ectoparasites parasitize the inside or the body surface of host animals, particularly, warm-blooded animals. Specifically, the ectoparasites parasitize the backs, armpits, lower abdomens, inner thighs, or the like of host animals and live by obtaining nutrients such as blood or dandruff from the animals. As the ectoparasite, mites, lice, fleas, a mosquito, a stable fly, a flesh fly or the like may be exemplified. Specific examples of the ectoparasite controllable with the ectoparasite control agent of the present invention will be shown below.

(1) *Acari*

mites of the family *Dermanyssidae,* mites of the family *Macronyssidae,* mites of the family *Laelapidae,* mites of the family *Varroidae,* mites of the family *Argasidae,* mites of the family *Ixodidae,* mites of the family *Psoroptidae,* mites of the family *Sarcoptidae,* mites of the family *Knemidokoptidae,* mites of the family *Demodixidae,* mites of the family *Trombiculidae,* and insect parasitic mites such as mites of the family *Canestriniidae.*

(2) The order *Phthiraptera*

lice of the family *Haematopinidae,* lice of the family *Linognathidae,* bird lice of the family *Menoponidae,* bird lice of the family *Philopteridae,* and bird lice of the family *Trichodectidae.*

(3) The order *Siphonaptera*

fleas of the family *Pulicidae,* for example, *Ctenocephalides canis* and *Ctenocephalides felis* of *Ctenocephalides* spp.; fleas of the family *Tungidae,* fleas of the family *Ceratophyllidae,* and fleas of the family *Leptopsyllidae.*

(4) The order *Hemiptera*

(5) Insect pests of the order *Diptera*

mosquitos of the family *Culicidae,* black flies of the family *Simuliidae,* biting midges of the family *Ceratopogonidae,* horseflies of the family *Tabanidae,* flies of the family *Muscidae,* tsetse flies of the family *Glossinidae,* flesh flies of the family *Sarcophagidae,* flies of the family *Hippoboscidae,* flies of the family *Calliphoridae,* and flies of the family *Oestridae.*

[Endoparasite control agent or expellant]

The endoparasite control agent or expellant of the present invention contains at least one active ingredient

selected from the heteroaryl azole compounds of the present invention. The amount of the heteroaryl azole compound contained in the endoparasite control agent or expellant of the present invention is not particularly limited as long as its endoparasite control effect is exhibited.

The parasite targeted by the endoparasite control agent or expellant of the present invention parasitizes the inside of host animals, particularly, warm-blooded animals or fishes (endoparasite). As the host animal for which the endoparasite control agent or expellant of the present invention is effective, a warm-blooded animal such as a human and a livestock mammal (e.g., a cow, a horse, a pig, sheep, and a goat), a laboratory animal (e.g., a mouse, a rat, and a sand rat), a pet animal (e.g., a hamster, a guinea pig, a dog, a cat, a horse, a squirrel, a rabbit, and a ferret), wild and zoo mammals (e.g., a monkey, a fox, a deer, and a buffalo), a fowl (e.g., a turkey, a duck, a chicken, a quail, and a goose), and a pet bird (e.g., a pigeon, a parrot, a myna bird, a Java sparrow, a parakeet, a Bengalese finch, and a canary bird); and fishes such as salmon, trout, and carp may be exemplified. Parasitic diseases mediated by parasites may be prevented or treated by controlling and expelling the parasites.

As the parasite to be controlled or expelled, the following may be exemplified.

(1) Nematodes of the order *Dioctophymatida*

(a) kidney worms of the family *Dioctophymatidae,* for example, *Dioctophyma renale* of *Dioctophyma* spp.;
(b) kidney worms of the family *Soboliphymatidae,* for example, *Soboliphyme abei* and *Soboliphyme baturini* of *Soboliphyme* spp.

(2) Nematodes of the order *Trichocephalida*

(a) trichinae of the family *Trichinellidae,* for example, *Trichinella spiralis* of *Trichinella* spp.;
(b) whipworms of the family *Trichuridae,* for example, *Capillaria annulata, Capillaria contorta, Capillaria hepatica, Capillaria perforans, Capillaria plica,* and *Capillaria suis* of *Capillaria* spp.; and *Trichuris vulpis, Trichuris discolor, Trichuris ovis, Trichuris skrjabini,* and *Trichuris suis* of *Trichuris* spp.

(3) Nematodes of the order *Rhabditida*
threadworms of the family *Strongyloididae,* for example, *Strongyloides papillosus, Strongyloides planiceps, Strongyloides ransomi, Strongyloides suis, Strongyloides stercoralis, Strongyloides tumefaciens,* and *Strongyloides* ratti of *Strongyloides* spp.
(4) Nematodes of the order *Strongylida*
hookworms of the family *Ancylostomatidae,* for example, *Ancylostoma braziliense, Ancylostoma caninum, Ancylostoma duodenale,* and *Ancylostoma tubaeforme* of *Ancylostoma* spp.; *Uncinaria stenocephala* of *Uncinaria* spp.; and *Bunostomum phlebotomum* and *Bunostomum trigonocephalum* of *Bunostomum* spp.
(5) Nematodes of the order *Strongylida*

(a) nematodes of the family *Angiostrongylidae,* for example, *Aelurostrongylus abstrusus* of *Aelurostrongylus* spp.; and *Angiostrongylus vasorum* and *Angiostrongylus cantonesis* of *Angiostrongylus* spp.;
(b) nematodes of the family *Crenosomatidae,* for example, *Crenosoma aerophila* and *Crenosoma vulpis* of *Crenosoma* spp.;
(c) nematodes of the family *Filaroididae,* for example, *Filaroides hirthi* and *Filaroides osleri* of *Filaroides* spp.;
(d) lungworms of the family *Metastrongylidae,* for example, *Metastrongylus apri, Metastrongylus asymmetricus, Metastrongylus pudendotectus,* and *Metastrongylus salmi* of *Metastrongylus* spp.;
(e) gapeworms of the family *Syngamidae,* for example, *Cyathostoma bronchialis* of *Cyathostoma* spp.; and *Syngamus skrjabinomorpha* and *Syngamus trachea* of *Syngamus* spp.

(6) Nematodes of the order *Strongylida*

(a) nematodes of the family *Molineidae,* for example, *Nematodirus filicollis* and *Nematodirus spathiger* of *Nematodirus* spp.;
(b) nematodes of the family *Dictyocaulidae,* for example, *Dictyocaulus filaria* and *Dictyocaulus viviparus* of *Dictyocaulus* spp.;
(c) nematodes of the family *Haemonchidae,* for example, *Haemonchus contortus* of *Haemonchus* spp.; and *Mecistocirrus digitatus* of *Mecistocirrus* spp.;
(d) nematodes of the family *Haemonchidae,* for example, *Ostertagia ostertagi* of *Ostertagia* spp.;
(e) nematodes of the family *Heligmonellidae,* for example, *Nippostrongylus braziliensis* of *Nippostrongylus* spp.;
(f) nematodes of the family *Trichostrongylidae,* for example, *Trichostrongylus axei, Trichostrongylus colubri-*

*formis,* and *Trichostrongylus tenuis* of *Trichostrongylus* spp.; *Hyostrongylus rubidus* of *Hyostrongylus* spp.; and *Obeliscoides cuniculi* of *Obeliscoides* spp.

(7) Nematodes of the order *Strongylida*

(a) nematodes of the family *Chabertiidae,* for example, *Chabertia ovina* of *Chabertia* spp.; and *Oesophagostomum brevicaudatum, Oesophagostomum columbianum, Oesophagostomum dentatum, Oesophagostomum georgianum, Oesophagostomum maplestonei, Oesophagostomum quadrispinulatum, Oesophagostomum radiatum, Oesophagostomum venulosum,* and *Oesophagostomum watanabei* of *Oesophagostomum* spp.;
(b) nematodes of the family *Stephanuridae,* for example, *Stephanurus dentatus* of *Stephanurus* spp.;
(c) nematodes of the family *Strongylidae,* for example, *Strongylus asini, Strongylus edentatus, Strongylus equinus,* and *Strongylus vulgaris* of *Strongylus* spp.

(8) Nematodes of the order *Oxyurida*
nematodes of the family *Oxyuridae,* for example, *Enterobius anthropopitheci* and *Enterobius vermicularis* of *Enterobius* spp.; *Oxyuris equi* of *Oxyuris* spp.; and *Passalurus ambiguous* of *Passalurus* spp.
(9) Nematodes of the order *Ascaridida*

(a) nematodes of the family *Ascaridiidae,* for example, *Ascaridia galli* of *Ascaridia* spp.;
(b) nematodes of the family *Heterakidae,* for example, *Heterakis beramporia, Heterakis brevispiculum, Heterakis gallinarum, Heterakis pusilla,* and *Heterakis putaustralis* of *Heterakis* spp.;
(c) nematodes of the family *Anisakidae,* for example, *Anisakis simplex* of *Anisakis* spp.;
(d) nematodes of the family Ascarididae, for example, *Ascaris lumbricoides* and *Ascaris suum* of *Ascaris* spp.; and *Parascaris equorum* of *Parascaris* spp.;
(e) nematodes of the family *Toxocaridae,* for example, *Toxocara canis, Toxocara leonina, Toxocara suum, Toxocara vitulorum,* and *Toxocara cati* of *Toxocara* spp.

(10) Nematodes of the order *Spirurida*

(a) nematodes of the family *Onchocercidae,* for example, *Brugia malayi, Brugia pahangi,* and *Brugia patei* of *Brugia* spp.; *Dipetalonema reconditum* of *Dipetalonema* spp.; *Dirofilaria immitis* of *Dirofilaria* spp.; *Filaria oculi* of *Filaria* spp.; and *Onchocerca cervicalis, Onchocerca gibsoni,* and *Onchocerca gutturosa* of *Onchocerca* spp.;
(b) nematodes of the family *Setariidae,* for example, *Setaria digitata, Setaria equina, Setaria labiatopapillosa,* and *Setaria marshalli* of *Setaria* spp.; and *Wuchereria bancrofti* of *Wuchereria* spp.;
(c) nematodes of the family *Filariidae,* for example, *Parafilaria multipapillosa* of *Parafilaria* spp.; and *Stephanofilaria assamensis, Stephanofilaria dedoesi, Stephanofilaria kaeli, Stephanofilaria okinawaensis,* and *Stephanofilaria stilesi* of *Stephanofilaria* spp.

(11) Nematodes of the order *Spirurida*

(a) nematodes of the family *Gnathostomatidae,* for example, *Gnathostoma doloresi* and *Gnathostoma spinigerum* of *Gnathostoma* spp.;
(b) nematodes of the family *Habronematidae,* for example, *Habronema majus, Habronema microstoma,* and *Habronema muscae* of *Habronema* spp.; and *Draschia megastoma* of *Draschia* spp.;
(c) nematodes of the family *Physalopteridae,* for example, *Physaloptera canis, Physaloptera cesticillata, Physaloptera erdocyona, Physaloptera felidis, Physaloptera gemina, Physaloptera papilloradiata, Physaloptera praeputialis, Physaloptera pseudopraerutialis, Physaloptera rara, Physaloptera sibirica,* and *Physaloptera vulpineus* of *Physaloptera* spp.;
(d) nematodes of the family *Gongylonematidae,* for example, *Gongylonema pulchrum* of *Gongylonema* spp.;
(e) nematodes of the family *Spirocercidae,* for example, *Ascarops strongylina* of *Ascarops* spp.;
(f) nematodes of the family *Thelaziidae,* for example, *Thelazia callipaeda, Thelazia gulosa, Thelazia lacrymalis, Thelazia rhodesi,* and *Thelazia skrjabini* of *Thelazia* spp.

[Control agent for other pests]

The heteroaryl azole compound of the present invention is additionally excellent in control effect on insect pests that have a stinger or venom and harm humans and animals, insect pests that mediate various pathogens or disease-causing microbes, or insect pests that cause discomfort to humans (toxic pests, hygienic pests, and obnoxious pests, etc.).

Specific examples thereof will be shown below.

(1) Insect pests of the order *Hymenoptera*
bees of the family *Argidae,* bees of the family *Cynipidae,* bees of the family *Diprionidae,* ants of the family *Formicidae,* bees of the family *Mutillidae,* and bees of the family *Vespidae.*
(2) Other insect pests
*Blattodea,* termites, *Araneae,* centipedes, millipedes, crustacea, and *Cimex lectularius.*

[Seed treatment agent or vegetative propagation organ treatment agent]

The seed treatment agent or vegetative propagation organ treatment agent of the present invention contains at least one active ingredient selected from the heteroaryl azole compounds of the present invention. The amount of the heteroaryl azole compound contained in the seed treatment agent or vegetative propagation organ treatment agent of the present invention is not particularly limited as long as its control effect is exhibited.
The vegetative propagation organ means a plant root, stem, leaf, or the like having the ability to grow when the site is separated from the body and placed in soil. As the vegetative propagation organ, for example, a tuberous root, a creeping root, a bulb, a corm or solid bulb, a tuber, a rhizome, a stolon, a rhizophore, a cane cutting, a propagule and a vine cutting are exemplified. The stolon is also called runner. The propagule is also called bulblet and is classified into a broad bud and a bulbil. The vine cutting means a shoot (generic name for leaves and stems) of sweet potato, Japanese yam, or the like. [情報システム部1] The bulb, the corm or solid bulb, the tuber, the rhizome, the cane cutting, the rhizophore and the tuberous root are also collectively called flower bulb. The cultivation of tubers and roots is started by planting tubers in soil. The tubers used are generally called seed tubers.
The seed treatment agent or vegetative propagation organ treatment agent of the present invention refers to a formulation such as a wettable powder, wettable granules, a flowable concentrate, or a dust produced by mixing at least one active ingredient selected from the heteroaryl azole compounds of the present invention with an appropriate solid carrier or liquid carrier, and, if necessary, adding a surfactant or other pharmaceutical aids to the mixture. This composition is usually used as a mixture with a binder. Also, the composition may contain a binder. As the composition containing a binder, a flowable concentrate (FS) for seed treatment is exemplified.
An adhesive substance that does not phytotoxically affect plant seeds or vegetative propagation organs is used as the binder. Specifically, at least one component selected from the group consisting of polyvinyl acetate, polyvinyl alcohol, cellulose including ethylcellulose, methylcellulose, hydroxymethylcellulose, hydroxypropylcellulose, and carboxymethylcellulose, polyvinylpyrrolidone, starch, modified starch, dextrin, maltodextrin, polysaccharides including alginate and chitosan, proteins including gelatin and casein, gum arabic, shellac, calcium lignosulfonate, and a methacrylamide monomer may be used.
Pests may be efficiently controlled by treating seed tubers with at least one compound selected from the heteroaryl azole compounds of the present invention. As the method for treating a seed tuber with the heteroaryl azole compound, dipping treatment, dust coating treatment, coating treatment or the like is exemplified. For the planting of seed tubers using a tractor, the seed tubers may be treated by spraying a chemical containing the heteroaryl azole compound onto the seed tubers on the tractor.

[Granular agrochemical composition for paddy rice seedling nursery box treatment]

The granular agrochemical composition for paddy rice seedling nursery box treatment (hereinafter, referred to as the "granular agrochemical composition") of the present invention contains at least one active ingredient selected from the heteroaryl azole compounds of the present invention. The amount of the heteroaryl azole compound contained in the granular agrochemical composition of the present invention is not particularly limited as long as its control effect is exhibited.
The granular agrochemical composition may be obtained by various methods, but is obtained, for example, by the extrusion granulation or the like of a powdery composition produced with the mixture of one or more active ingredients with, if necessary, a surfactant, a binder and an inorganic carrier, etc. with a wet granulation method. More specifically, a granular agrochemical composition is prepared by mixing uniformly the active ingredient adjusted to a predetermined particle size with a necessary surfactant, binder and inorganic carrier, adding an appropriate amount of water thereto, kneading the mixture, shaping them by extrusion through a screen having opened pores, and drying them. The size of the pores used in this operation is usually preferably 0.5 mm to 1.5 mm.
The particle size of the granular agrochemical composition thus obtained is not particularly limited, but is preferably 0.5 mm to 1.5 mm, and particularly preferably 0.7 mm to 1.5 mm, in terms of average particle size. The granules having such a particle size are obtained by granulation, drying and subsequent sifting.

The granular agrochemical composition is blended, if necessary, with a surfactant, a binder and an inorganic carrier. Among them, as the surfactant, for example, a nonionic surfactant such as polyethylene glycol higher fatty acid ester, polyoxyethylene alkyl ether, polyoxyethylene alkyl aryl ether, polyoxyethylene aryl phenyl ether, and sorbitan monoalkylate; a polycarboxylic acid-type polymer activator such as alkyl aryl sulfonate, dialkyl sulfonate, alkyl sulfuric acid ester salt, alkyl phosphoric acid ester salt, alkyl aryl sulfuric acid ester salt, alkyl aryl phosphoric acid ester salt, polyoxyethylene alkyl ether sulfuric acid ester salt, naphthalenesulfonate and a condensate thereof, ligninsulfonate, a copolymer of acrylic acid and itaconic acid or a copolymer of methacrylic acid and itaconic acid, a copolymer of maleic acid and styrene, a copolymer of maleic acid and diisobutylene and their alkali metal salts; polyoxyethylene aryl phenyl ether phosphoric acid ester salt; an anionic surfactant such as polyoxyethylene aryl phenyl ether sulfuric acid ester salt, or the like is exemplified. The amount of this surfactant added is usually 0.1 parts by weight to 5 parts by weight, though the amount is not particularly limited.

As the binder to be blended to the granular agrochemical composition, for example, carboxymethylcellulose metal salt, polyvinyl alcohol, pregelatinized starch, dextrin, xanthan gum, guar seed gum, sucrose, polyvinylpyrrolidone, polyacrylic acid metal salt or the like is exemplified. The amount of the binder added is usually 0.1 parts by weight to 5 parts by weight, though the amount is not particularly limited. The inorganic carrier to be blended to the granular agrochemical composition is not particularly limited. For example, clays, calcium carbonate, talc, diatomaceous earth, zeolite, attapulgite, gypsum, porcelain stone or the like is exemplified.

[Soil treatment agent]

The soil treatment according to the present invention is, for example, a method of directly controlling pests by applying an active ingredient to the rhizospheres of plants to be protected from harm such as eating by the pest, or controlling pests by penetrating and transporting an active ingredient to the inside of plant bodies from their roots or the like. Specifically, for example, planting hole treatment (planting hole spraying and planting hole soil-incorporation), plant foot treatment (plant foot spraying, plant foot soil-incorporation, plant foot irrigation, and plant foot treatment at latter half of the seedling raising period), planting furrow treatment (planting furrow spraying and planting furrow soil-incorporation), planting row treatment (planting row spraying, planting row soil-incorporation, and planting row spraying at the growing period), planting row treatment at sowing (planting row spraying at sowing and planting row soil-incorporation at sowing), broadcast treatment (broadcast soil spraying and broadcast soil-incorporation), band dressing, submerged treatment (broadcast submerged application and frame submerged application), other soil spraying treatments (foliar granule spraying at the growing period, spraying under tree crowns or around main stems, soil surface spraying, soil surface incorporation, sowing hole spraying, surface spraying on the ribbing ground, and inter-plant spraying), other irrigation treatments (soil irrigation, irrigation at the seedling raising period, chemical injection treatment, irrigation on the plant foot, chemical drip irrigation, and chemigation), seedling nursery box treatment (seedling nursery box spraying, seedling nursery box irrigation, and seedling nursery box chemical flooding), seedling nursery tray treatment (seedling nursery tray spraying, seedling nursery tray irrigation, and flooded nursery tray spraying), nursery bed treatment (nursery bed spraying, nursery bed irrigation, flooded nursery bed spraying), seedling dipping, nursery soil-incorporation treatment (seedbed soil-incorporation and cover soil-incorporation), spraying before soil covering at sowing, spraying after soil covering at sowing, stem injection treatment, trunk injection treatment, trunk spraying treatment, and other treatments (plowing, surface soil-incorporation, soil incorporation into rain dropping lines, planting spot treatment, flower cluster granule spraying, and paste fertilizer mixing) are exemplified.

Water culture medium treatment is, for example, a method of protecting plants from harm ascribable to pests by applying an active ingredient to water culture media or the like and thereby penetrating and transporting an active ingredient to the inside of bodies of the plants to be protected from harm such as eating by the pests, from their roots or the like. Specifically, for example, water culture medium incorporation, water culture medium mixing or the like is exemplified.

The active ingredient may be applied to a plant by a method such as wrapping around the plant, tacking across the neighborhood of the plant, spreading over plant foot soil, or covering a cultivation region of the plant with a formulation allowed to contain the composition of the present invention by a method such as dipping, impregnation, coating, or kneading in a carrier such as a sheet-, strap-, tape- or net-shaped resin, paper, or cloth.

[Bait agent]

The bait agent of the present invention contains at least one active ingredient selected from the heteroaryl azole compounds of the present invention. The amount of the heteroaryl azole compound contained in the bait agent of the present invention is not particularly limited as long as its control effect is exhibited.

The bait agent of the present invention may contain, if necessary, an ingestibility improving component such as a sugar, a carbohydrate, or a milk constituent, a synergist, an aversive agent to prevent accidental ingestion, a

preservative, a flavor, an attractant, or the like. The bait agent of the present invention may usually be prepared by mixing the heteroaryl azole compound with water and, if necessary, other components described above. In the preparation of the bait agent of the present invention, the heteroaryl azole compound may be the heteroaryl azole compound itself, or may be in the form of a preparation such as a dust, a wettable powder, a microcapsule, or a flowable concentrate.

As the pest that may be effectively controlled with the bait agent of the present invention, a cockroach such as *Periplaneta americana, Blattella germanica,* and *Periplaneta fuliginosa,* a click beetle such as *Melanotus okinawensis,* an ant such as *Monomorium intrudens* and *Formica fusca,* a deathwatch beetle such as *Lasioderma serricorne* and *Stegobium paniceum,* a flour beetle such as *Tribolium castaneum* and *Tribolium confusum,* a flat bark beetle such as *Oryzaephilus surinamensis* and *Cryptolestes pusillus,* a white ant such as *Coptotermes formosanus* and *Reticulitermes speratus,* a fly such as *Musca domestica, Fannia canicularis, Phoridae,* and *Phlebotominae,* and a mosquito such as *Culex pipiens, Aedes albopictus,* anopheles, and chironomids are exemplified.

The form of application of the bait agent of the present invention may be the bait agent of the present invention itself or may be, for example, in a state where a nonwoven fabric, sponge, absorbent cotton, or the like is impregnated with the bait agent. This aqueous bait agent or the nonwoven fabric, sponge, absorbent cotton, or the like impregnated with the bait agent is placed in a container, for example, a cup, a tray, or a bottle, and subjected to insect pest expelling. In this respect, a cover is put on the outside of the bait-containing container of the present invention to prepare an apparatus having some degree of space where insect pests are capable of residing in order to ingest the bait agent of the present invention. This generally improves ingestibility and is thus effective.

[Plant growth promoter]

The plant growth promoter of the present invention contains at least one active ingredient selected from the heteroaryl azole compounds of the present invention. The amount of the heteroaryl azole compound contained in the plant growth promoter of the present invention is not particularly limited as long as its effect of promoting plant growth is exhibited.

The plant growth promoter of the present invention may be appropriately blended, if necessary, with other components, a carrier, or the like.

The heteroaryl azole compound of the present invention alone may be used as a plant growth promoter, but may usually be used as a formulation such as a wettable powder, a liquid formulation, an oil formulation, a dust, a granular formulation, or a suspension concentrate (flowable) by mixing the heteroaryl azole compound as an active ingredient with common adjuvants such as a solid carrier, a liquid carrier, a dispersant, a diluent, an emulsifier, a spreading agent and a thickener.

As the solid carrier or the liquid carrier, for example, talc, clay, bentonite, kaolin, diatomaceous earth, montmorillonite, mica, vermiculite, gypsum, calcium carbonate, white carbon, wood flour, starch, alumina, silicate, glycopolymer, waxes, water, alcohols (methyl alcohol, ethyl alcohol, n-propyl alcohol, isopropyl alcohol, n-butyl alcohol, ethylene glycol, benzyl alcohol, etc.), a petroleum fraction (petroleum ether, kerosene, solvent naphtha, etc.), aliphatic or alicyclic hydrocarbons (n-hexane, cyclohexane, etc.), aromatic hydrocarbons (benzene, toluene, xylene, ethylbenzene, chlorobenzene, cumene, methylnaphthalene, etc.), halogenated hydrocarbons (chloroform, dichloromethane, etc.), ethers (isopropyl ether, ethylene oxide, tetrahydrofuran, etc.), ketones (acetone, methyl ethyl ketone, cyclohexanone, methyl isobutyl ketone, etc.), esters (ethyl acetate, butyl acetate, ethylene glycol acetate, amyl acetate, etc.), acid amides (dimethylformamide, dimethylacetanilide, etc.), nitriles (acetonitrile, propionitrile, acrylonitrile, etc.), sulfoxides (dimethyl sulfoxide, etc.), alcohol ethers (ethylene glycol monomethyl ether, ethylene glycol monoethyl ether, etc.) or the like is exemplified.

As the adjuvant, for example, a nonionic surfactant (polyoxyethylene alkyl ether, polyoxyethylene alkyl ester, polyoxyethylene alkyl phenyl ether, polyoxyethylene sorbitan alkyl ester, sorbitan alkyl ester, etc.), an anionic surfactant (alkyl benzenesulfonate, alkyl sulfosuccinate, polyoxyethylene alkyl sulfate, aryl sulfonate, etc.), a cationic surfactant (alkylamines, polyoxyethylene alkylamines, quaternary ammonium salts, etc.), an amphoteric surfactant (alkylaminoethylglycine, alkyldimethylbetaine, etc.), polyvinyl alcohol, hydroxypropylcellulose, carboxymethylcellulose, gum arabic, tragacanth gum, xanthan gum, polyvinyl acetate, gelatin, casein, sodium alginate or the like is exemplified.

As other components, other active ingredients such as the fungicide, insecticide or miticide, nematicide, and pesticide for soil insect pests described above; a plant regulating agent, a synergist, a fertilizer, a soil improvement agent, animal feed or the like is exemplified.

The content of the heteroaryl azole compound of the present invention in the plant growth promoter differs variously depending on a preparation form, an application method, and other conditions, but is preferably 0.5 to 95% by mass, and particularly preferably in the range of 2 to 70% by mass.

The plant to which the plant growth promoter is applied is not particularly limited. For example, cereals of the family *Poaceae,* such as rice, barley, wheat, Japanese millet, corn, and foxtail millet; vegetables such as pumpkin, turnip, cabbage, daikon radish, Chinese cabbage, spinach, bell pepper, and tomato; flowers and ornamental plants such as

chrysanthemum, gerbera, pansy, orchid, peony, and tulip; beans such as azuki bean, kidney bean, soybean, peanut, broad bean, and garden pea; tubers and roots such as potato, sweet potato, eddo, Japanese yam, and taro; *Allium* such as green onion, onion, and rakkyo, or the like is exemplified.

As a method for applying the plant growth promoter of the present invention, application to plants (foliage application), application to plant growing soil (soil application), application to paddy water (submerged application), application to seeds (seed treatment), or the like is exemplified.

The amount of the plant growth promoter of the present invention applied differs depending on a plant to which the plant growth promoter is applied, etc. In case of foliage application, 50 to 300 L/10 are of a solution containing 1 to 10000 ppm, preferably 10 to 1000 ppm of the active ingredient is preferably applied, and in case of soil application and submerged application, 0.1 to 1000 g/are, particularly preferably 10 to 100 g/10 are of active ingredient is preferably applied. In case of seed treatment, 0.001 to 50 g of the active ingredient per 1 kg of seeds is preferably applied.

[Formulated preparation]

Some formulated preparations of the pest control agent, the insecticide or miticide, the ectoparasite control agent or the endoparasite control agent or expellant of the present invention will be shown. However, additives and addition ratios should not be limited by these examples and may be changed in wide ranges. The term "part" in the formulated preparations represents part by weight.

Hereinafter, the formulated preparations for agriculture or horticulture and for paddy rice will be shown.

(Formulation 1: Wettable powder)

40 parts of the heteroaryl azole compound of the present invention, 53 parts of diatomaceous earth, 4 parts of higher alcohol sulfuric acid ester, and 3 parts of alkyl naphthalenesulfonate are uniformly mixed and finely milled to obtain a wettable powder containing 40% of the active ingredient.

(Formulation 2: Emulsion)

30 parts of the heteroaryl azole compound of the present invention, 33 parts of xylene, 30 parts of dimethylformamide, and 7 parts of polyoxyethylene alkyl allyl ether are mixed and dissolved to obtain an emulsion containing 30% of the active ingredient.

(Formulation 3: Granular formulation)

5 parts of the heteroaryl azole compound of the present invention, 40 parts of talc, 38 parts of clay, 10 parts of bentonite, and 7 parts of sodium alkyl sulfate are uniformly mixed, finely milled, and then granulated into a granular shape of 0.5 to 1.0 mm in diameter to obtain a granular formulation containing 5% of the active ingredient.

(Formulation 4: Granular formulation)

5 parts of the heteroaryl azole compound of the present invention, 73 parts of clay, 20 parts of bentonite, 1 part of dioctyl sulfosuccinate sodium salt, and 1 part of potassium phosphate are well milled and mixed, and after addition of water, the mixture is well kneaded, then granulated, and dried to obtain a granular formulation containing 5% of the active ingredient.

(Formulation 5: Suspension)

10 parts of the heteroaryl azole compound of the present invention, 4 parts of polyoxyethylene alkyl allyl ether, 2 parts of polycarboxylic acid sodium salt, 10 parts of glycerin, 0.2 parts of xanthan gum, and 73.8 parts of water are mixed and wet-milled until the particle size becomes 3 microns or smaller to obtain a suspension containing 10% of the active ingredient.

Hereinafter, the formulated preparations of the ectoparasite control agent or the endoparasite control agent or expellant will be shown.

(Formulation 6: Granules)

5 parts of the heteroaryl azole compound of the present invention are dissolved in an organic solvent to obtain a solution. The solution is sprayed onto 94 parts of kaolin and 1 part of white carbon. Then, the solvent is evaporated

under reduced pressure. This type of granules may be mixed with animal feed.

(Formulation 7: Injectable filler)

0.1 to 1 parts of the heteroaryl azole compound of the present invention and 99 to 99.9 parts of peanut oil are uniformly mixed and then sterilized by filtration through a sterilizing filter.

(Formulation 8: Pore-on formulation)

5 parts of the heteroaryl azole compound of the present invention, 10 parts of myristic acid ester, and 85 parts of isopropanol are uniformly mixed to obtain a pore-on formulation.

(Formulation 9: Spot-on formulation)

10 to 15 parts of the heteroaryl azole compound of the present invention, 10 parts of palmitic acid ester, and 75 to 80 parts of isopropanol are uniformly mixed to obtain a spot-on formulation.

(Formulation 10: Spray formulation)

1 part of the heteroaryl azole compound of the present invention, 10 parts of propylene glycol, and 89 parts of isopropanol are uniformly mixed to obtain a spray formulation.
Next, the present invention will be more specifically described with reference to Examples of compounds. However, the present invention is not limited by the following Examples of compounds by any means.

[Example 1]

Synthesis of 2-(5-(ethylsulfonyl)-6-(1-methyl-5-(perfluoropropyl)-1H-imidazol-2-yl)pyridin-3-yl)pyrimidine (compound No. b-19)

(Step 1) Synthesis of 2-bromo-5-chloro-3-(ethylthio)pyridine

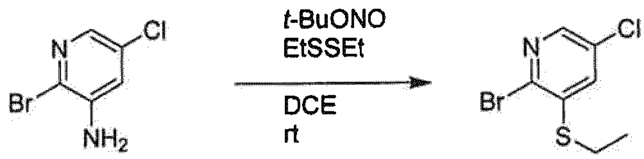


3-Amino-2-bromo-5-chloropyridine (40.0 g) and diethyl disulfide (16.5 g) were dissolved in 1,2-dichloroethane (770 ml), and the obtained solution was stirred at room temperature. t-Butyl nitrite (29.8 g) was added thereto, and the mixture was heated overnight at 50°C. The obtained solution was poured into water, followed by extraction with chloroform. The obtained organic layer was washed with saturated brine, dried over anhydrous magnesium sulfate, and filtered. The filtrate was concentrated under reduced pressure. The obtained concentrate was purified by silica gel column chromatography to obtain 30.0 g of the title compound (yield: 62%).
$^1$H-NMR of the obtained title compound will be shown below.
$^1$H-NMR ($CDCl_3$) $\delta$: 8.08 (1H, d), 7.37 (1H, d), 2.96 (2H, q), 1.42 (3H, t).

(Step 2) Synthesis of 5-chloro-3-(ethylthio)-2-(1-methyl-1H-imidazol-2-yl) pyridine

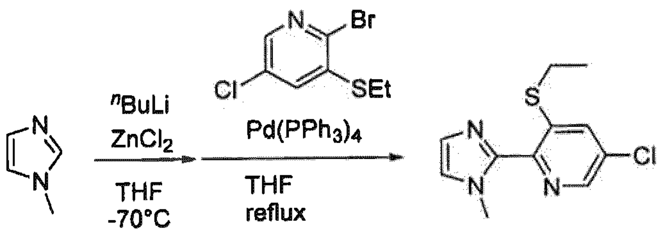


In a reaction vessel, 1-methyl-1H-imidazole (4.0 g) was dissolved in tetrahydrofuran (200 ml), and the reaction vessel was replaced with nitrogen. Then, the reaction solution was cooled to -70°C. A 2.65 M solution of n-butyllithium in n-hexane (21 ml) was added dropwise thereto, and the mixture was stirred at -70°C for 30 minutes. A 1 M solution of zinc chloride in tetrahydrofuran (83 ml) was added thereto, and the mixture was warmed to room temperature and stirred for 1 hour. Then, 2-bromo-5-chloro-3-(ethylthio)pyridine (10.4 g) and tetrakis(triphenylphosphine)palladium (2.4 g) were added thereto, and the reaction vessel was replaced with nitrogen. Then, the reaction mixture was stirred overnight under heating to reflux. The obtained solution was poured into an aqueous Rochelle salt solution, followed by extraction with ethyl acetate. The obtained organic layer was washed with an aqueous sodium hydroxide solution and saturated brine, dried over anhydrous magnesium sulfate, and filtered. The filtrate was concentrated under reduced pressure. The obtained concentrate was purified by silica gel column chromatography to obtain 9.65 g of the title compound (yield: 92%).

$^1$H-NMR of the obtained title compound will be shown below.
$^1$H-NMR ($CDCl_3$) $\delta$: 8.31 (1H, d), 7.59 (1H, d), 7.21 (1H, d), 6.99 (1H, d), 3.85 (3H, s), 2.92 (2H, q), 1.37 (3H, t).

(Step 3) Synthesis of 5-chloro-3-(ethylsulfonyl)-2-(1-methyl-1H-imidazol-2-yl) pyridine

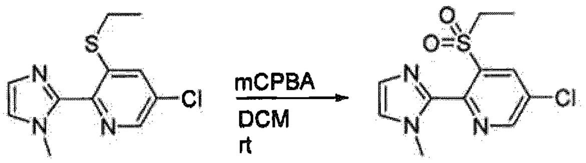


5-Chloro-3-(ethylthio)-2-(1-methyl-1H-imidazol-2-yl)pyridine (0.80 g) was dissolved in dichloromethane (20 ml), and the solution was cooled to 0°C. 70% m-chloroperbenzoic acid (1.71 g) was added thereto, and the mixture was stirred at room temperature for 5 hours. The obtained solution was poured into a mixed solution of a saturated aqueous solution of sodium bicarbonate and a saturated aqueous solution of sodium thiosulfate, followed by extraction with dichloromethane. The obtained organic layer was washed with saturated brine, dried over anhydrous magnesium sulfate, and filtered. The filtrate was concentrated under reduced pressure. The obtained concentrate was purified by silica gel column chromatography to obtain 0.84 g of the title compound (yield: 93%).

$^1$H-NMR of the obtained title compound will be shown below.
$^1$H-NMR ($CDCl_3$) $\delta$: 8.83 (1H, d), 8.46 (1H, d), 7.14 (1H, d), 7.05 (1H, d), 3.93 (2H, q), 3.68 (3H, s), 1.34 (3H, t).

(Step 4) Synthesis of 2-(5-bromo-1-methyl-1H-imidazol-2-yl)-5-chloro-3-(ethylsulfonyl)pyridine

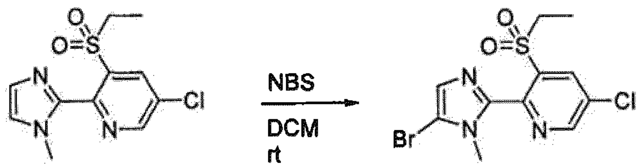


5-Chloro-3-(ethylsulfonyl)-2-(1-methyl-1H-imidazol-2-yl)pyridine (0.84 g) was dissolved in dichloromethane (30 ml), and the solution was cooled to 0°C. N-Bromosuccinimide (0.50 g) was added thereto, and the mixture was stirred at room temperature for 3 hours. The obtained solution was poured into water, followed by extraction with dichloromethane. The obtained organic layer was washed with saturated brine, dried over anhydrous magnesium sulfate, and filtered.

The filtrate was concentrated under reduced pressure, and the obtained concentrate was purified by silica gel column chromatography to obtain 0.93 g of the title compound (yield: 89%).

$^1$H-NMR of the obtained title compound will be shown below.

$^1$H-NMR ($CDCl_3$) $\delta$: 8.83 (1H, d), 8.46 (1H, d), 7.13 (1H, s), 3.88 (2H, q), 3.62 (3H, s), 1.35 (3H, t).

(Step 5) Synthesis of 5-chloro-3-(ethylsulfonyl)-2-(5-iodo-1-methyl-1H-imidazol-2-yl) pyridine

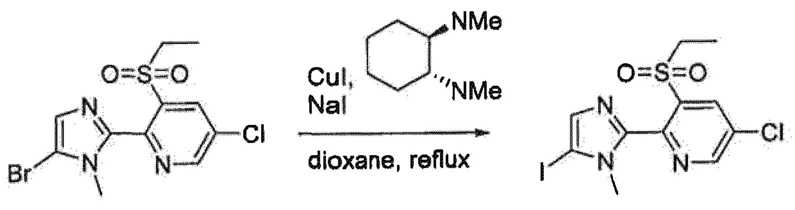


In a reaction vessel, 2-(5-bromo-1-methyl-1H-imidazol-2-yl)-5-chloro-3-(ethylsulfonyl)pyridine (0.93 g) was dissolved in dioxane (26 ml), and the reaction vessel was replaced with argon. Then, the reaction solution was stirred at room temperature. Sodium iodide (1.92 g), copper iodide (0.1 g), and trans-N,N'-dimethylcyclohexane-1,2-diamine (0.15 g) were added thereto, and the mixture was stirred overnight under heating to reflux. The obtained solution was poured into water, followed by extraction with dichloromethane. The obtained organic layer was washed with saturated brine, dried over anhydrous magnesium sulfate, and filtered. The filtrate was concentrated under reduced pressure, and the obtained concentrate was purified by silica gel column chromatography to obtain 0.93 g of the title compound (yield: 89%).

$^1$H-NMR of the obtained title compound will be shown below.

$^1$H-NMR ($CDCl_3$) $\delta$: 8.84 (1H, d), 8.46 (1H, d), 7.23 (1H, s), 3.87 (2H, q), 3.62 (3H, s), 1.34 (3H, t).

(Step 6) Synthesis of 5-chloro-3-(ethylsulfonyl)-2-(1-methyl-5-(perfluoropropyl)-1H-imidazol-2-yl)pyridine

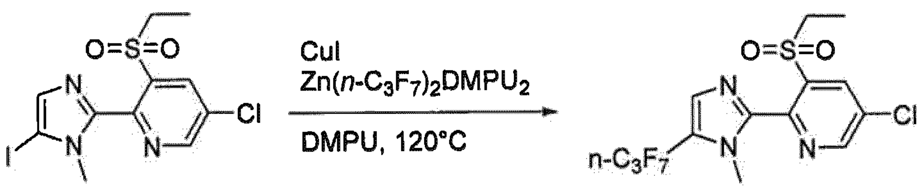


In a reaction vessel, 5-chloro-3-(ethylsulfonyl)-2-(5-iodo-1-methyl-1H-imidazol-2-yl)pyridine (0.68 g) was dissolved in N,N'-dimethylpropyleneurea (3 ml), and the reaction vessel was replaced with argon. Then, the reaction solution was stirred at room temperature. Copper iodide (0.57 g) and bis(n-perfluoropropyl)zinc/DMPU complex (1.96 g) were added thereto, and the mixture was stirred overnight at 120°C. The obtained solution was poured into a saturated aqueous solution of Rochelle salt, followed by extraction with ethyl acetate. The obtained organic layer was washed with water and saturated brine, dried over anhydrous magnesium sulfate, and filtered. The filtrate was concentrated under reduced pressure, and the obtained concentrate was purified by silica gel column chromatography to obtain 0.61 g of the title compound (yield: 82%).

$^1$H-NMR of the obtained title compound will be shown below.

$^1$H-NMR ($CDCl_3$) $\delta$: 8.89 (1H, d), 8.47 (1H, d), 7.50 (1H, s), 3.73 (2H, q), 3.65 (3H, s), 1.34 (3H, t).

(Step 7) Synthesis of 2-(5-(ethylsulfonyl)-6-(1-methyl-5-(perfluoropropyl)-1H-imidazol-2-yl)pyridin-3-yl)pyrimidine

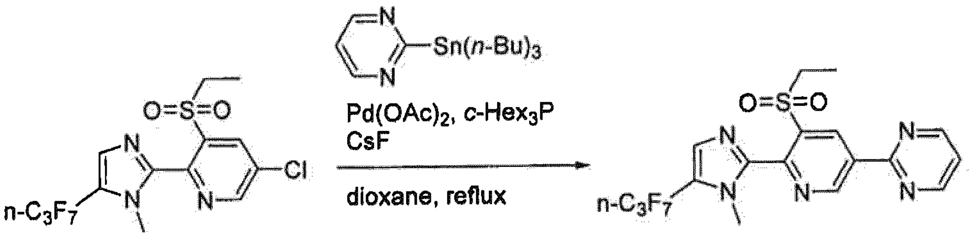


In a reaction vessel, 5-chloro-3-(ethylsulfonyl)-2-(1-methyl-5-(perfluoropropyl)-1H-imidazol-2-yl)pyridine (0.29 g) was dissolved in dioxane (10 ml), and the reaction vessel was replaced with nitrogen. Then, the reaction solution was stirred at room temperature. 2-(Tributylstannyl)pyrimidine (0.35 g), palladium(II) acetate (0.03 g), a solution of 18% tricyclohexylphosphine in toluene (0.36 g), and cesium fluoride (0.22 g) were added thereto, and the mixture was stirred overnight under heating to reflux. The obtained solution was poured into a 10% aqueous potassium fluoride solution, followed by extraction with ethyl acetate. The obtained organic layer was washed with water and saturated brine, dried over anhydrous magnesium sulfate, and filtered. The filtrate was concentrated under reduced pressure, and the obtained concentrate was purified by silica gel column chromatography to obtain 0.10 g of the title compound (yield: 31%).

$^{1}H$-NMR and $^{19}F$-NMR of the obtained title compound will be shown below.

$^{1}H$-NMR ($CDCl_3$) δ: 9.94 (1H, d), 9.48 (1H, d), 8.92 (2H, d), 7.52 (1H, s), 7.38 (1H, t), 3.75 (2H, q), 3.70 (3H, s), 1.37 (3H, t); $^{19}F$-NMR (376 MHz, $CDCl_3$-$C_6F_6$) : δ -80.41 (3H, t), -107.20 (2H, q), -125.60--125.68 (2H, m).

[Example 2]

Synthesis of 5-(ethylsulfonyl)-2-(1-methyl-1H-1,2,4-triazol-3-yl)-4-(1-methyl-5-(perfluorobutyl)-1H-imidazol-2-yl)pyrimidine (compound No. a-2)

(Step 1) Synthesis of 2-chloro-1-(5-iodo-1-methyl-1H-imidazol-2-yl)ethan-1-one

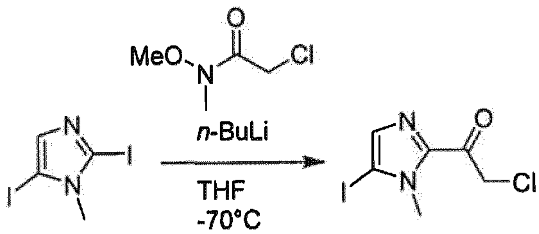


In a reaction vessel, 2,5-diiodo-1-methyl-1H-imidazole (34.6 g) was dissolved in tetrahydrofuran (1130 ml), and the reaction vessel was replaced with nitrogen. Then, the reaction solution was cooled to -70°C. n-Butyllithium (2.65 M, a solution in n-hexane, 42 ml) was added dropwise thereto, and the mixture was stirred at - 70°C for 30 minutes. 2-Chloro-N-methoxy-N-methylacetamide (17.0 g) was added thereto, and the mixture was stirred at -70°C for 1 hour. The obtained solution was poured into a saturated aqueous solution of ammonium chloride, followed by extraction with ethyl acetate. The obtained organic layer was washed with saturated brine, dried over anhydrous magnesium sulfate, and filtered. The filtrate was concentrated under reduced pressure to obtain a concentrate.

(Step 2) Synthesis of 2-(ethylthio)-1-(5-iodo-1-methyl-1H-imidazol-2-yl) ethan-1-one

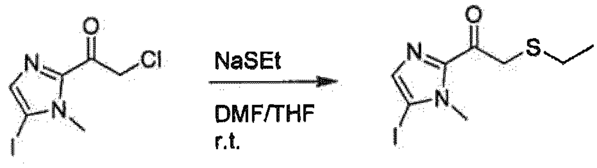

The concentrate obtained in step 1 (unpurified 2-chloro-1-(5-iodo-1-methyl-1H-imidazol-2-yl)ethan-1-one) was dissolved in a mixed solvent of N,N-dimethylformamide (100 ml) and tetrahydrofuran (100 ml), and the obtained solution was stirred at 0°C. Sodium ethyl mercaptan (10.0 g, 80%) was added thereto, and the mixture was stirred overnight at room temperature. The obtained solution was poured to water, followed by extraction with ethyl acetate. The obtained organic layer was washed with saturated brine, dried over anhydrous magnesium sulfate, and filtered. The filtrate was concentrated under reduced pressure, and the concentrate was purified by silica gel column chromatography to obtain 5.74 g of the title compound (yield: 18%, 2 steps).

$^1$H-NMR of the obtained title compound will be shown below.

$^1$H-NMR(400 MHz, $CDCl_3$): δ 7.28 (1H, s), 4.01 (3H, s), 3.94 (2H, s), 2.64 (2H, q), 1.28 (3H, t).

(Step 3) Synthesis of 2-(ethylsulfonyl)-1-(5-iodo-1-methyl-1H-imidazol-2-yl) ethan-1-one

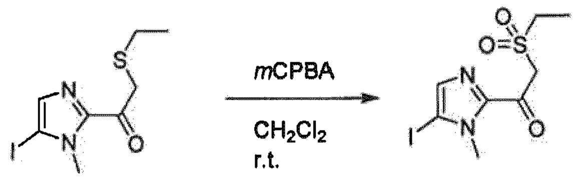


2-(Ethylthio)-1-(5-iodo-1-methyl-1H-imidazol-2-yl)ethan-1-one (5.7 g) was dissolved in dichloromethane (190 ml), and the obtained solution was stirred at 0°C. m-Chloroperbenzoic acid (70%, 10 g) was added thereto, and the mixture was stirred at room temperature for 5 hours. The obtained solution was poured into a mixed solution of a saturated aqueous solution of sodium bicarbonate and a saturated aqueous solution of sodium thiosulfate, followed by extraction with dichloromethane. The obtained organic layer was washed with saturated brine, dried over anhydrous magnesium sulfate, and filtered. The filtrate was concentrated under reduced pressure, and the concentrate was purified by silica gel column chromatography to obtain 5.2 g of the title compound (yield: 81%).

$^1$H-NMR of the obtained title compound will be shown below.

$^1$H-NMR(400 MHz, $CDCl_3$): δ 7.35 (1H, s), 4.81 (2H, s), 4.02 (3H, s), 3.28 (2H, q), 1.46 (3H, t).

(Step 4) Synthesis of 3-(dimethylamino)-2-(ethylsulfonyl)-1-(5-iodo-1-methyl-1H-imidazol-2-yl)prop-2-en-1-one

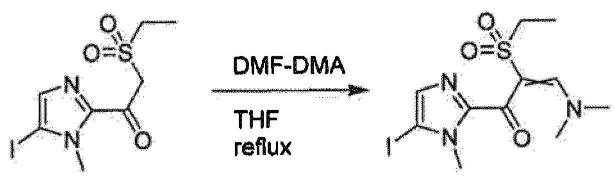


2-(Ethylsulfonyl)-1-(5-iodo-1-methyl-1H-imidazol-2-yl)ethan-1-one (0.34 g) was dissolved in tetrahydrofuran (5 ml), and the obtained solution was stirred at room temperature. N,N-Dimethylformamide dimethyl acetal (0.23 g) was added thereto, and the mixture was stirred under heating to reflux for 4 hours. The obtained solution was concentrated under reduced pressure.

(Step 5) Synthesis of 5-(ethylsulfonyl)-4-(5-iodo-1-methyl-1H-imidazol-2-yl)-2-(1-methyl-1H-1,2,4-triazol-3-yl)pyrimidine

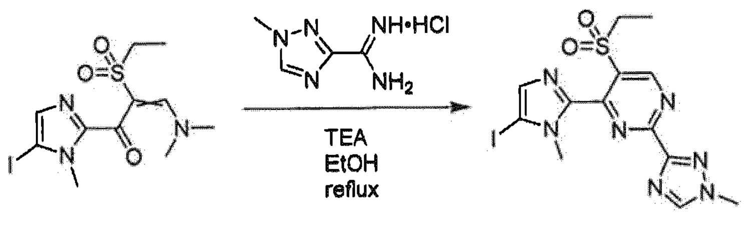

The concentrate obtained in step 4 (unpurified 3-(dimethylamino)-2-(ethylsulfonyl)-1-(5-iodo-1-methyl-1H-imidazol-2-yl)prop-2-en-1-one) was dissolved in ethanol (10 ml), and the solution was stirred at room temperature. Triethylamine (0.30 g) and 1-methyl-1H-1,2,4-triazole-3-carboximidamide hydrochloride (0.20 g) were added thereto, and the mixture was stirred under heating to reflux for 5 hours. The obtained solution was poured into a saturated aqueous solution of ammonium chloride, followed by extraction with dichloromethane. The obtained organic layer was washed with saturated brine, dried over anhydrous magnesium sulfate, and filtered. The filtrate was concentrated under reduced pressure, and the obtained concentrate was purified by silica gel column chromatography to obtain 0.44 g of the title compound (yield: 95%, 2 steps).

$^1$H-NMR of the obtained title compound will be shown below.

$^1$H-NMR(400 MHz, $CDCl_3$): δ 9.50 (1H, s), 8.25 (1H, s), 7.31 (1H, s), 4.11 (3H, s), 4.07 (2H, q), 3.94 (3H, s), 1.41 (3H, t).

(Step 6) Synthesis of 5-(ethylsulfonyl)-2-(1-methyl-1H-1,2,4-triazol-3-yl)-4-(1-methyl-5-(perfluorobutyl)-1H-imidazol-2-yl) pyrimidine

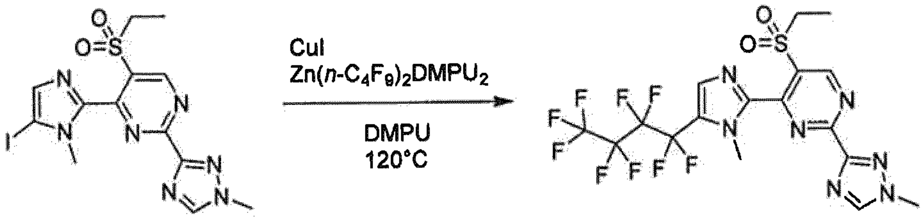


In a reaction vessel, 5-(ethylsulfonyl)-4-(5-iodo-1-methyl-1H-imidazol-2-yl)-2-(1-methyl-1H-1,2,4,-triazol-3-yl)pyrimidine (0.2 g) was dissolved in N,N'-dimethylpropyleneurea (3 ml), and the reaction vessel was replaced with argon and then stirred at room temperature. Copper iodide (0.17 g) and bis(n-perfluorobutyl)zinc/DMPU complex (0.66 g) synthesized with reference to the method described in Chem. Eur. J. 2015, 21, 96-100 were added thereto, and the mixture was stirred at 110°C for 3 hours. The obtained solution was poured into a saturated aqueous solution of Rochelle salt, followed by extraction with ethyl acetate. The obtained organic layer was washed with water and saturated brine, dried over anhydrous magnesium sulfate, and filtered. The filtrate was concentrated under reduced pressure, and the obtained concentrate was purified by silica gel column chromatography to obtain 0.06 g of the title compound (yield: 25%).

$^1$H-NMR and $^{19}$F-NMR of the obtained title compound will be shown below.
$^1$H-NMR(400 MHz, $CDCl_3$): δ 9.53 (1H, s), 8.27 (1H, s), 7.54 (1H, s), 4.12 (3H, s), 3.89 (3H, s), 3.88 (2H, q), 1.40 (3H, t); $^{19}$F-NMR (376 MHz, $CDCl_3$-$C_6F_6$) : δ -81.3 (s, 3F), - 106.3(s, 2F), -121.8(s, 2F), -126.0(s, 2F).

[Example 3]

Synthesis of 5-(ethylsulfonyl)-4-(4-methyl-5-(nonafluorobutyl)-4H-1,2,4-triazol-3-yl)-2,2'-bipyrimidine (compound No. a-5)

(Step 1) Synthesis of 2,2,3,3,4,4,5,5,5-nonafluoropentanehydrazide

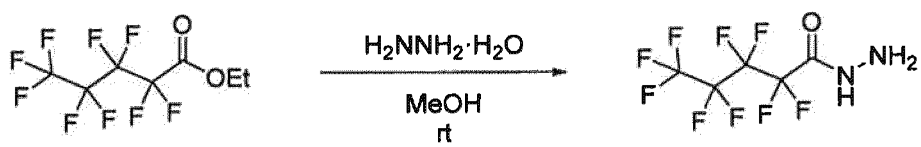


Ethyl 2,2,3,3,4,4,5,5,5-nonafluoropentanoate (15.8 g) was dissolved in methanol (108 ml), and the solution was stirred at room temperature. Hydrazine monohydrate (2.7 g) was added dropwise thereto, and the mixture was stirred at room temperature for 30 minutes. The obtained solution was concentrated under reduced pressure.

(Step 2) Synthesis of N-methyl-2-(2,2,3,3,4,4,5,5,5-nonafluoropentanoyl)hydrazine-1-carbothioamide

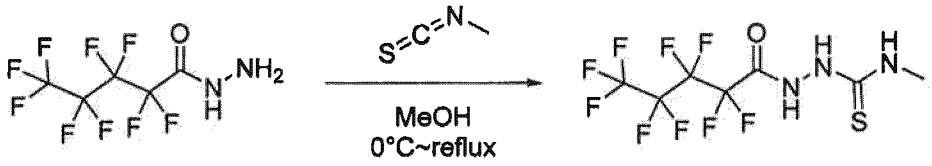


16.4 g of the concentrate obtained in step 1 (unpurified 2,2,3,3,4,4,5,5,5-nonafluoropentanehydrazide) was dissolved in methanol (54 ml), and the solution was stirred at 0°C. A solution of methyl isothiocyanate (3.95 g) in methanol (54 mL) was added dropwise thereto, and the mixture was stirred at 0°C for 10 minutes. Then, the reaction mixture was stirred under heating to reflux for 3 hours. The obtained solution was allowed to cool to room temperature and then concentrated under reduced pressure.

(Step 3) Synthesis of 4-methyl-5-(nonafluorobutyl)-2,4-dihydro-3H-1,2,4-triazole-3-thione

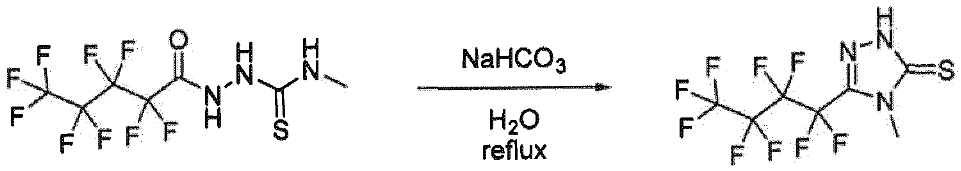


23.5 g of the concentrate (containing unpurified N-methyl-2-(2,2,3,3,4,4,5,5,5-nonafluoropentanoyl)hydrazine-1-carbothioamide) obtained in step 2 was suspended in water (270 ml), and the suspension was stirred at room temperature. Sodium bicarbonate (45.5 g) was added thereto, and the mixture was stirred under heating to reflux for 4 hours. The obtained solution was allowed to cool to room temperature and then adjusted to pH 2 with concentrated hydrochloric acid, followed by extraction with ethyl acetate. The obtained organic layer was washed with saturated brine, dried over anhydrous magnesium sulfate, and filtered. The filtrate was concentrated under reduced pressure.

(Step 4) Synthesis of 4-methyl-3-(nonafluorobutyl)-4H-1,2,4-triazole

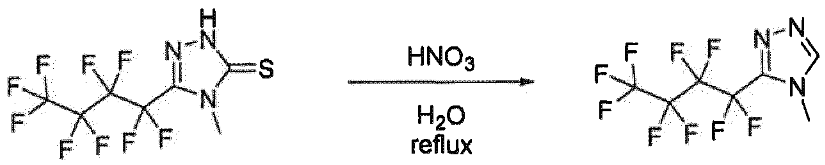


15.7 g of the concentrate (containing 4-methyl-5-(nonafluorobutyl)-2,4-dihydro-3H-1,2,4-triazole-3-thione) obtained in step 3 was suspended in water (424 ml), and the suspension was stirred at room temperature. Nitric acid (47 ml) was added thereto, and the mixture was stirred under heating to reflux for 4 hours. The obtained solution was allowed to cool to room temperature and then adjusted to pH 12 with a 30% aqueous sodium hydroxide solution under cooling in an ice bath, followed by extraction with chloroform. The obtained organic layer was dried over anhydrous magnesium sulfate and filtered. The filtrate was concentrated under reduced pressure, and the obtained concentrate was purified by silica gel column chromatography to obtain 10.5 g of the title compound (yield: 75%, 4 steps).

$^1$H-NMR of the obtained title compound will be shown below.

$^1$H-NMR(400 MHz, $CDCl_3$): $\delta$ 8.25 (s, 1H), 3.86 (s, 3H) .

(Step 5) Synthesis of 3-bromo-4-methyl-5-(nonafluorobutyl)-4H-1,2,4-triazole

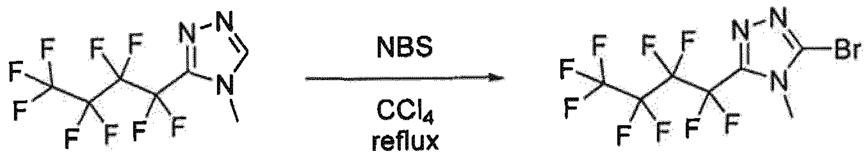


4-Methyl-3-(nonafluorobutyl)-4H-1,2,4-triazole (3.44 g) was dissolved in carbon tetrachloride (114 mL), and the solution was stirred at room temperature. N-Bromosuccinimide (2.24 g) was added thereto, and the mixture was stirred under heating to reflux for 20 hours. The obtained solution was allowed to cool to room temperature and then concentrated under reduced pressure. The obtained concentrate was purified by silica gel column chromatography to obtain 2.49 g of the title compound (yield: 57%).

$^1$H-NMR of the obtained title compound will be shown below.

$^1$H-NMR(400 MHz, $CDCl_3$): δ 3.79 (s, 3H).

(Step 6) Synthesis of 2-chloro-1-(4-methyl-5-(nonafluorobutyl)-4H-1,2,4-triazol-3-yl)ethan-1-one

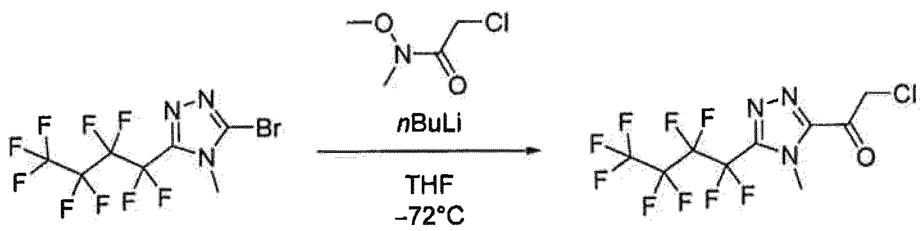


3-Bromo-4-methyl-5-(nonafluorobutyl)-4H-1,2,4-triazole (1.50 g) was dissolved in tetrahydrofuran (16 ml), and the reaction system was replaced with nitrogen and then cooled to -72°C. n-Butyllithium (2.76 M, a solution in n-hexane, 1.70 ml) was added dropwise thereto, and the mixture was stirred at -72°C for 30 minutes. A solution of 2-chloro-N-methoxy-N-methylacetamide (0.652 g) in tetrahydrofuran (4 ml) was added dropwise thereto, and the mixture was stirred at -72°C for 1 hour. The obtained solution was poured into a saturated aqueous solution of ammonium chloride, followed by extraction with ethyl acetate. The obtained organic layer was washed with saturated brine, dried over anhydrous magnesium sulfate, and filtered. The filtrate was concentrated under reduced pressure, and the obtained concentrate was purified by silica gel column chromatography to obtain 0.90 g of the title compound (yield: 60%).

$^1$H-NMR of the obtained title compound will be shown below.

$^1$H-NMR(400 MHz, $CDCl_3$) : δ 5.04 (s, 2H), 4.13 (s, 3H) .

(Step 7) Synthesis of 2-(ethylthio)-1-(4-methyl-5-(nonafluorobutyl)-4H-1,2,4-triazol-3-yl)ethan-1-one

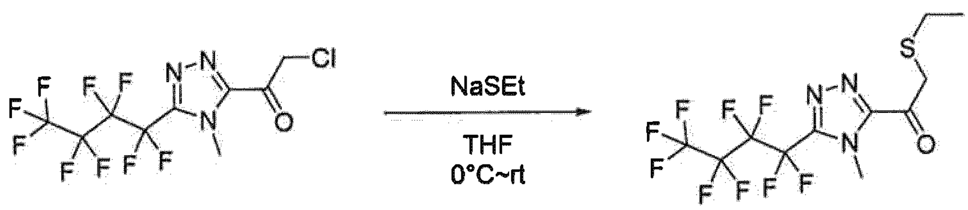


2-Chloro-1-(4-methyl-5-(nonafluorobutyl)-4H-1,2,4-triazol-3-yl)ethan-1-one (0.90 g) was dissolved in tetrahydrofuran (24 ml), and the solution was stirred at room temperature. Sodium ethyl mercaptan (90%, 0.245 g) was added thereto, and the mixture was stirred at room temperature for 6 hours. Sodium ethyl mercaptan (90%, 0.245 g) was further added thereto, and the mixture was stirred at room temperature for 1 hour. The obtained solution was poured into a

saturated aqueous solution of ammonium chloride, followed by extraction with ethyl acetate. The obtained organic layer was washed with saturated brine, dried over anhydrous magnesium sulfate, and filtered. The filtrate was concentrated under reduced pressure.

(Step 8) Synthesis of 2-(ethylsulfonyl)-1-(4-methyl-5-(nonafluorobutyl)-4H-1,2,4-triazol-3-yl)ethan-1-one

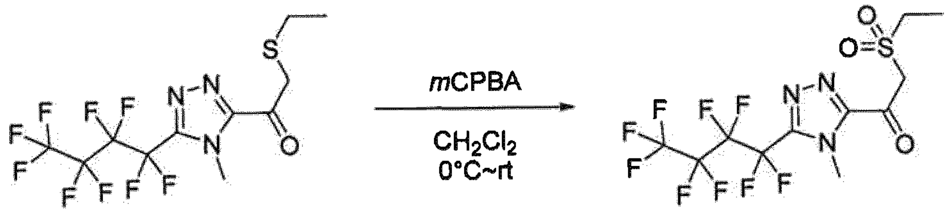


0.98 g of the concentrate (containing 2-(ethylthio)-1-(4-methyl-5-(nonafluorobutyl)-4H-1,2,4-triazol-3-yl)ethan-1-one) obtained in step 7 was dissolved in dichloromethane (24 ml), and the solution was stirred at 0°C. m-Chloroperbenzoic acid (70%, 1.32 g) was added thereto, and the mixture was stirred at room temperature for 3 hours. The obtained solution was poured into a mixed solution of a saturated aqueous solution of sodium bicarbonate and a saturated aqueous solution of sodium thiosulfate, followed by extraction with chloroform. The obtained organic layer was dried over anhydrous magnesium sulfate and filtered. The filtrate was concentrated under reduced pressure. The obtained concentrate was purified by silica gel column chromatography to obtain 0.807 g of the title compound (yield: 78%, 2 steps).
$^1$H-NMR of the obtained title compound will be shown below.
$^1$H-NMR(400 MHz, $CDCl_3$) : δ 4.92 (s, 2H), 4.11 (s, 3H), 3.31 (q, 2H), 1.48 (t, 3H).

(Step 9) Synthesis of 3-(dimethylamino)-2-(ethylsulfonyl)-1-(4-methyl-5-(nonafluorobutyl)-4H-1,2,4-triazol-3-yl)prop-2-en-1-one

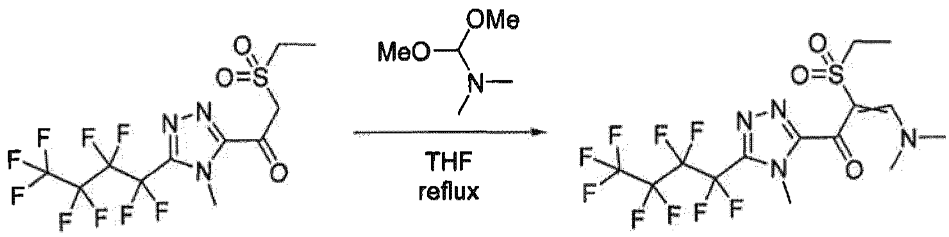


2-(Ethylsulfonyl)-1-(4-methyl-5-(nonafluorobutyl)-4H-1,2,4-triazol-3-yl)ethan-1-one (0.20 g) was dissolved in tetrahydrofuran (2.3 ml), and the solution was stirred at room temperature. N,N-Dimethylformamide dimethyl acetal (0.274 g) was added thereto, and the mixture was stirred under heating to reflux for 1.5 hours. The obtained solution was concentrated under reduced pressure.

(Step 10) Synthesis of 5-(ethylsulfonyl)-4-(4-methyl-5-(nonafluorobutyl)-4H-1,2,4-triazol-3-yl)-2,2'-bipyrimidine

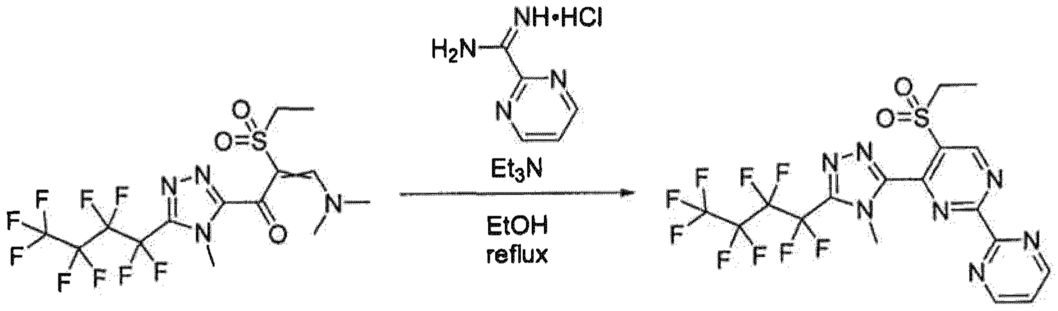


0.27 g of the concentrate (containing 3-(dimethylamino)-2-(ethylsulfonyl)-1-(4-methyl-5-(nonafluorobutyl)-4H-1,2,4-triazol-3-yl)prop-2-en-1-one) obtained in step 9 was dissolved in ethanol (2.3 ml), and the solution was stirred at room temperature. Triethylamine (0.209 g) and 2-amidinopyrimidine hydrochloride (0.109 g) were added thereto, and the mixture was stirred under heating to reflux for 1 hour. The obtained solution was poured into a saturated aqueous solution of ammonium chloride, followed by extraction with chloroform. The obtained organic layer was dried over anhydrous magnesium sulfate and filtered. The filtrate was concentrated under reduced pressure, and the obtained concentrate was purified by silica gel column chromatography to obtain 0.10 g of the title compound (yield: 40%, 2 steps).

$^{1}H$-NMR and $^{19}F$-NMR of the obtained title compound will be shown below.

$^{1}H$-NMR(400 MHz, $CDCl_3$) : $\delta$ 9.70 (s, 1H), 9.09 (d, 2H), 7.56 (t, 1H), 3.92 (s, 3H), 3.83 (q, 2H), 1.42 (t, 3H) ; $^{19}F$-NMR (376 MHz, $CDCl_3$-$C_6F_6$) : $\delta$-81.28 (t, 3F), -109.73 (t, 2F), -122.00 - -122.09 (m, 2F), -125.72 - -125.81 (m, 2F).

Some compounds of the present invention produced in the same way as in Examples described above are shown in Tables 1 to 3. Table 1 shows the substituents of the compound represented by the formula (I). In the tables, properties, melting point (m.p.), or refractive index ($n_D$) are also shown as the physical properties of each compound.

In the tables, Me represents a methyl group, Et represents an ethyl group, $^{c}Pr$ represents a cyclopropyl group, and Ac represents an acetyl group.

[Table 1]

| Compound No. | Structure | Physical properties |
|---|---|---|
| a-1 | 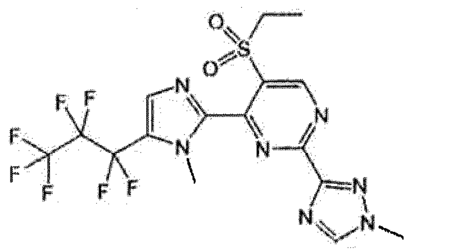 | m.p.: 186-188 (°C) |
| a-2 | 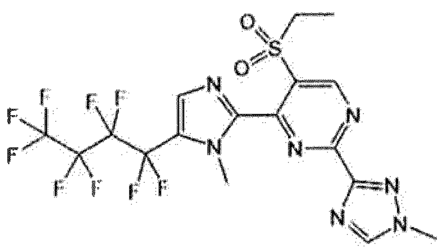 | m.p.: 188-190 (°C) |
| a-3 | 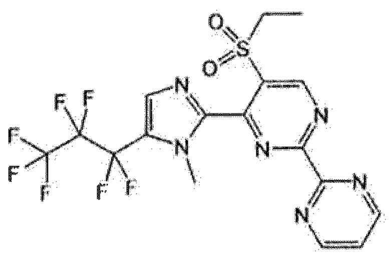 | m.p.: 207-209 (°C) |

(continued)

| Compound No. | Structure | Physical properties |
|---|---|---|
| a-4 | 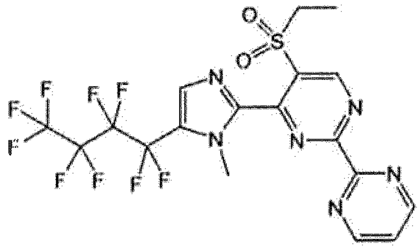 | m.p.: 208-210 (°C) |
| a-5 | 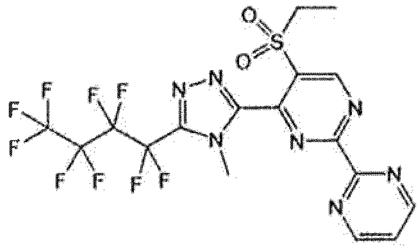 | m.p.: 172-174 (°C) |
| a-6 | 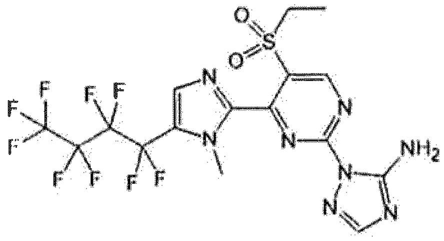 | m.p.: 208-210 (°C) |
| a-7 | 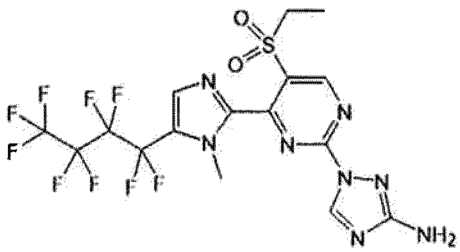 | m.p.: 219-221 (°C) |
| a-8 | 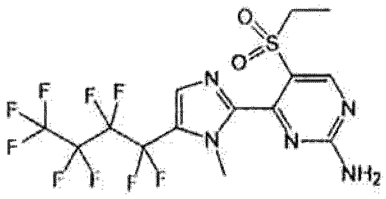 | m.p.: 184-186 (°C) |
| a-9 | 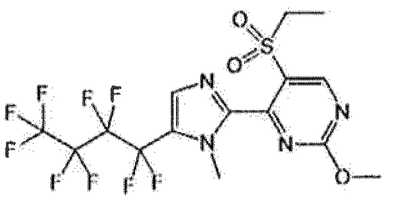 | m.p.: 86-88 (°C) |

(continued)

| Compound No. | Structure | Physical properties |
|---|---|---|
| a-10 | 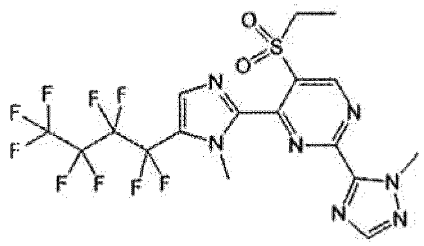 | amorhous |

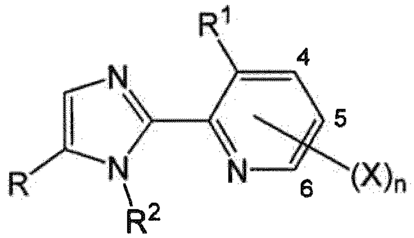

(I)

[Table 2]

| Compound No. | $(X)_n$ | $R^1$ | $R^2$ | R | Physical properties |
|---|---|---|---|---|---|
| b-1 | 5-$^{c}$Pr | $SO_2Et$ | Me | $CF_2CF_2CF_2CF_3$ | m.p.: 138-140(°C) |
| b-2 | - | SEt | Me | $CF_2CF_2CF_2CF_3$ | nd(20.8°C): 1.4817 |
| b-3 | - | $SO_2Et$ | Me | $CF_2CF_2CF_2CF_3$ | m.p.: 83-85(°C) |
| b-4 | 5-(pyrimidin-2-yl) | $SO_2Et$ | Me | $CF_2CF_2CF_2CF_3$ | m.p.: 114-116(°C) |
| b-5 | 6-(1H-1,2,4-triazol-1-yl) | $SO_2Et$ | Me | $CH(OAc)CF_2CF_2CF_2CF_3$ | m.p.: 183-185(°C) |
| b-6 | 6-(1H-1,2,4-triazol-1-yl) | $SO_2Et$ | Me | $CH(OH)CF_2CF_2CF_2CF_3$ | m.p.: 179-181(°C) |
| b-7 | 5-$CF_3$ | $SO_2Et$ | Me | $CF_2CF_2CF_2CF_3$ | m.p.: 88-90(°C) |
| b-8 | 6-(1H-1,2,4-triazol-1-yl) | $SO_2Et$ | Me | $CF_2CF_2CF_2CF_3$ | m.p.: 129-131(°C) |
| b-9 | 6-(pyrimidin-2-yl) | $SO_2Et$ | Me | $CF_2CF_2CF_2CF_3$ | m.p.: 125-127(°C) |
| b-10 | 6-(1H-1,2,4-triazol-1-yl) | $SO_2Et$ | Me | $CHFCF_2CF_2CF_2CF_3$ | m.p.: 143-145(°C) |
| b-11 | - | $SO_2Et$ | Me | $CHFCF_2CF_2CF_2CF_3$ | m.p.: 78-80(°C) |
| b-12 | 6-$NH_2$ | $SO_2Et$ | Me | $CF_2CF_2CF_2CF_3$ | m.p.: 53-55(°C) |
| b-13 | 6-(1H-1,2,4-triazol-1-yl) | $SO_2Et$ | Me | $CClHCF_2CF_2CF_2CF_3$ | m.p.: 162-164(°C) |
| b-14 | - | $SO_2Et$ | Me | $CClHCF_2CF_2CF_2CF_3$ | m.p.: 94-96(°C) |
| b-15 | 5-$CF_3$ | $SO_2Et$ | Me | $CF_2CF_2CF_3$ | m.p.: 109-110(°C) |
| b-16 | 6-(1H-1,2,4-triazol-1-yl) | $SO_2Et$ | Me | $CF_2CF_2CF_3$ | m.p.: 147-149(°C) |
| b-17 | 6-(1H-1,2,4-triazol-1-yl) | $SO_2Et$ | Me | $CF_2CF_3$ | m.p.: 151-152(°C) |
| b-18 | 5-$^{c}$Pr | $SO_2Et$ | Me | $CHFCF_2CF_2CF_3$ | m.p.: 106-108(°C) |
| b-19 | 5-(pyrimidin-2-yl) | $SO_2Et$ | Me | $CF_2CF_2CF_3$ | m.p.: 133-135(°C) |
| b-20 | 5-$^{c}$Pr | $SO_2Et$ | Me | $CF_2CF_2CF_3$ | m.p.: 118-120(°C) |
| b-21 | 5-(pyrimidin-2-yl) | $SO_2Et$ | Me | $CHFCF_2CF_2CF_3$ | m.p.: 164-167(°C) |
| b-22 | 5-(pyrimidin-2-yl) | $SO_2Et$ | Me | $CF_2CF_3$ | m.p.: 148-150(°C) |
| b-23 | 5-Br | $SO_2Et$ | Me | $CHFCF_2CF_3$ | m.p.: 177-180(°C) |
| b-24 | 5-(pyrimidin-2-yl) | $SO_2Et$ | Me | $CHFCF_2CF_3$ | m.p.: 170-173(°C) |
| b-25 | 5-$^{c}$Pr | $SO_2Et$ | Me | $CHFCF_2CF_3$ | m.p.: 130-132(°C) |

| | | | | | |
|---|---|---|---|---|---|
| b-26 | 5-$^{c}$Pr | $SO_2Et$ | Me | $CF_2CF_3$ | m.p.: 148-150(°C) |
| b-27 | 5-(pyridin-2-yl) | $SO_2Et$ | Me | $CHFCF_2CF_3$ | m.p.: 128-131(°C) |
| b-28 | 5-(1H-1,2,4-triazol-1-yl) | $SO_2Et$ | Me | $CHFCF_2CF_3$ | amorphous |
| b-29 | 5-(pyridin-2-yl) | $SO_2Et$ | Me | $CF_2CF_2CF_3$ | m.p.: 116-118(°C) |
| b-30 | 5-(1-CN-2-OEt-2-oxoethyl) | $SO_2Et$ | Me | $CF_2CF_2CF_3$ | m.p.: 164-166(°C) |
| b-31 | 5-(pyrimidin-2-yl) | $SO_2Et$ | Me | $CHFCF(CF_3)_2$ | white solid |
| b-32 | 5-(pyrimidin-2-yl) | $SO_2Et$ | Me | $CHClCF_2CF_3$ | m.p.: 149-151(°C) |
| b-33 | 5-(thiazol-2-yl) | $SO_2Et$ | Me | $CHFCF_2CF_3$ | m.p.: 138-141(°C) |
| b-34 | 5-(3,5-$F_2$-phenyl) | $SO_2Et$ | Me | $CHFCF_2CF_3$ | m.p.: 134-136(°C) |
| b-35 | 5-(pyrimidin-5-yl) | $SO_2Et$ | Me | $CHFCF_2CF_3$ | m.p.: 128-131(°C) |
| b-36 | 5-(3-$CF_3$-1H-pyrazol-1-yl) | $SO_2Et$ | Me | $CF_2CF_2CF_3$ | m.p.: 111-113(°C) |
| b-37 | 5-(pyridin-2-yloxy) | $SO_2Et$ | Me | $CF_2CF_2CF_3$ | viscous oil |
| b-38 | 5-(2-oxopyridin-1(2H)-yl) | $SO_2Et$ | Me | $CF_2CF_2CF_3$ | m.p.: 147-150(°C) |
| b-39 | 5-vinyl | $SO_2Et$ | Me | $CHFCF_2CF_3$ | m.p.: 128-130(°C) |
| b-40 | 5-formyl | $SO_2Et$ | Me | $CHFCF_2CF_3$ | viscous oil |
| b-41 | 5-(NHCO$^{c}$Pr) | $SO_2Et$ | Me | $CF_2CF_2CF_3$ | m.p.: 215-217(°C) |
| b-42 | 5-(2-CN-propan-2-yl) | $SO_2Et$ | Me | $CF_2CF_2CF_3$ | m.p.: 138-140(°C) |
| b-43 | 5-(pyridin-2-yl) | $SO_2Et$ | Me | $CHFCF(CF_3)_2$ | m.p.: 141-143(°C) |
| b-44 | 5-(pyridin-2-yl) | $SO_2Et$ | Me | $CHClCF(CF_3)_2$ | m.p.: 160-162(°C) |
| b-45 | 5-(1H-1,2,4-triazol-1-yl) | $SO_2Et$ | Me | $CF_2CF_2CF_3$ | m.p.: 145-147(°C) |
| b-46 | 6-(pyrimidin-2-yl) | $SO_2Et$ | Me | $CHFCF(CF_3)_2$ | m.p.: 145-147(°C) |
| b-47 | 6-(1H-1,2,4-triazol-1-yl) | $SO_2Et$ | Me | $CHFCF(CF_3)_2$ | m.p.: 141-143(°C) |
| b-48 | 5-(pyrimidin-2-yl) | $SO_2Et$ | Me | $CH_2CH(CF_3)_2$ | m.p.: 184-187(°C) |
| b-49 | 5-$CF_3$ | $SO_2Et$ | Me | $CHFCF(CF_3)_2$ | m.p.: 122-124(°C) |
| b-50 | - | $SO_2Et$ | Me | $CHFCF(CF_3)_2$ | m.p.: 94-97(°C) |

| | | | | | |
|---|---|---|---|---|---|
| b-51 | 5-(1-CN-$^{c}$Pr) | $SO_2Et$ | Me | $CF_2CF_2CF_3$ | m.p.: 158-160(°C) |
| b-52 | 5-$^{c}$Pr | $SO_2Et$ | Me | $CHFCF(CF_3)_2$ | m.p.: 125-127(°C) |
| b-53 | 5-$^{c}$Pr | $SO_2Et$ | Me | $CHClCF(CF_3)_2$ | m.p.: 123-125(°C) |
| b-54 | 5-(3-CN-pyridin-2-yl) | $SO_2Et$ | Me | $CF_2CF_2CF_3$ | m.p.: 118-120(°C) |
| b-55 | 5-(3-F-pyridin-2-yl) | $SO_2Et$ | Me | $CF_2CF_2CF_3$ | m.p.: 161-163(°C) |
| b-56 | 5-(1-CN-$^{c}$Pr) | $SO_2Et$ | Me | $CHFCF(CF_3)_2$ | amorphous |
| b-57 | 5-(1-(pyridin-2-yl)-$^{c}$Pr) | $SO_2Et$ | Me | $CHFCF(CF_3)_2$ | m.p.: 71-75(°C) |
| b-58 | 5-(2-F-phenyl) | $SO_2Et$ | Me | $CF_2CF_2CF_3$ | m.p.: 128-130(°C) |
| b-59 | 5-(pyridin-2-yl) | $SO_2Et$ | Me | $CF_2CF_2CF_2CF_3$ | m.p.: 90-92(°C) |
| b-60 | 5-(3,5-$F_2$-phenyl) | $SO_2Et$ | Me | $CF_2CF_2CF_3$ | m.p.: 133-135(°C) |
| b-61 | 5-(3-F-pyridin-2-yl) | $SO_2Et$ | Me | $CF_2CF_2CF_2CF_3$ | m.p.: 124-126(°C) |
| b-62 | 5-(3-Cl-pyridin-2-yl) | $SO_2Et$ | Me | $CF_2CF_2CF_3$ | m.p.: 138-140(°C) |
| b-63 | 5-$^{c}$Pr | $SO_2Et$ | Me | $CHFCF_2CF_2CF_2CF_3$ | m.p.: 135-138(°C) |
| b-64 | 5-(pyrimidin-2-yl) | $SO_2Et$ | Me | $CHFCF_2CF_2CF_2CF_3$ | m.p.: 170-172(°C) |
| b-65 | 5-$^{c}$Pr | $SO_2Et$ | Me | $CH_2CH(CF_3)_2$ | viscous oil |
| b-66 | 5-(pyridin-2-yl) | $SO_2Et$ | Me | $CHFCF_2CF_2CF_2CF_3$ | m.p.: 71-75(°C) |
| b-67 | 5-(pyrimidin-2-yl) | $SO_2Et$ | Me | $CHFCH_2CH_2CF_3$ | white solid |
| b-68 | 5-($OCH_2CF_2CF_3$) | $SO_2Et$ | Me | $CHFCF(CF_3)_2$ | $n_D$ ( 21.6 °C ) 1.4513 |
| b-69 | 5-($OCMe_2CF_3$) | $SO_2Et$ | Me | $CHFCF(CF_3)_2$ | m.p.: 71-75(°C) |
| b-70 | 5-$^{c}$Pr | $SO_2Et$ | Me | $CH_2OCH_2CF_2CF_3$ | m.p.: 71-75(°C) |
| b-71 | 5-$^{c}$Pr | $SO_2Et$ | Me | $CH_2OCH_2CF_2CF_2CF_3$ | m.p.: 71-75(°C) |
| b-72 | 5-$^{c}$Pr | $SO_2Et$ | Me | $CH_2OCF_2CHFOCF_3$ | viscous oil |

[Table 3]

| Compound No. | Structure | Physical properties |
|---|---|---|
| c-1 | 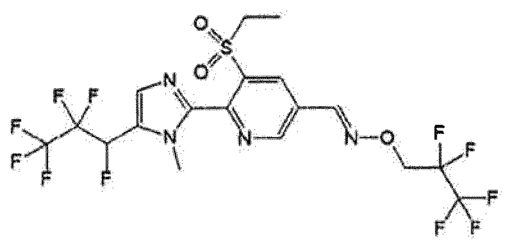 | m.p.: 140-142 (°C) |

(continued)

| Compound No. | Structure | Physical properties |
|---|---|---|
| c-2 | 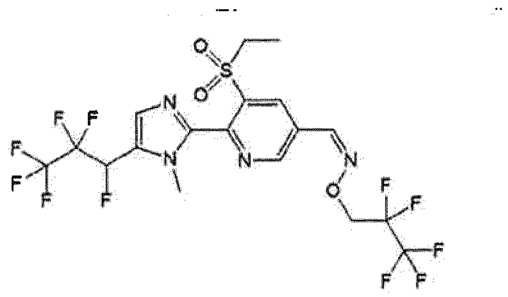 | white solid |
| o-3 | 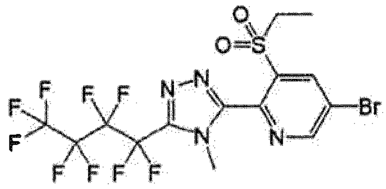 | m.p.: 74-76 (°C) |
| c-4 | 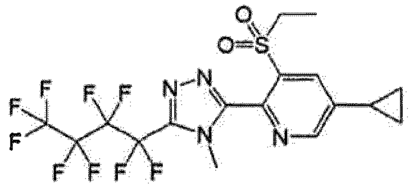 | m.p.: 126-128 (°C) |
| c-5 | 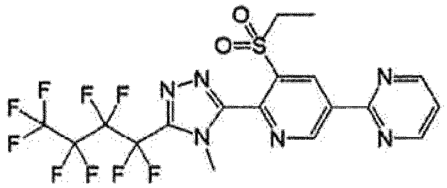 | m.p.: 151-153 (°C) |
| c-6 | 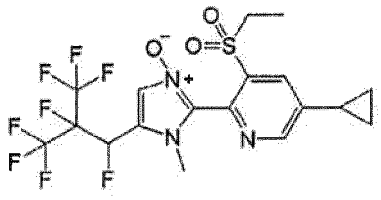 | m.p.: 186-191 (°C) |
| c-7 | 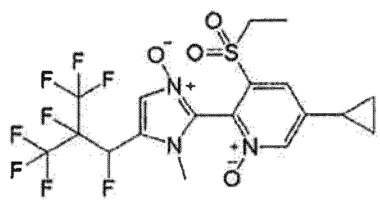 | m.p.: 203-205 (°C) |
| c-8 | 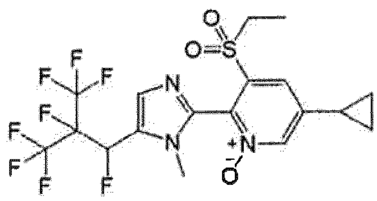 | m.p.: 174-178 (°C) |

(continued)

| Compound No. | Structure | Physical properties |
|---|---|---|
| c-9 | 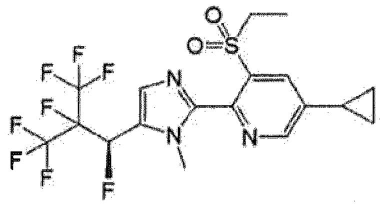 | |
| c-10 | 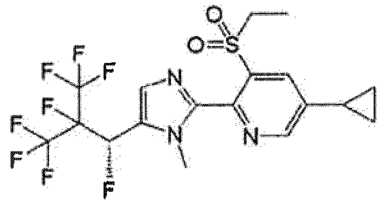 | |
| c-11 | 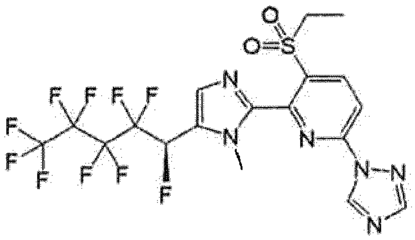 | |
| c-12 | 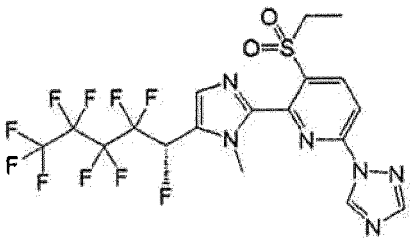 | |
| c-13 | 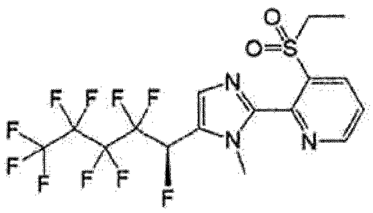 | |
| c-14 | 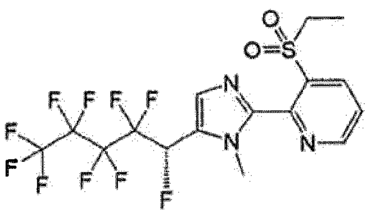 | |

(continued)

| Compound No. | Structure | Physical properties |
|---|---|---|
| c-15 | 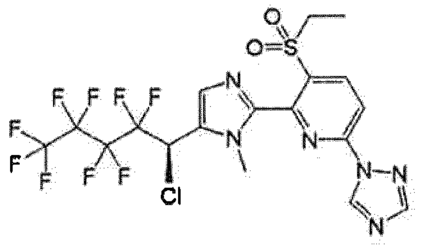 | |
| c-16 | 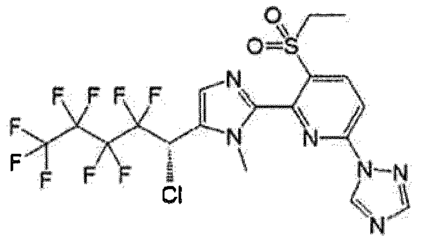 | |
| c-17 | 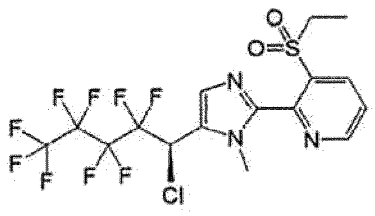 | |
| c-18 | 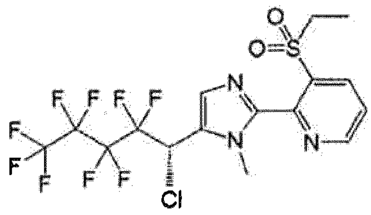 | |
| c-19 | 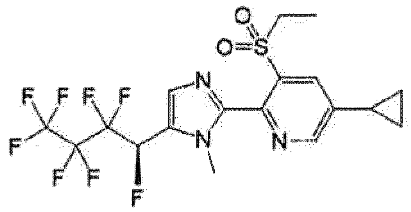 | |
| c-20 | 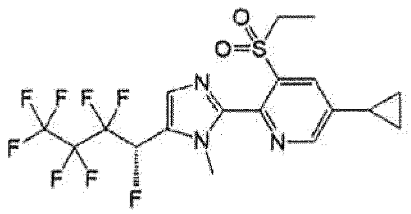 | |

(continued)

| Compound No. | Structure | Physical properties |
|---|---|---|
| c-21 | 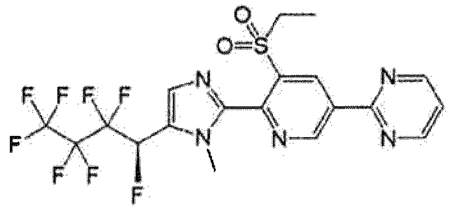 | |
| c-22 | 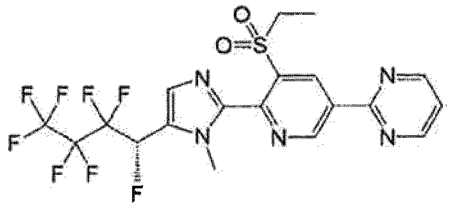 | |
| c-23 | 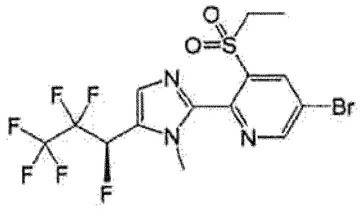 | |
| c-24 | 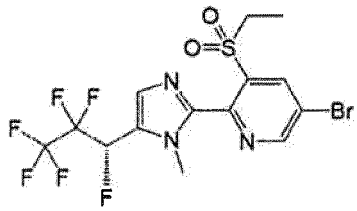 | |
| c-25 | 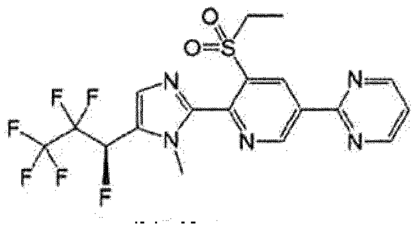 | |
| c-26 | 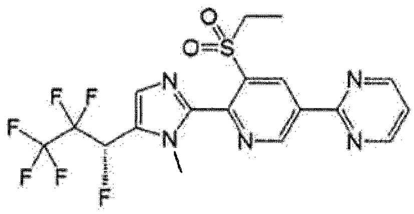 | |

(continued)

| Compound No. | Structure | Physical properties |
|---|---|---|
| c-27 | 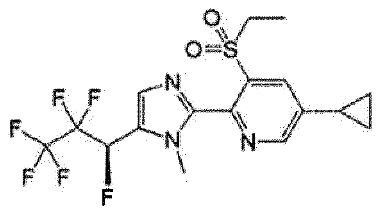 | |
| c-28 | 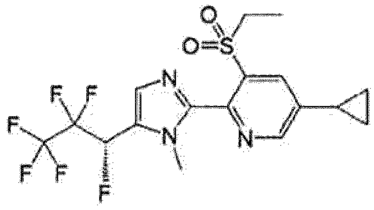 | |
| c-29 | 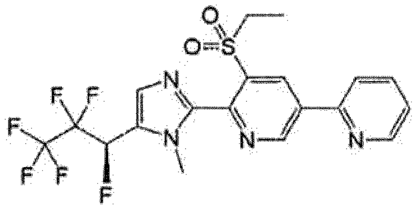 | |
| c-30 | 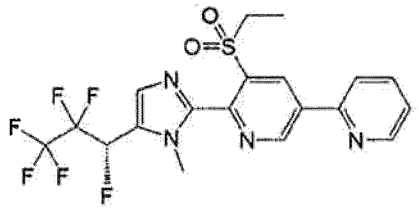 | |
| c-31 | 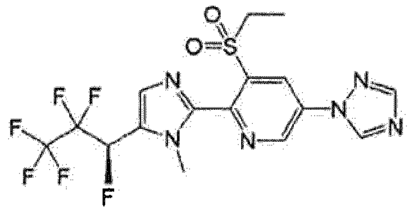 | |
| c-32 | 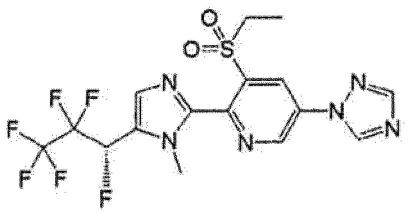 | |
| c-33 | 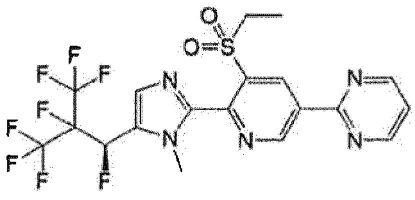 | |

(continued)

| Compound No. | Structure | Physical properties |
|---|---|---|
| c-34 | 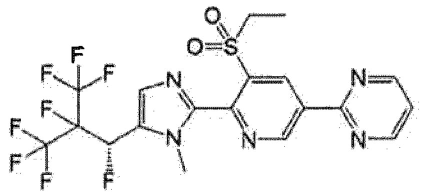 | |
| c-35 | 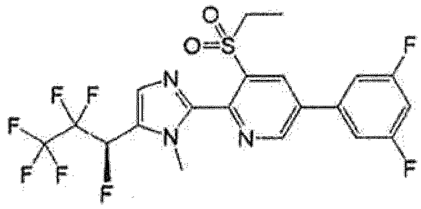 | |
| c-36 | 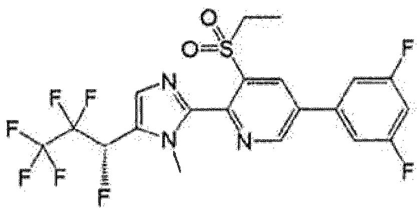 | |
| c-37 | 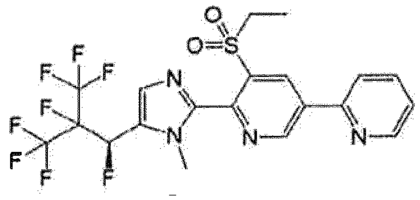 | |
| c-38 | 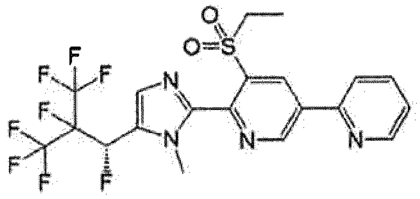 | |
| c-39 | 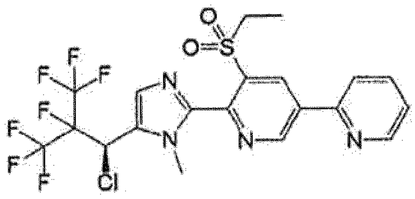 | |
| c-40 | 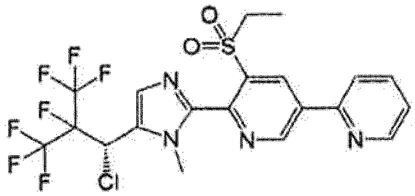 | |

(continued)

| Compound No. | Structure | Physical properties |
|---|---|---|
| c-41 | 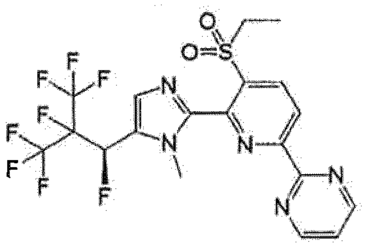 | |
| c-42 | 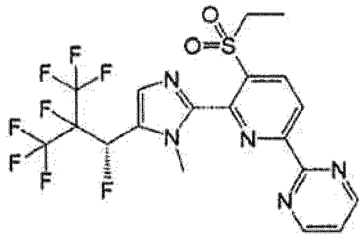 | |
| c-43 | 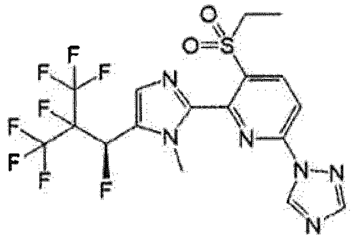 | |
| c-44 | 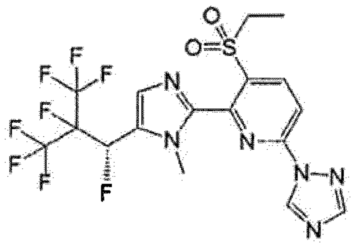 | |
| c-45 | 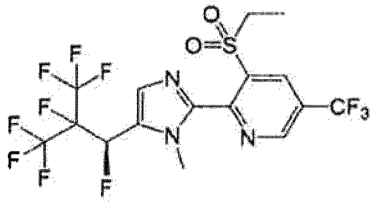 | |
| c-46 | 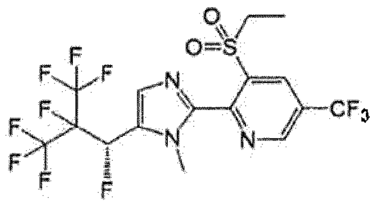 | |

(continued)

| Compound No. | Structure | Physical properties |
|---|---|---|
| c-47 | 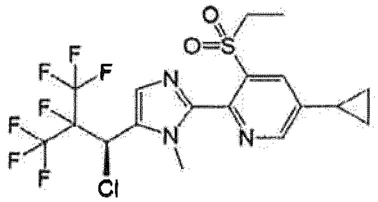 | |
| c-48 | 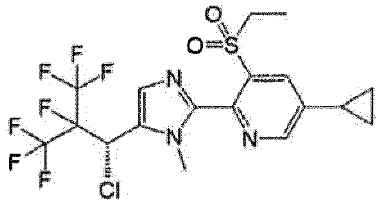 | |
| c-49 | 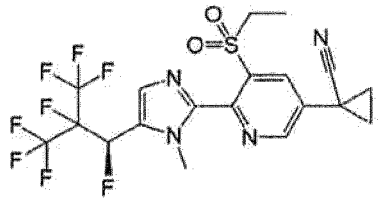 | |
| c-50 | 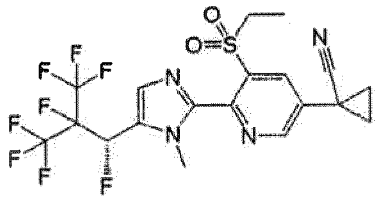 | |
| c-51 | 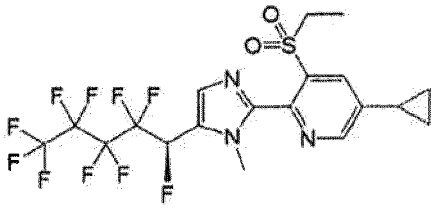 | |
| c-52 | 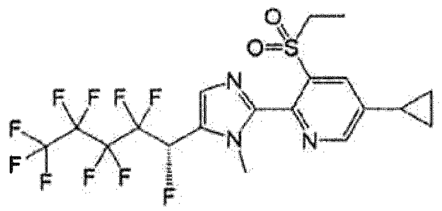 | |
| c-53 | 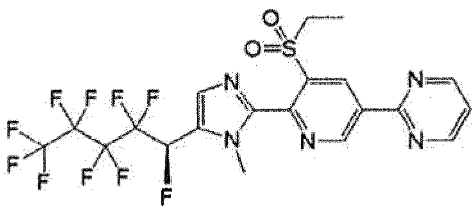 | |

(continued)

| Compound No. | Structure | Physical properties |
|---|---|---|
| c-54 | 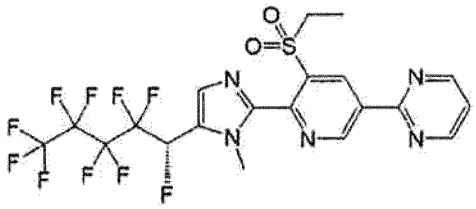 | |
| c-55 | 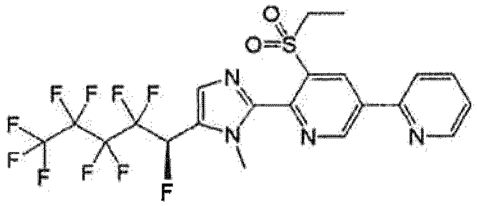 | |
| c-56 | 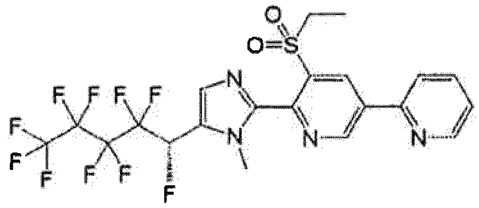 | |
| c-57 | 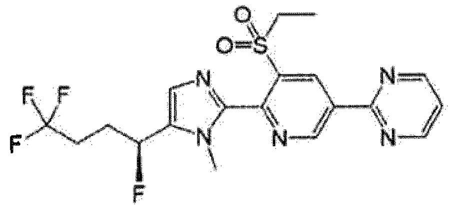 | |
| c-58 | 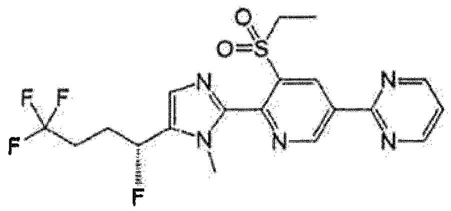 | |
| c-59 | 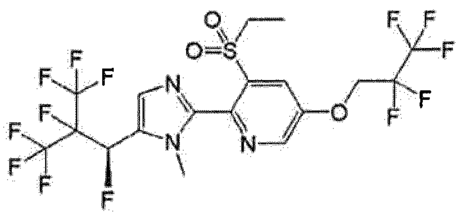 | |
| c-60 | 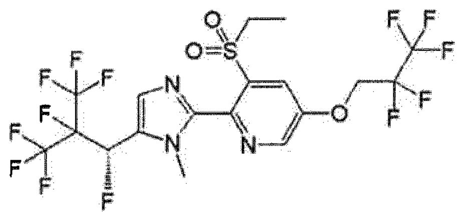 | |

(continued)

| Compound No. | Structure | Physical properties |
|---|---|---|
| c-61 | 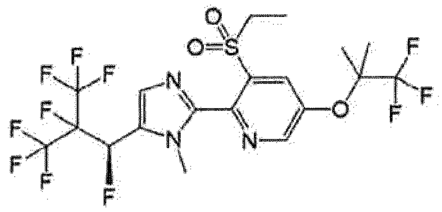 | |
| c-62 | 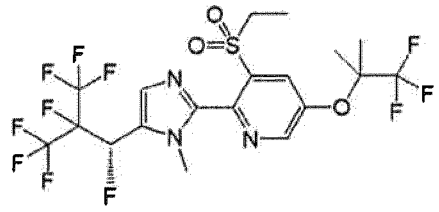 | |

The $^1$H-NMR data of compounds having physical properties of viscous oil, amorphous or white solid among the compounds shown in Tables 1 to 3 will be shown below. Compound No. a-10: $^1$H-NMR ($CDCl_3$) δ: 9.55 (1H, s), 8.10 (1H, s), 7.56 (1H, s), 4.44 (3H, s), 3.93 (2H, q), 3.91 (3H, s), 1.42 (3H, t).
Compound No. b-28: $^1$H-NMR(400 MHz, CDCl3) δ: 9.37 (d, 1H), 8.80 (s, 1H), 8.78 (d, 1H), 8.25 (s, 1H), 7.44 (d, 1H), 5.92 (ddd, 1H), 3.88 (q, 2H), 3.70 (s, 3H), 1.38 (t, 3H).
Compound No. b-31: $^1$H-NMR(400 MHz, CDCl3) δ: 9.93 (d, 1H), 9.47 (d, 1H), 8.91 (d, 2H), 7.40 (dd, 1H), 7.37 (t, 1H), 6.10 (ddd, 1H), 3.80 (q, 2H), 3.67 (d, 3H), 1.37 (t, 3H).
Compound No. b-37: $^1$H-NMR(400 MHz, CDCl3) δ: 8.83 (d, 1H), 8.31 (d, 1H), 8.19 (dd, 2H), 7.83 (dt, 1H), 7.50 (s, 1H), 7.16 (dd, 1H), 7.12 (d, 1H), 3.70 (q, 2H), 3.67 (s, 3H), 1.33 (t, 3H).
Compound No. b-40: $^1$H-NMR(400 MHz, CDC13) δ: 10.28 (s, 1H), 9.35 (d, 1H), 8.92 (d, 1H), 7.45 (d, 1H), 5.90 (ddd, 1H), 3.96-3.86 (m, 2H), 3.74 (s, 3H), 1.37 (t, 3H).
Compound No. b-56: $^1$H-NMR(400 MHz, CDCl3) δ: 8.99 (1H, d), 8.19 (1H, d), 7.37 (1H, dd), 6.08 (1H, dd), 3.76 (2H, q), 3.62 (3H, s), 2.00 (2H, dd), 1.63 (2H, dd), 1.31 (3H, t).
Compound No. b-65: $^1$H-NMR(400 MHz, CDC13) δ: 8.67 (1H, d), 8.01 (1H, d), 7.01 (1H, s), 3.71 (2H, q), 3.49 (3H, s), 3.30-3.17 (3H, m), 2.10-2.03 (1H, m), 1.28 (3H, t), 1.24-1.19 (2H, m), 0.93-0.88 (2H, m).
Compound No. b-67: $^1$H-NMR(400 MHz, CDCl3) δ: 9.91 (1H, d), 9.47 (1H, d), 8.91 (2H, d), 7.36 (1H, t), 7.23 (1H, d), 5.59 (1H, ddd), 3.92-3.80 (2H, m), 3.69 (3H, s), 2.56-2.33 (4H, m), 1.38 (3H, t) .
Compound No. b-72: $^1$H-NMR(400 MHz, CDCl3) δ: 8.68 (1H, d), 8.02 (1H, d), 7.21 (1H, s), 5.76 (1H, dt), 5.09 (2H, s), 3.74 (2H, q), 3.55 (3H, s), 2.11-2.05 (1H, m), 1.30 (3H, t), 1.25-1.16 (2H, m), 0.96-0.89 (2H, m).
Compound No. c-2: $^1$H-NMR(400 MHz, CDCl3) δ:9.31 (d, 1H), 8.89 (d, 1H), 7.58 (s, 1H), 7.43 (d, 1H), 5.91 (ddd, 1H), 4.79 (t, 2H), 3.89-3.78 (m, 2H), 3.71 (s, 3H), 1.34 (t, 3H) .

[Biological test]

Test Examples given below show that the heteroaryl azole compound of the present invention is useful as an active ingredient for pest control agents and ectoparasite control agents. The term "part" is based on weight.

(Preparation of test emulsion)

5 parts of the heteroaryl azole compound of the present invention, 93.6 parts of dimethylformamide, and 1.4 parts of polyoxyethylene alkyl aryl ether were mixed and dissolved to prepare emulsion (I) containing 5% of the active ingredient.
For a control, 98.5 parts of dimethylformamide and 1.5 parts of polyoxyethylene alkyl aryl ether were mixed and dissolved to prepare emulsion (II).
An insecticidal rate was calculated according to the following expression:

$$\text{Insecticidal rate (\%)} = (\text{The number of dead insects} / \text{The number of tested insects}) \times 100$$

(Test Example 1) Test of efficacy on *Mythimna separata*

0.8 g of commercially available artificial feed (Insecta LFS, manufactured by Nosan Corp.) and 1 μl of the emulsion (I) were well mixed to obtain test feed.
A plastic test container (capacity: 1.4 ml) was packed with 0.2 g of the test feed per treatment plot. Then, two second instar larvae of *Mythimna separata* were inoculated to each treatment plot. A plastic lid was put on the test container so as to prevent escape of the second instar larvae of *Mythimna separata.* The container was placed in a thermostat chamber of 25°C. On the fifth day, the insecticidal rate and the food intake were examined. The test was conducted in duplicate.
The insecticidal rate and the food intake of a control plot were examined in the same way as in Test Example 1 except that the emulsion (I) was changed to the emulsion (II).
Compounds of compound Nos. a-1, a-2, a-6, a-8, a-9, b-1, b-2, b-3, b-4, b-7, b-8, b-9, b-10, b-11, b-12, b-13, b-15, b-16, b-17, b-18, b-20, b-22, b-23, b-24, b-25, b-26, b-27, b-29, b-32, b-34, b-35, b-36, b-39, b-41, b-42, b-43, b-44, b-45, b-52, b-53, b-54, b-55, b-57, b-58, b-59, b-60, b-61, b-62, b-63, b-64, b-66, b-68, b-69, c-1, c-5 and c-8 were tested for their efficacy on *Mythimna separata.* All the compounds had an insecticidal rate of 100% for *Mythimna separata* or a food intake of 10% or less as compared with the control plot. As is evident, the heteroaryl azole compound of the present invention is effective for *Mythimna separata.*

(Test Example 2) Test of efficacy on *Mythimna separata*

The emulsion (I) was diluted with water such that the concentration of the compound of the present invention was 125 ppm. Corn leaves were dipped in the dilution for 30 seconds. The resulting corn leaves were placed in a petri dish, and five second instar larvae of *Mythimna separata* were released. The petri dish was placed in a thermostat chamber having a temperature of 25°C and a humidity of 60%. Life and death were determined after 6 days from the release of the insects, and the insecticidal rate was calculated. The test was conducted in duplicate.
Compounds of compound Nos. a-2, b-4, b-18, b-19 and b-21 were tested for their efficacy on *Mythimna separata.* All the compounds exhibited an insecticidal rate of 80% or more for *Mythimna separata.*

(Test Example 3) Test of efficacy on *Plutella xylostella*

The emulsion (I) was diluted with water such that the concentration of the compound of the present invention was 125 ppm. Cabbage leaves were dipped in the dilution for 30 seconds. The resulting cabbage leaves were placed in a petri dish. Five second instar larvae of *Plutella xylostella* were released thereto. The petri dish was placed in a thermostat chamber having a temperature of 25°C and a humidity of 60%. Life and death were determined after 3 days from the release of the insects, and the insecticidal rate was calculated. The test was conducted in duplicate.
Compounds of compound Nos. a-1, a-2, a-4, a-5, a-6, a-10, b-1, b-4, b-8, b-9, b-10, b-13, b-15, b-16, b-18, b-19, b-20, b-21, b-22, b-24, b-25, b-27, b-29, b-31, b-34, b-36, b-37, b-41, b-42, b-43, b-44, b-45, b-47, b-48, b-51, b-52, b-54, b-55, b-56, b-58, b-60, b-61, b-62, b-64, b-67, b-69, c-1, c-4 and c-5 were tested for their efficacy on *Plutella xylostella.* All the compounds exhibited an insecticidal rate of 80% or more for *Plutella xylostella.*

(Test Example 4) Test of efficacy on *Spodoptera litura*

The emulsion (I) was diluted with water such that the concentration of the compound of the present invention was 125 ppm. Cabbage leaves were dipped in the dilution for 30 seconds. The resulting cabbage leaves were placed in a petri dish. Five second instar larvae of *Spodoptera litura* were released thereto. The petri dish was placed in a thermostat chamber having a temperature of 25°C and a humidity of 60%. Life and death were determined after 6 days from the release of the insects, and the insecticidal rate was calculated. The test was conducted in duplicate.
Compounds of compound Nos. b-18, b-20, b-21 and b-51 were tested for their efficacy on *Spodoptera litura.* All the compounds exhibited an insecticidal rate of 80% or more for *Spodoptera litura.*

(Test Example 5) Test of efficacy on *Aphis craccivora*

Seedlings of black-eyed peas were raised in 10-cm pots. *Aphis craccivora* nymphs were inoculated onto primary

leaves. The emulsion (I) was diluted with water such that the concentration of the compound of the present invention was 125 ppm. The dilution was sprayed to the black-eyed peas parasitized by the *Aphis craccivora* nymphs. The black-eyed peas were placed in a thermostat chamber having a temperature of 25°C and a humidity of 60%. Life and death of *Aphis craccivora* were determined after 4 days from the spraying, and the insecticidal rate was calculated. The test was conducted in duplicate.

Compounds of compound Nos. a-1, a-2, a-6, a-9, b-1, b-2, b-3, b-4, b-7, b-8, b-9, b-11, b-12, b-15, b-16, b-17, b-18, b-20, b-22, b-23, b-24, b-25, b-26, b-27, b-29, b-32, b-35, b-36, b-38, b-40, b-41, b-42, b-43, b-44, b-45, b-52, b-53, b-54, b-55, b-57, b-58, b-59, b-60, b-61, b-62, b-63, b-64, b-66, b-69, b-70, b-71, c-7 and c-8 were tested for their efficacy on *Aphis craccivora.* All the compounds exhibited an insecticidal rate of 80% or more for *Aphis craccivora.*

(Test Example 6) Test of efficacy on *Phyllotreta striolata*

The emulsion (I) was diluted with water such that the concentration of the compound of the present invention was 125 ppm to prepare a test chemical. The test chemical was sprayed to Qing geng cai seedlings (at the seventh true leaf stage) planted in 10-cm pots. The Qing geng cai seedlings were dried in air and then placed in a plastic cup. Ten *Phyllotreta striolata* adults were released thereto. The plastic cup was stored in a thermostat chamber having a temperature of 25°C and a humidity of 65%. Life and death were determined after 7 days from the release of the insects, and the insecticidal rate was calculated. The test was conducted in duplicate.

Compounds of compound Nos. a-1, a-2, a-6, b-1, b-4, b-8, b-9, b-18, b-19, b-21, b-22, b-24, b-29, b-31, b-34, b-37, b-43, b-48, b-52, b-55, b-58, b-59, b-61, b-62, b-69, c-1, c-2 and c-5 were tested for their efficacy on *Phyllotreta striolata* adults. All the compounds exhibited an insecticidal rate of 80% or more for *Phyllotreta striolata* adults.

(Test Example 7) Test of efficacy on *Phyllotreta striolata*

The emulsion (I) was diluted with water such that the concentration of the compound of the present invention was 2 ppm to prepare a test chemical. The test chemical was sprayed to Qing geng cai seedlings (at the seventh true leaf stage) planted in 10-cm pots. The Qing geng cai seedlings were dried in air and then placed in a plastic cup. Ten *Phyllotreta striolata* adults were released thereto. The plastic cup was stored in a thermostat chamber having a temperature of 25°C and a humidity of 65%. Life and death were determined after 7 days from the release of the insects, and the insecticidal rate was calculated. The test was conducted in duplicate.

Compounds of compound Nos. a-1, a-2, a-6, b-1, b-4, b-8, b-18, b-19, b-21, b-24, b-29, b-31, b-34, b-43, b-48, b-52, b-55, b-58, b-59, b-61, b-62 and c-1 were tested for their efficacy on *Phyllotreta striolata* adults. All the compounds exhibited an insecticidal rate of 80% or more for *Phyllotreta striolata* adults.

(Test Example 8) Test of efficacy on *Nilaparvata lugens*

The emulsion (I) was diluted with water such that the concentration of the compound of the present invention was 125 ppm. Young seedlings of rice were dipped in the dilution for 30 seconds. The young seedlings of rice were dried in air and then placed in a plastic case. Five second instar larvae of *Nilaparvata lugens* were released thereto. The plastic case was stored in a thermostat chamber having a temperature of 25°C and a humidity of 65%. Life and death were determined after 7 days from the inoculation, and the insecticidal rate was calculated. The test was conducted in duplicate.

Compounds of compound Nos. a-1, a-2, a-3, a-4, a-9, b-3, b-4, b-7, b-9, b-15, b-17, b-19, b-28, b-34, b-46, b-47, b-48, b-51 and b-67 were tested for their efficacy on *Nilaparvata lugens.* All the compounds exhibited an insecticidal rate of 80% or more for *Nilaparvata lugens.*

(Test Example 9) Test of efficacy on *Musca domestica*

The compound of the present invention was diluted with acetone and added dropwise at 100 ppm per g of a cube of sugar. The cube of sugar was placed in a plastic cup. Ten female adults of *Musca domestica* were released, and a lid was put on the plastic cup. The plastic cup was stored at 25°C. Life and death were determined after 24 hours from the release of the insects, and the insecticidal rate was calculated. The test was conducted in duplicate.

A compound of compound No. b-4 was tested for its efficacy on *Musca domestica.* The compound exhibited an insecticidal rate of 80% or more for *Musca domestica.*

All the compounds selected at random from among the heteroaryl azole compounds of the present invention exerted the effect as described above. It may therefore be understood that the heteroaryl azole compound of the present invention, including unillustrated compounds, is a compound having an effect such as a pest control effect, particularly, a miticidal or insecticidal effect. It may also be understood that the heteroaryl azole compound of the present invention

is a compound also having an effect on parasites harmful to humans and animals, such as ectoparasites.

## Claims

1. A compound represented by the formula (I), an N-oxide compound, stereoisomer, tautomer or hydrate thereof or a salt of any of these compounds:

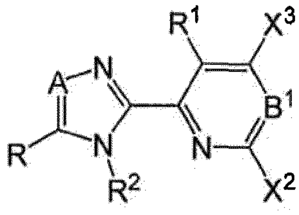

(I)

wherein

A represents CH or a nitrogen atom;
$B^1$ represents $CX^1$ or a nitrogen atom;
$X^1$, $X^2$ and $X^3$ each independently represent a hydrogen atom, a substituted or unsubstituted Cl-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$;
$R^3$ and $R^4$ each independently represent a hydrogen atom, a C1-6 alkyl group, or a C1-6 haloalkyl group;
$R^5$, $R^6$, $R^7$ and $R^8$ each represent a C1-6 alkyl group;
q represents 0 or 1;
$R^1$ represents a substituted or unsubstituted C1-6 alkylthio group, a substituted or unsubstituted C1-6 alkylsulfinyl group, or a substituted or unsubstituted C1-6 alkylsulfonyl group;
$R^2$ represents a substituted or unsubstituted C1-6 alkyl group; and
R represents a substituted or unsubstituted C1-6 alkyl group.

2. The compound according to claim 1, an N-oxide compound, stereoisomer, tautomer or hydrate thereof or a salt of any of these compounds, wherein the formula (I) is the formula (II) :

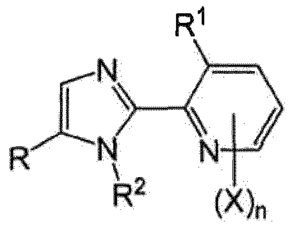

(II)

wherein

$R^1$, $R^2$, and R have the same meanings as described in the formula (I);
X represents a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkoxycarbonyl group, a formyl group, a substituted or unsubstituted C1-6 alkylthio group, a substituted or unsubstituted C1-6 alkylsulfinyl group, a substituted or unsubstituted C1-6

alkylsulfonyl group, a substituted or unsubstituted C3-8 cycloalkyl group, a substituted or unsubstituted C6-10 aryl group, a substituted or unsubstituted 3- to 6-membered heterocyclyl group, a substituted or unsubstituted C6-10 aryloxy group, a substituted or unsubstituted 5- or 6-membered heteroaryloxy group, a substituted or unsubstituted amino group, a substituted or unsubstituted aminocarbonyl group, a substituted or unsubstituted hydrazinyl group, a nitro group, a cyano group, a halogeno group, a group represented by the formula: $-CR^3=N-OR^4$, a group represented by the formula: $-N=CHNR^5R^6$, or a group represented by the formula: $-N=S(O)_qR^7R^8$;
$R^3$ and $R^4$ each independently represent a hydrogen atom, a Cl-6 alkyl group, or a C1-6 haloalkyl group;
$R^5$, $R^6$, $R^7$ and $R^8$ each represent a C1-6 alkyl group;
q represents 0 or 1; and
n represents a chemically acceptable number of X and is any integer of 0 to 3, and when n is 2 or larger, each of the X is the same or different.

3. The compound according to claim 1, an N-oxide compound, stereoisomer, tautomer or hydrate thereof or a salt of any of these compounds, wherein the formula (I) is the formula (III) :

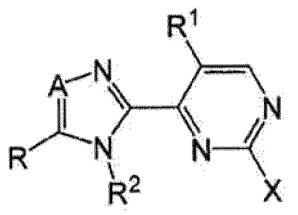
(III)

wherein

A, $R^1$, $R^2$, and R have the same meanings as described in the formula (I); and
X has the same meaning as described in the formula (II) .

4. The compound according to claim 1, an N-oxide compound, stereoisomer, tautomer or hydrate thereof or a salt of any of these compounds, wherein in the formula (I), R represents a C1-6 haloalkyl group.

5. A pest control agent comprising at least one active ingredient selected from the group consisting of a compound according to any one of claims 1 to 4, an N-oxide compound, stereoisomer, tautomer and hydrate thereof and a salt of any of these compounds.

6. An insecticide or miticide comprising at least one active ingredient selected from the group consisting of a compound according to any one of claims 1 to 4, an N-oxide compound, stereoisomer, tautomer and hydrate thereof and a salt of any of these compounds.

7. An ectoparasite control agent comprising at least one active ingredient selected from the group consisting of a compound according to any one of claims 1 to 4, an N-oxide compound, stereoisomer, tautomer and hydrate thereof and a salt of any of these compounds.

8. An endoparasite control agent or expellant comprising at least one active ingredient selected from the group consisting of a compound according to any one of claims 1 to 4, an N-oxide compound, stereoisomer, tautomer and hydrate thereof and a salt of any of these compounds.

9. A seed treatment agent or vegetative propagation organ treatment agent comprising at least one active ingredient selected from the group consisting of a compound according to any one of claims 1 to 4, an N-oxide compound, stereoisomer, tautomer and hydrate thereof and a salt of any of these compounds.

10. A granular agrochemical composition for paddy rice seedling nursery box treatment comprising at least one active ingredient selected from the group consisting of a compound according to any one of claims 1 to 4, an N-oxide compound, stereoisomer, tautomer and hydrate thereof and a salt of any of these compounds.

11. A soil treatment agent comprising at least one active ingredient selected from the group consisting of a compound according to any one of claims 1 to 4, an N-oxide compound, stereoisomer, tautomer and hydrate thereof and a salt of any of these compounds.

**12.** A bait agent comprising at least one active ingredient selected from the group consisting of a compound according to any one of claims 1 to 4, an N-oxide compound, stereoisomer, tautomer and hydrate thereof and a salt of any of these compounds.

**13.** A plant growth promoter comprising at least one active ingredient selected from the group consisting of a compound according to any one of claims 1 to 4, an N-oxide compound, stereoisomer, tautomer and hydrate thereof and a salt of any of these compounds.

**INTERNATIONAL SEARCH REPORT**

International application No. PCT/JP2019/038480

A. CLASSIFICATION OF SUBJECT MATTER
Int.Cl. see extra sheet

According to International Patent Classification (IPC) or to both national classification and IPC

B. FIELDS SEARCHED

Minimum documentation searched (classification system followed by classification symbols)
Int.Cl. C07D403/14, A01N43/54, A01N43/653, A01P5/00, A01P7/02, A01P7/04, A61K31/4439, A61K31/444, A61K31/506, A61P33/14, C07D401/04, C07D401/14, C07D403/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2017/104741 A1 (NIPPON SODA CO., LTD.) 22 June 2017, entire text & US 2018/0362470 A1 (entire text) & EP 3395801 A1 & CN 108430976 A & KR 10-2018-0095813 A & BR 112018011691 A & TW 201726628 A | 1, 2, 4-13<br>3 |
| A | WO 2016/012395 A1 (SYNGENTA PARTICIPATIONS AG) 28 January 2016, entire text (Family: none) | 1-13 |
| A | WO 99/02518 A1 (NIPPON SODA CO., LTD.) 21 January 1999, entire text & AU 8127898 A | 1-13 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 November 2019 (15.11.2019) | 10 December 2019 (10.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/038480

C (Continuation). DOCUMENTS CONSIDERED TO BE RELEVANT

| Category* | Citation of document, with indication, where appropriate, of the relevant passages | Relevant to claim No. |
|---|---|---|
| A | FILE REGISTRY ON SIN, RN 2166828-15-3, Entered SIN: 31 December 2017 | 1-13 |
| A | FILE REGISTRY ON SIN, RN 2169190-00-3, Entered SIN: 03 January 2018 | 1-13 |
| P, A | EP 3453706 A1 (BASF SE) 13 March 2019, entire text (Family: none) | 1-13 |

Form PCT/ISA/210 (continuation of second sheet) (January 2015)

**INTERNATIONAL SEARCH REPORT**

International application No.
PCT/JP2019/038480

CLASSIFICATION OF SUBJECT MATTER
C07D403/14(2006.01)i, A01N43/54(2006.01)i, A01N43/653(2006.01)i, A01P5/00(2006.01)i, A01P7/02(2006.01)i, A01P7/04(2006.01)i, A61K31/4439(2006.01)i, A61K31/444(2006.01)i, A61K31/506(2006.01)i, A61P33/14(2006.01)i, C07D401/04(2006.01)i, C07D401/14(2006.01)i, C07D403/04(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018187675 A
- JP 2018202997 A
- WO 2017104741 A
- WO 2018052035 A


### Non-patent literature cited in the description


- **COLBY. S.R.** Calculating Synergistic and Antagonistic Responses of Herbicide Combinations. *Weeds,* 1967, vol. 15, 20-22
- *Chem. Eur. J.,* 2015, vol. 21, 96-100